# EUROPEAN PATENT SPECIFICATION

(11) **EP 1 012 281 B1**
(45) Date of publication and mention of the grant of the patent: **29.11.2006**
(21) Application number: 98933785.2
(22) Date of filing: 13.07.1998
(51) Int. Cl.: C12N 15/12, C07K 14/705, C07K 16/28, C12N 5/10, A61K 38/17, G01N 33/53, G01N 33/68

(54) **HUMAN LEUKOCYTE CALCIUM ACTIVATED POTASSIUM CHANNEL POLYPEPTIDE**
MENSCHLICHES, DURCH KALZIUM AKTIVIERBARES KALIUMKANALPOLYPEPTID AUS LEUKOZYTEN
P0LYPEPTIDE HUMAIN DU CANAL POTASSIUM ACTIVE PAR LE CALCIUM

(30) Priority: 15.07.1997 GB 9714760; 09.10.1997 GB 9721366
(43) Date of publication of application: 28.06.2000
(73) Proprietor: AstraZeneca UK Limited, London W1Y 6LN (GB)
(72) Inventor: AIYAR, Jayashree, Wilmington, DE 19850-8437 (US); LOGSDON, Naomi, Jean, Wilmington, DE 19850-8437 (US)
(74) Representative: Phillips, Neil Godfrey Alasdair
(86) International application number: PCT/GB1998/002058
(87) International publication number: WO 1999/003882

(56) References cited:
- WO-A-98/11139
- KOHLER M ET AL: "Small-conductance, calcium-activated potassium channels from mammalian brain [see comments]" SCIENCE, SEP 20 1996, 273 (5282) P1709-14, XP002091031 UNITED STATES cited in the application
- JOINER WJ ET AL: "hSK4, a member of a novel subfamily of calcium-activated potassium channels." PROC NATL ACAD SCI U S A, SEP 30 1997, 94 (20) P11013-8, XP002091032 UNITED STATES
- LOGSDON NJ ET AL: "A novel gene, hKCa4, encodes the calcium-activated potassium channel in human T lymphocytes." J BIOL CHEM, DEC 26 1997, 272 (52) P32723-6, XP002091033 UNITED STATES

## Description

### ABSTRACT OF THE DISCLOSURE

A novel human leukocyte calcium activated potassium channel polypeptide is described which is expressed at a high level in activated T-cells. A full length cDNA which encodes the novel calcium activated potassium channel polypeptide is disclosed as well as the interior structural region and the amino acid residue sequence of the native biological molecule. Methods are provided to identify compounds that modulate the biological activity of a human leukocyte calcium-activated potassium channel.

### FIELD OF THE INVENTION

The present invention relates to nucleic acid and amino acid sequences of a novel human lymph node derived calcium activated potassium channel polypeptide and to the use of these sequences to identify compounds that modulate the activity of human leukocytes. The invention also related to the diagnosis, study, prevention, and treatment of disease related to dysfunctional leukocytes.

### BACKGROUND OF THE INVENTION

Hematopoietic stem cells give rise to all formed elements of the blood including the leukocytes which are generally implicated in various disease conditions including acute and chronic inflammation, asthma, allergies, graft rejection, proliferative disorders, anemias, septic shock and related disorders, psoriasis, neurodegenerative diseases with immunological components, as well as autoimmune diseases including rheumatoid arthritis, type-1 diabetes mellitus, multiple sclerosis, myasthenia gravis, systemic lupus erythematosus, Sjogren's syndrome, mixed connective tissue disease, and experimental allergic encephalomyelitis (EAE). Leukocytes comprise neutrophils, eosinophils, basophils, lymphocytes (which include T-cells and B-cells), and monocytes. Leukocytes can traverse every epithelium in the body regardless of its shape, thickness, or permeability. Gallin, E. K., *et al.,* Inflammation: Basic Principles and Clinical Correlates, Second Edition, 441-458 (1992).

The central lymphoid organs comprise bone marrow and thymus, where stem cells give rise to a diverse progeny of blood cell components including lymphocytes. Phagocytes, neutrophils, and monocytes develop in the bone marrow. After leaving the marrow they enter the bloodstream, where they form a circulating and marginating pool. Monocytes migrate into the tissues and body cavities, where they differentiate into macrophages. Lymphatic tissues in mammals are classified as either central or peripheral where they generate functional precursor lymphocytes or provide microenvironments for interaction of lymphoid cells with antigen and accessory cells. Lymphocytes are any of the mononuclear, non-phagocytic leukocytes, that are the body's immunologically competant cells and their precursors.

Movement of T and B lymphocytes and mononuclear cells through and within lymphatic tissue is vitally important for the intricate inductive and regulatory cellular interactions which take place during the initiation and course of *in vivo* immune responses including inflammation. At the top of the inflammatory response cascade are T-lymphocytes. T-cells respond to noxious substances by activation and sending out more than a dozen different chemical signals which attract inflammatory cells. Vogel, G., Science, 276:1643 (1997); *Immunophysiology,* Edited by Oppenheim, J.J., et al., Oxford (1990).

The T-cell directs the participation of leukocytes including B-cells and macrophages in overall physiological humoral and cellular immune responses. T-cells are responsible for directing cells to proliferate, migrate, and differentiate into appropriate effector cells via inter- and intracellular signal transduction. The importance of the bioactive integrity of a normal T-lymphocyte is readily perceived in clinical medicine today a result of the striking demonstration of the consequences of T-cell dysfunction. *Textbook of Internal Medicine,* Chp. 146, Lippincott, Phil. (1989).

The emigration of inflammatory cells from the blood into the tissues represents one of the most important components of Inflammatory response. However, it is not the actual accumulation of cells that is so critical but, rather, what they do in the tissue upon their arrival. Certain leukocytes (e.g., neutrophils and eosinophils), when appropriately stimulated, move from blood to tissues, and within seconds, release their contents into an endocytic vacuole or, by fusion with the plasma membrane, to the exterior of the cell. Thus, a major function in inflammatory processes are accomplished. The category of phagocytic inflammatory cells comprises neutrophils, eosinophils, and the mononuclear phagocytic series. Gallin, E. K., *et al.,* Inflammation: Basic Principles and Clinical Correlates, Second Edition, 441-458 (1992).

The chronic inflammatory disease, rheumatoid arthritis, for instance, is believed to be mediated by actvated T-ells that infiltrate the synovial membrane and initiate a series of inflammatory processes. Panayi. G.S., *et al., The Importance of the T-Cell in Initiating and Maintaining the Chronic Synovitis of Rheumatoid Arthritis,* Arthritis Rheum, 35:729 (1992). Accumulating evidence also indicates that the autoimmune disease multiple sclerosis (MS) is mediated by autoreactive T-lymphocytes. Stinissen. P., *et al.,* Crit. Rev. Immunol., 17(1):33 (1997). Autoreactive T-cells have been demonstrated to undergo *in vivo* activation and clonal expansion in patients with MS. Zhang, J., *et al.,* J. Mol. Med., 74(11):653 (1996). In diabetes mellitus, abnormal T-cells systematically destroy pancreatic islet cells such that they prove incapable of producing insulin. Moreover, another propelling recent development in the implication of overactive T-Cells is the recognition that a particular subset of T-lymphocytes appear to be a major culprit in asthma and other allergic diseases, by responding with undue vigor to apparently harmless invaders (rates of asthma per capita in the developing world have increased dramatically in the last several decades; doubling in the U.S. since 1980). *New Clues to Asthma Therapies:* Vogel, G., Science, 276:1643 (1997).

Leukocytes receive and respond to a variety of stimuli as part of their role in host defenses and immune function. Physiologic T-cell activation, for example, is achieved when T-cells encounter antigen presenting cells (APCs) bearing their cognate ligand (i.e., a particular peptide bound to a specific class I or class II MHC molecule). *Development and Function of Lymphocytes* (Paul, W. E., Inflammation: Basic Principles and Clinical Correlates, Second Edition. Edited by J. I. Gallin, I. M. Godlstein, and R. Snyderman. Raven Press, Ltd., New York (1992)).

Ion channels and fluxes have long been suspected to play a role in lymphocyte signal transduction. By controlling ion fluxes across the plasma membrane, potassium channels mediate changes in intracellular ion concentrations and membrane potential in response to a variety of stimuli. Calcium-activated potassium (K(Ca)) channels cause lymphocytes to hyperpolarize in response to the elevation of intracellular Ca²⁺ triggered by antigen-receptor engagement. The expression of both voltage-gated K(V) and K(Ca) channels is up-regulated as cells progress towards division following mitogenic stimulation. The regulatory aspects of lymphocyte potassium channels including why they are required for T-cell activation, as well as causal relationships among ion channels, membrane potential, mitogen stimulation and lymphokine gene expression has been reviewed. Lewis, R.S. and Cahalan, M.D., *Potassium and Calcium Channels in Lymphocytes,* Annual Review of Immunol., 13:623 (1995).

Recent advances in cloning of calcium activated potassium channels have greatly expanded the understanding of the structure of these macromolecules. Full-length cDNAs as well as peptide components of a previously unidentified potassium channel from the brain have been identified. Kohler, M., *et al.,* Science, 273:1709 (1996).

Thus far, the consensus is that at least two types of calcium-activated potassium K(Ca) channels, readily distinguished by their different conductances and pharmacological profiles, are expressed in a lineage-specific pattern in lymphocytes. The genes for these channels have not yet been cloned. Lewis. R.S. and Cahalan, M.D., *Potassium and Calcium Channels in Lymphocytes,* Annual Review of Immunol., 13:623 (1995).

T- and B-cell mitogens stimulate an increase in both the voltage-gated and calcium activated potassium channel density, and there is a positive correlation between K⁺-channel density and proliferative activity in thymocytes. Moreover, pharmacological studies clearly suggest a requirement for functional K⁺ channels in the activation of T and B cells. *Id*.

A number of marketed drugs function as potassium channel antagonists. The most important of these include the compounds Glyburide, Glipizide and Tolbutamide. These potassium channel antagonists are useful as antidiabetic agents. Potassium channel antagonists are also utilized as Class III antiarrhythmic agents and to treat acute infractions in humans. A number of naturally occurring toxins are known to block potassium channels including apamin, iberiotoxin, charybdotoxin (CTX), margatoxin, noxiustoxin, kaliotoxin, dendrotoxin(s), mast cell degranuating (MCD) peptide, and beta -bungarotoxin (beta -BTX).

A variety of pharmacological agents have been demonstrated to block the voltage-gated potassium channel and, in parallel, inhibit activation and proliferation of T and B-lymphocytes. Lewis, R.S. and Cahalan, M.D., *Potassium and Calcium Channels in Lymphocytes,* Annual Review of Immunol., 13:623 (1995).

Unfortunately, no compound has yet been found to block *calcium activated potassium channels* exclusively, especially in human leukocytes. Accordingly, there remains a need to identify a calcium activated potassium channel of leukocyte origin as a pharmacological target for screening candidate compounds for the modulation of cell activity. Such modulators are potentially useful in treating disorders manifested by dysfunctional leukocytes. There continues to be a need for an ability to screen for such compounds. Selective channel blockers are moreover needed to address the contribution of calcium activated potassium channels to the membrane potential and to signaling during T-cell activation. Grissmer, S., *et al., Calcium-activated Potassium Channels in Resting and Activated Human T Lymphocytes,* J. Gen. Physiol, 102:601 (1993). The central role of potassium channels in regulating numerous cell functions makes them particularly important targets for therapeutic development. Specific immune therapies designed to control T-cell activation and proliferation are likely to improve the clinical course of various autoimmune diseases.

### SUMMARY OF THE INVENTION

The present invention is directed to an isolated and purified polynucleotide molecule, which encodes a polypeptide of a potassium channel, comprising a nucleic acid sequence encoding the polypeptide having the sequence of SEQ ID NO:3. Isolated and purified polynucleotides for the present invention include but are not limited to SEQ ID NO:1, SEQ ID NO:7 (calcium-activated potassium channel cDNA) and SEQ ID NO:2 (calcium-activated potassium channel structural coding region).

In addition, the current invention is directed to a purified polypeptide comprising the amino acid sequence of SEQ ID NO:3.

The invention is further directed to an expression vector for expression of a polypeptide of a calcium-activated potassium channel in a recombinant host, wherein said vector contains a polynucleotide comprising a nucleic acid sequence encoding the polypeptide of a calcium-activated potassium channel having the sequence of SEQ ID NO:3.

Further the invention is directed to a host cell containing an expression vector for expression of a polypeptide of a calcium-activated potassium channel, wherein said vector contains a polynucleotide comprising a nucleic acid sequence encoding the polypeptide of a calcium-activated potassium channel having the sequence of SEQ ID NO:3.

The instant invention is further directed to a method of identifying compounds that modulate a potassium channel activity, comprising:
(a) combining a candidate compound modulator of the activity of a potassium channel with the potassium channel having the sequence of SEQ ID NO:3 and
(b) measuring an effect of the modulator on the channel.

The invention is also directed to a method of identifying compounds that modulate the activity of a potassium channel, comprising:
(a) combining a candidate compound modulator of a potassium channel activity with a host-cell expressing the polypeptide of a potassium channel having the sequence substantially as depicted in SEQ ID NO:3, and
(b) measuring an effect of the modulator on the channel.

The invention is further directed to an antisense polynucleotide molecule comprising the complement of SEQ ID NO:2.

The current invention is also drawn toward an antibody specific for a purified polypeptide comprising the amino acid sequence of SEQ ID NO:3.

The current invention is also drawn toward a diagnostic composition comprising an antibody specific for a purified polypeptide comprising the amino acid sequence of SEQ ID NO:3 for the identification of a polypeptide sequence comprising the amino acid sequence of SEQ ID NO:3.

### BRIEF DESCRIPTION OF THE FIGURES

Figure 1 displays SEQ ID NO: 1 which is the 2262 base full-length cDNA nucleic acid sequence which encodes the novel human lymph node derived calcium activated potassium channel polypeptide (hKCa4) described herein.
Figure 2 displays SEQ ID NO:2 which is the translated structural region, **ATG** to **TAG,** of the cDNA nucleic acid sequence which encodes the novel human lymph node derived calcium activated potassium channel polypeptide described herein.
Figure 3 displays SEQ ID NO:3 which is the 427 amino acid residue sequence of the novel human lymph node derived calcium activated potassium channel polypeptide described herein.
Figure 4 shows SEQ ID NO:4 which is the ammo acid residue sequence of the HSK1 brain derived small-conductance calcium activated potassium channel polypeptide (Kohler, M.. *et al.,* Science, 273:1709 (1996)).
Figure 5 shows SEQ ID NO:5 which is the amino acid residue sequence of the RSK2 brain derived small-conductance calcium activated potassium channel polypeptide (Kohler, M., *et al.,* Science, 273:1709 (1996)).
Figure 6 shows SEQ ID NO:6 which is the amino acid residue sequence of the RSK3 brain derived small-conductance calcium activated potassium channel polypeptide (Kohler, M., *et al.,* Science, 273:1709 (1996)).

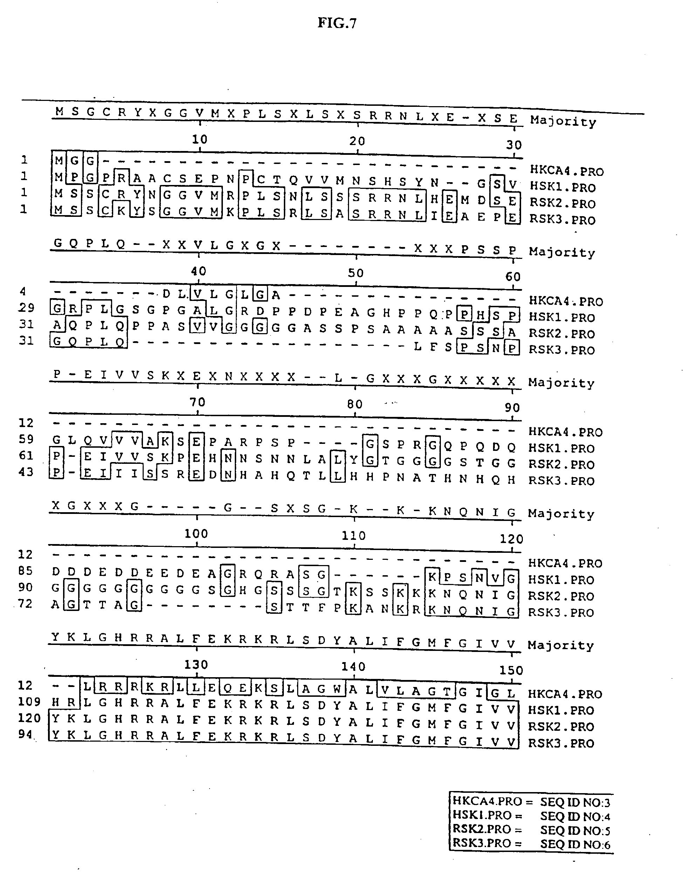

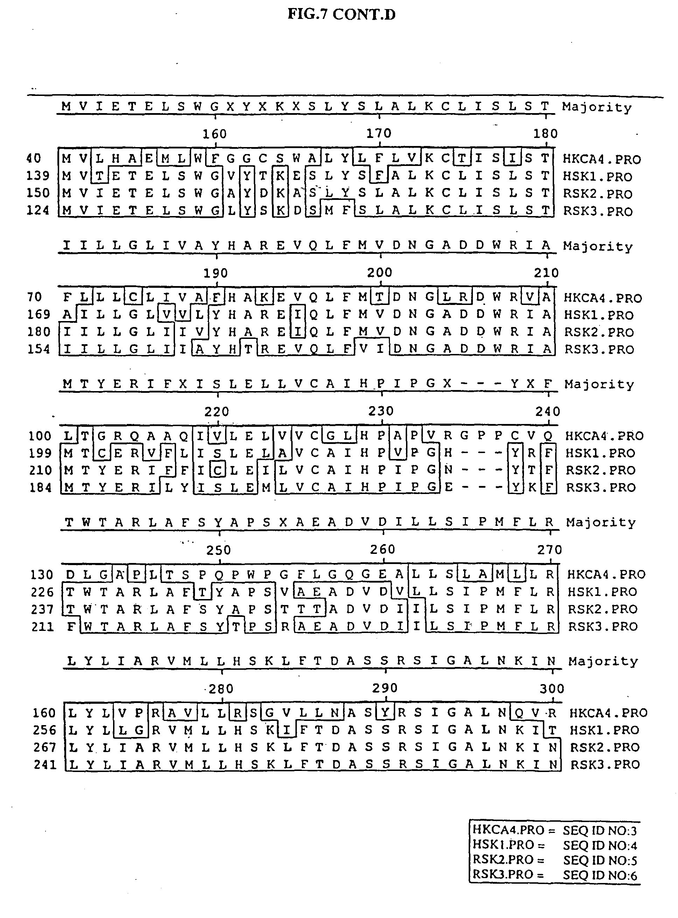

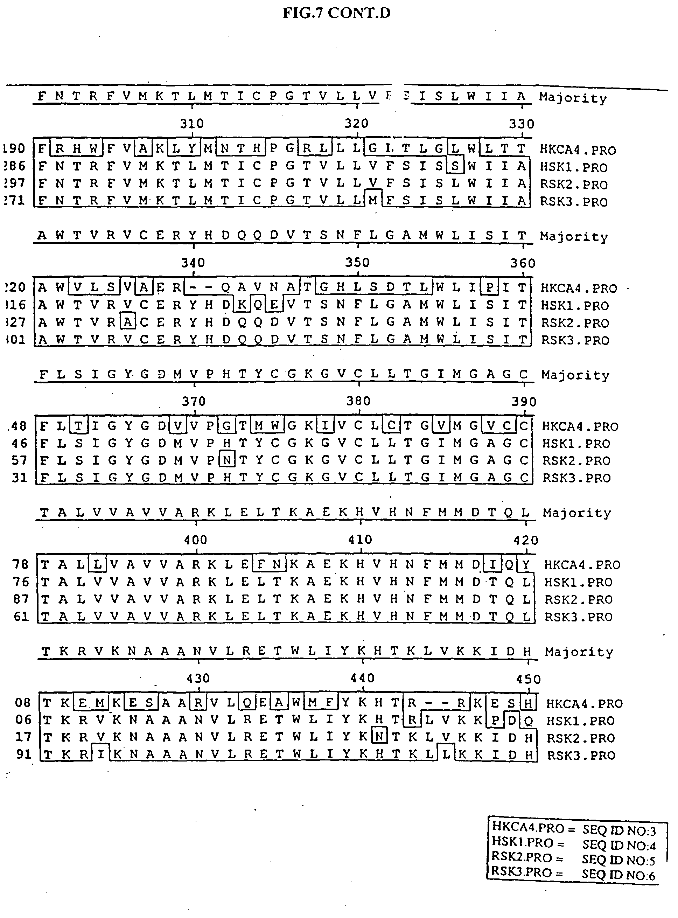

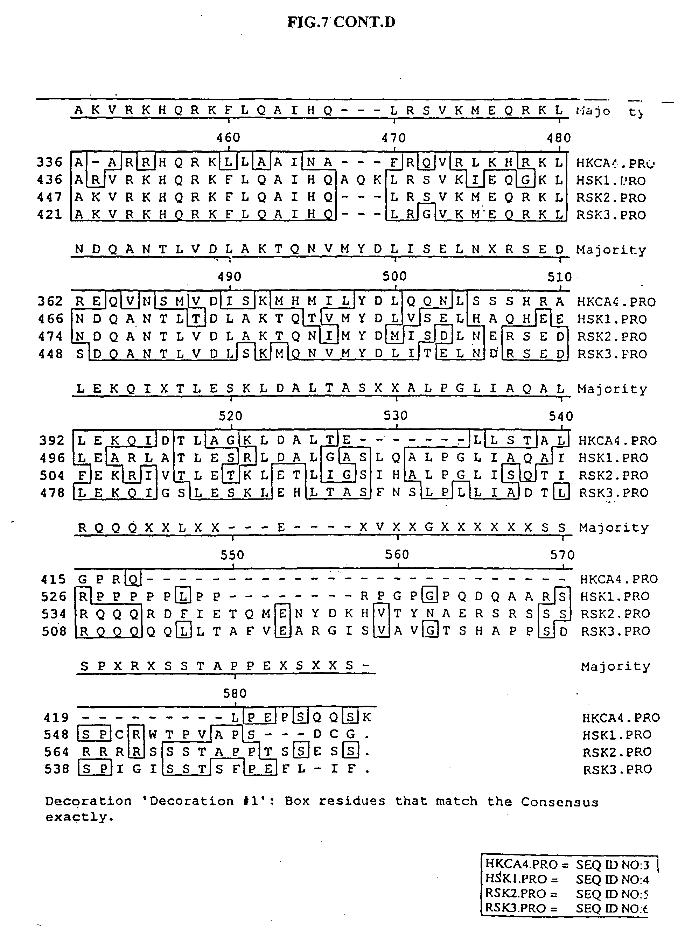

Figure 7 shows a comparison between the amino acid residue sequence of the novel human lymph node derived calcium activated potassium channel polypeptide described herein (SEQ ID NO:3) (designated HKCA4), and the amino acid residue sequences of the brain derived small-conductance calcium activated potassium channel polypeptides HSKl (SEQ ID NO:4), RSK2 (SEQ ID NO:5), and RSK3 (SEQ ID NO:6). Conserved amino acid residues are boxed. Dashes represent gaps introduced to optimize the alignment. Sequences shown in this figure were produced using the multisequence alignment program of DNASTAR software (DNASTAR Inc, Madison WI).
Figure 8 displays a hydrophobicity plot of the 427 amino acid residue sequence of the novel human lymph node derived calcium activated potassium channel polypeptide (SEQ ID NO:3). Six (6) transmembrane regions are indicated. S 1 through S6. Kyte, J., Doolittle, R.F., J. Mol. Biol., 157:105 (1982). (DNASTAR Inc. Madison WI).
Figure 9 shows a strong mRNA signal of the the novel human lymph node derived calcium activated potassium channel described herein upon T-cell activation in Northern blot analysis.

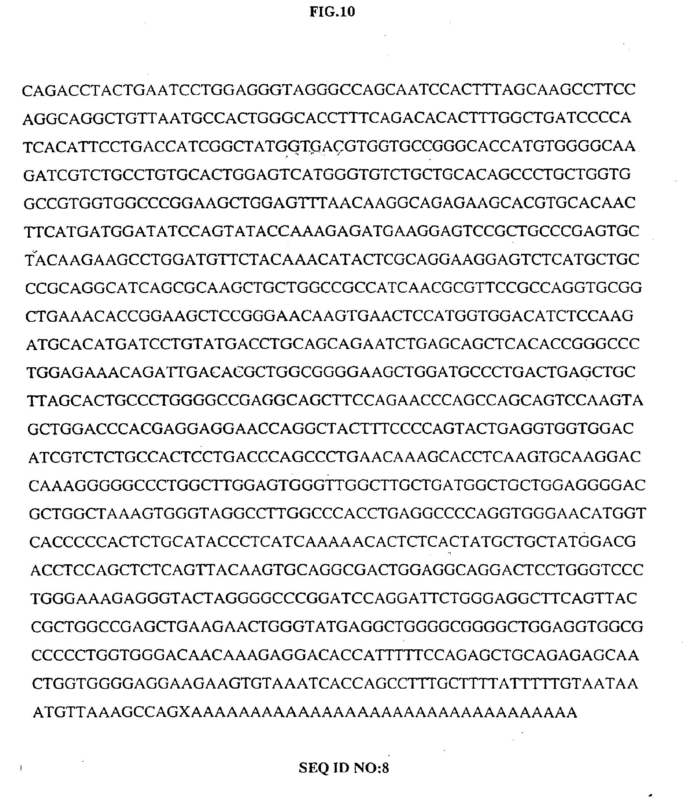

Figure 10 shows SEQ ID NO:8 which is a partial cDNA clone of the novel human lymph node derived calcium activated potassium channel polypeptide described herein.

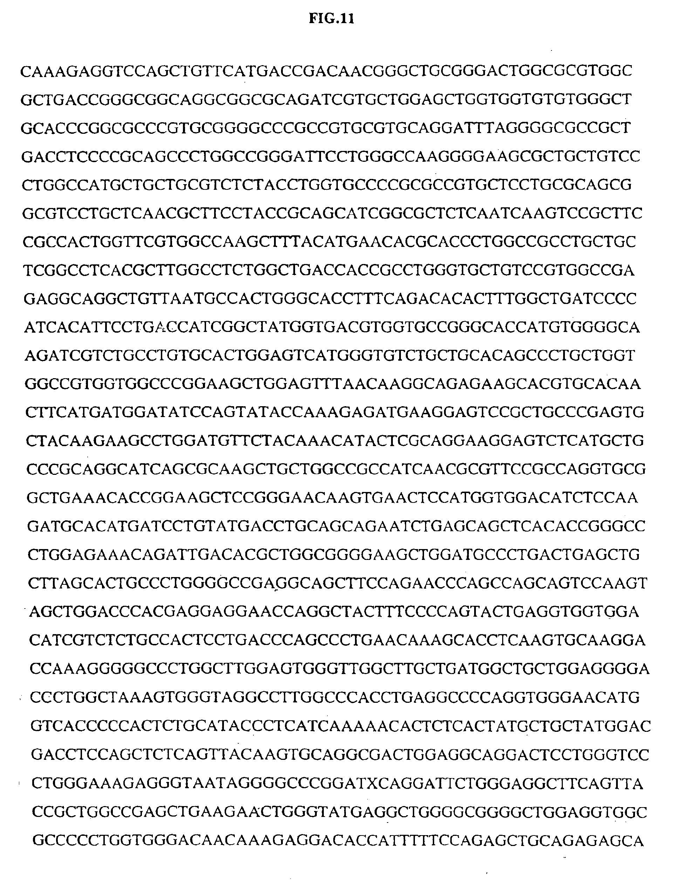

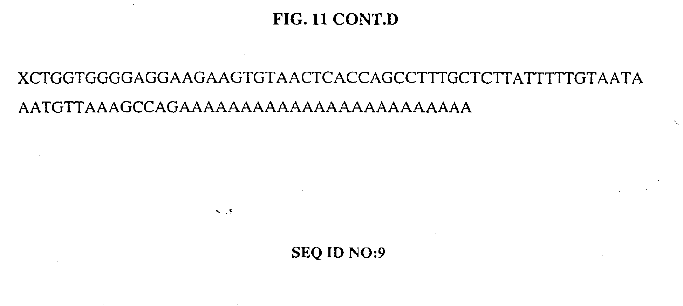

Figure 11 shows SEQ ID NO:9 which is a partial cDNA clone of the novel human lymph node derived calcium activated potassium channel polypeptide described herein.
Figure 12 shows SEQ ID NO:7 a 2238 base full-length cDNA nucleic acid sequence which comprises SEQ ID NO:2 and encodes the novel human lymph node derived calcium activated potassium channel polypeptide described herein (a 5' UTR variation of SEQ ID NO:1, the initiator codon and stop codon are underlined).

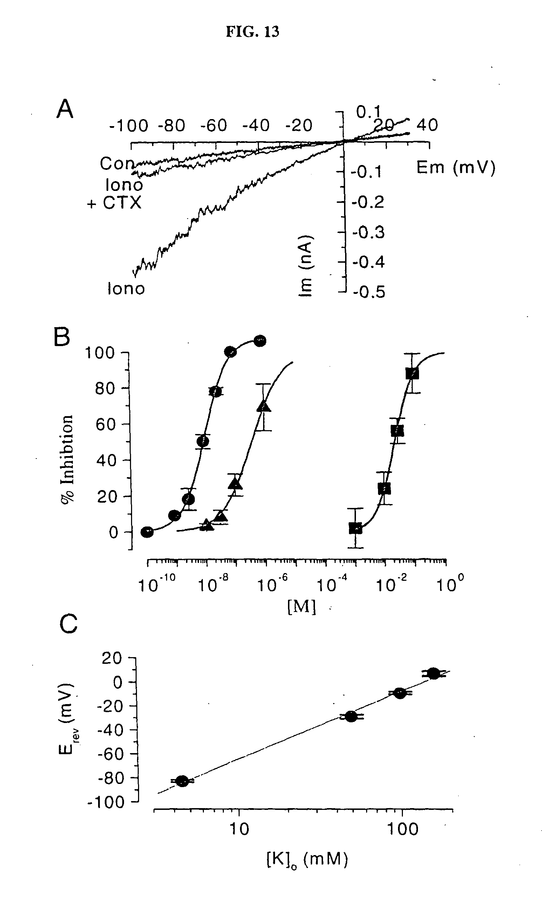

Figure 13 demonstrates KCa currents (biological activity) in HEK 293 cells transfected with SEQ ID NO:2 (a) Dependence of whole cell current on 1 µM ionomycin perfusion and block by CTX (100 nM). Superimposed currents were evoked by 200 ms voltage ramps from -100 to +40 mV (E_{hold} = -50 mV). The pipette solution contained 100 nM [Ca²⁺]_{free}, and the bath solution contained 160 mM K⁺. (b) Concentration-dependent block of KCa current by CTX (•), TEA (■), and Clotrimazole (▲). Protocol is the same as in (a), except that [Ca²⁺]_{free} was buffered to I µM in the pipette and ionomycin was omitted from the bath. Currents were measured at -95 mV on the ramp and normalized between control amplitude and that obtained during perfusion with 100 nM CTX in the same experiment. Each point is the mean ± SEM of 3-5 experiments. Solid lines are fits to a Hill equation of the following form: 100 /[1+ (K_{d}/x)ⁿ ], where *x* is concentration, K_{d} the concentration producing 50 percent inhibition, and n is the slope factor of the line. See text for fitted parameters (c) K⁺ selectivity of KCa current. Each point represents the mean ± SEM voltage (n = 3-6) where control current converged with current in CTX during voltage ramps at the designated K⁺₍ₒᵤₜ₎ concentration. Solid line is a linear regression to the data (slope 57 mV).

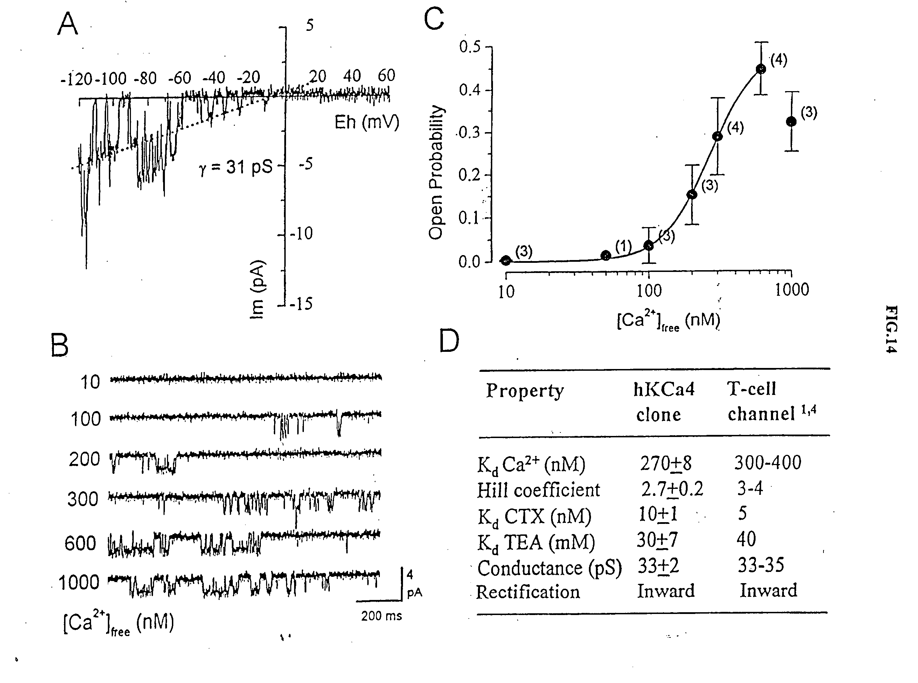

Figure 14 displays single channel records and calcium-sensitivity of the novel channel (SEQ ID NO:2): (a) Single-channel current from a cell-attached patch with 160 K⁺ in the pipette. The bath contained Ringer with 1 µM ionomycin. The patch was ramped over 100 ms from - 120 to +60 mV. A blank trace (i.e., no channel openings) was digitally subtracted. The dashed line represents a slope conductance of 31 pS. (b) Representative traces obtained from an inside-out patch excised from an hKCa4-transfected cell (E_{hold}= -80 mV). The pipette contained 160 mM K⁺ and patch was perfused with the [Ca²⁺]_{free} indicated. (c) Relationship between open probability and cytoplasmic [Ca²⁺]_{free} determined using the experimental protocol in (b). Single channel open probabilities were determined by the relative areas of Gaussian fits to amplitude-frequency histograms. Points are the mean ± SEM from designated number of experiments. Solid line is a fit to the Hill equation described in FIG.13b. (d) Summary table of data obtained from experiments shown in FIG.13 & 14 for SEQ ID NO:2-expressed in HEK cells compared to published data for the human T cell KCa channel.

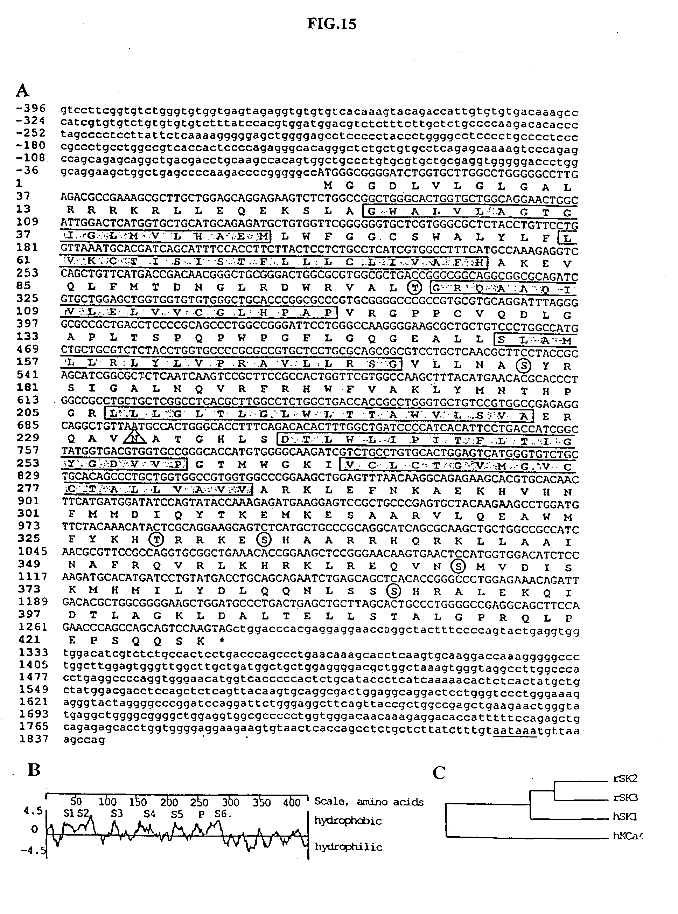

Figure 15 displays the nucleic acid sequence of SEQ ID NO:7 as well as the encoded amino acid sequence (SEQ ID NO:3). Predicted transmembrane regions are boxed and shaded. Consensus sites for glycosylation are indicated by a triangle, serine-threonine phosphorylation sites are circled, the polyadenylation signal is underlined, and the stop codon is indicated by an asterisk. (b) Hydropathy plot of SEQ ID NO:3 showing six predicted transmembrane segments and a pore between S5 and S6. (c) Amino acid dendrogram of SEQ ID NO:3 with the recently cloned small-conductance KCa channels, hSK1, rSK2 and rSK3 (SEQ ID NO:4, SEQ ID NO:5 and SEQ ID NO:6 respectively).
Figure 16 shows Northern blot analysis of SEQ ID NO:7 message: (a) in multiple tissues, (b) in resting and activated human T lymphocytes, showing up-regulation of message, (c) patch clamp analysis of representative T cells that were subject to Northerns in (*b*) showing up-regulation of KCa channel currents upon activation. Each superimposed current trace was elicited by a 200 ms voltage ramp from -100 to +40 mV (E_{hold} = -50 mV). Trace 1: Resting T cell, 2: Resting T cell plus 100 nM CTX. 3: Activated T cell, 4: Activated T cell plus 100 nM CTX. Components of Kv and KCa currents are shown.
Figure 17 shows patch clamp current recording of stable HEK293/SEQ ID NO:2 transfectants.
Figure 18 shows a dose-response curve for CTX-binding hKCa4 (SEQ ID NO:3) channels.
Figure 19 shows Western Blot analysis of anti-SEQ ID NO:3 antibodies.
Figure 20 demonstrates down-regulation of the calcium activated potassium channel (SEQ ID NO:3) currents in T cell-lines induced to anergy.
Figure 21 illustrates deletion analysis of the SEQ ID NO:3 COOH-tail to identify the calmodulin interaction site.
Figure 22 shows the full-length cDNA (SEQ ID NO: 13) of the murine Kca4 ortholog which includes the structural coding region (underlined ATG → TAG).
Figure 23 shows the native amino acid sequence (SEQ ID NO: 14) of the murine Kca4 homolog.
Figure 24 shows amino acid alignment of mouse and human KCa4 homolog sequences.

### DETAILED DESCRIPTION OF THE INVENTION

Unless defined otherwise, all technical and scientific terms used herein have the same meaning as is commonly understood by one of skill in the art to which this invention belongs.

Biological activity as used herein refers to the ability of a calcium activated potassium channel to allow transmembrane potassium ion flow and/or transport or regulate transmembrane potassium ion flow and/or transport. Biological activity as used herein is also intended to encompass pharmacological activity, *per se.* as in the ability of a subunit to bind or associate with at least one other channel peptide subunit, ligand, or cofactor (e.g., calmodulin and/or calcium) and/or otherwise modulate the biological activity of a potassium channel.

Biologically active fragment as used herein includes peptides which have been truncated with respect to either the N- or C-termini, or both; or the corresponding 5' or 3' end of the corresponding nucleic acid coding regions, or both, which fragments perform substantially the same function or encode peptides which perform substantially the same function in substantially the same way as the precursor. Biologically active fragment as used herein is also intended to encompass pharmacologically active dominant negative mutants contemplated herein.

Nucleic acid sequence as used herein refers to an oligonucleotide, nucleotide or polynucleotide sequence, and fragments or portions thereof, and to DNA or RNA of genomic or synthetic origin which may be double-stranded or single-stranded whether representing the sense or antisense strand. Similarly, amino acid sequence as used herein refers to peptide or protein sequences or portions thereof. The term 'purified' refers to molecules, either nucleic or amino acid sequences, that are removed from their natural environment and isolated or separated from at least one other component with which they are naturally associated.

'Substantially as depicted' as used herein refers to functional derivative proteins, variant peptides and DNA sequences that may have changes but perform substantially the same biochemical function in substantially the same way; however, 'substantially as depicted' as used herein also refers to dominant negative mutant versions.

Direct administration as used herein refers to the direct administration of nucleic acid constructs which encode embodiments (e.g., SEQ ID NO:3, dominant negative mutant, modulator compound molecule, antisense molecule, antibody molecule) of the present invention or fragments thereof; and the direct administration of embodiments of the present invention or fragments thereof, and the *in vivo* introduction of molecules of the present invention preferably via an effective eukaryotic expression vector in a suitable pharmaceutical carrier. Polynucleotides and therapeutic molecules of the present invention may also be delivered in the form of nucleic acid transcripts.

The term 'modulation' is used herein to refer to the capacity to either enhance or inhibit a functional property of a subunit or potassium channel. The term 'modulation' is also used herein to refer to the capacity to affect the biophysical activity of a cell. Modulate physiology as used herein refers to the biophysiological regulation of cells and/or tissue and the treatment of pathophysiological disorders related thereto.

Modulation or regulation of biological activity and/or pharmacological activity as used herein refers to binding, blocking, antagonization, repression, neutralization, or sequestration, of a potassium channel biomolecular structure including but not limited to the novel calcium activated potassium channel poypeptide described herein; as well as *up regulation* or agonization or activation of a potassium channel by a compound identified by means described herein.

The nucleic acid sequence also provides for the design of antisense molecules useful in downregulating, diminishing, or eliminating expression of the genomic nucleic acid sequence in cells including, but not limited to, leukocytes, endothelial cells, microglia, and tumor or cancer cells.

Expression vector as used herein refers to nucleic acid vector constructions which have components to direct the expression of heterologous protein coding regions including coding regions of the present invention through accurate transcription and translation in host cells. Expression vectors usually contain a promoter to direct polymerases to transcribe the heterologous coding region, a cloning site at which to introduce the heterologous coding region, and usually polyadenylation signals. Expression vectors include but are not limited to plasmids, retroviral vectors, viral and synthetic vectors.

Transformed host cells as used herein refer to cells which have coding regions of the present invention stably integrated into their genome, or episomally present as replicating or nonreplicating entities in the form of linear nucleic acid or transcript or circular plasmid or vector.

### Calcium activated potassium channels

Calcium-activated potassium (K(Ca)) channels cause lymphocytes to hyperpolarize in response to the elevation of intracellular Ca²⁺ triggered by antigen-receptor engagement. The expression of both voltage-gated K(V) and calcium-activated potassium K(Ca) channels is up-regulated as cells progress towards division following mitogenic stimulation. Calcium signal is required to enable a number of essential activation events closely linked to the opening and closing of calcium and potassium channels as demonstrated by Ca²⁺ imaging in single cells. Sustained signaling and oscillations depend absolutely on plasma-membrane Ca²⁺ channels that are activated indirectly by the depletion of intracellular calcium stores. Lewis, R.S. and Cahalan, M.D., *Potassium and Calcium Channels in Lymphocytes,* Annual Review of Immunol., 13:623 (1995).

The surface density of potassium channels in the plasma membrane of T-lymphocytes is regulated during development and during activation of mature cells by mitogens. For reviews, see Cahalan, *M.D., et al.;* Curr. Top. Membr., 39:357-394; Lewis, R.S*.. et al.,* Trends Neurosci.. 11:214-218. Expression of calcium activated potassium K(Ca) channels in human T-cells is profoundly up-regulated by mitogens, increasing from ~20 per resting cell to >500 channels per T-cell blasts treated with phytohemmaglutinin (PHA). Thus, the relative contribution of K(Ca) channels to the membrane potential appears to be greatly enhanced in activated cells and perhaps in memory cells. Lewis, R.S. and Cahalan, M.D., *Potassium and Calcium Channels in Lymphocytes,* Annual Review of Immunol.. 13:623 (1995).

Grissmer, S., *et al.,* have further demonstrated that mitogenic stimulation of human T-cells results in an increase in the cytosolic free Ca²⁺ concentration, that is vital for subsequent events leading to cell proliferation and secretion of lymphokines. Calcium activated potassium channels (K(Ca)) were characterized in resting and activated human peripheral blood T-lymphocytes using simultaneous patch-clamp recording and fura-2 monitoring of cytosolic Ca²⁺ concentration. Whole-cell experiments, using EGTA-buffered pipette solutions to raise [Ca²⁺]ᵢ to 1 µM, revealed a 25-fold increase in the number of conducting calcium-activated potassium channels per cell, from an average of 20 in resting T cells to >500 channels per cell in T cell blasts after mitogenic activation. *Calcium-activated Potassium Channels in Resting and Activated Human T Lymphocytes,* J. Gen. Physiol, 102:601 (1993). The high flow rate of calcium activated potassium channels, and discrete opening and closing, permits measurement of currents through single channels using standard patch-clamp techniques. Reported results indicate that expression of the charybdotoxin (CTX) -sensitive calcium activated potassium channel increases dramatically after activation. Activated T-cell blasts express many more calcium activated potassium channels than resting HPB T-lymphocytes. The increased number of K(Ca) channels reflects an increase primarily in the surface density of channels. J. Gen. Physiol, 102:601 (1993).

Charybdotoxin, (CTX), a 37 amino-acid peptide purified from the venom of scorpion *Leiurus quinquestriatus* (Gimenenz-Gallego et al., PNAS, 85: 3329-3333, 1988) has been shown to block mitogen-induced proliferation of human peripheral T-lymphocytes (Lin C et al., FASEB J. 6: 1693, 1992). This effect of CTX has been demonstrated to act via blockade of potassium channels which maintain the cell's resting membrane potential (Leonard R et al., PNAS 89: 10094-98. 1992). CTX is a blocker of both the calcium-activated potassium channel (KCa) and the voltage-gated potassium channel (Kvl.3) in T-cells (Grissmer et al., J. Gen Physiol. 102: 601-630, 1993). CTX affects only the calcium-dependenl activation pathways in lymphocytes (Garcia ML. J Bioenerg. Biomembr 23: 615-646, 1991). Radiolabeled CTX has been used as a ligand for high-throughput screening (HTS) of drugs for Kvl.3 (Hill RJ, Mol. Pharm. 48: 98-104, 1995; Deutsch C et al., J. Biol. Chem. 266: 3668-3674, 1991). Although other peptide toxins that selectively block Kv1.3 have been identified, there is no pharmacological agent that selectively blocks the KCa channel in lymphocytes with high potency. With the cloning of the novel calcium activated potassium channel described herein, it is now possible to generate a cell-line that over-expresses the channel which can then be used in high throughput screening (HTS) to screen for selective blockers of the calcium-activated potassium channel in T-lymphocytes. These channel-blocking agents could also be used as research tools to understand the physiological role of these channels in a variety of cells of hematopoietic and other origin.

Selective channel blockers are needed to address the contribution of calcium activated potassium channels to the membrane potential and to signaling during T-cell activation. Grissmer, S., *et al., Calcium-activated Potassium Channels in Resting and Activated Human T Lymphocytes,* J. Gen. Physiol, 102:601 (1993).

The calcium activated potassium channels described by Grissmer, S., *et al.,* were moreover reported to resemble those found in red blood cells, the cell type in which K(Ca) channels were first discovered through tracer flux measurements. Gardos, G., *The Function of Calcium in the Potassium Permeability of Human Erythrocytes,* Biochim. Biophys. Acta., 30:653 (1958); reviewed by Schwarz and Passow, *Calcium Activated Potassium Channels in Erythrocytes,* Annual Review of Physiology, 45:359 (1983); *see also,* Brugnara, C., et al., *Therapy with Oral Clotrimazole Induces Inhibition of the Gardos Channel and Reduction or Erythrocyte Dehydration in Patients with Sickel Cell Disease,* J. Clin. Investigation, 97(5):1227 (1996).

U.S. Patent No. 5,397,702, *Assay For and Treatment of Autoimmune Diseases,* issued March 14, 1995; U.S. Patent No. 5,637,470, *Screening array using cells expressing recombinant alpha and beta subunits of the mammalian large-conductance (maxi-K) potassium channel,* issued June 10, 1997; U.S. Patent No. 5,607,843, *Nucleic Acid Sequence Encoding Apamin Binding Protein,* issued March 4, 1997; and U.S. Patent No. 5.602,169, *3-substituted oxindole derivatives as potassium channel modulators,* issued Feb. 11, 1997 are referred to.

### IK (intermediate conductance) calcium activated potassium channels

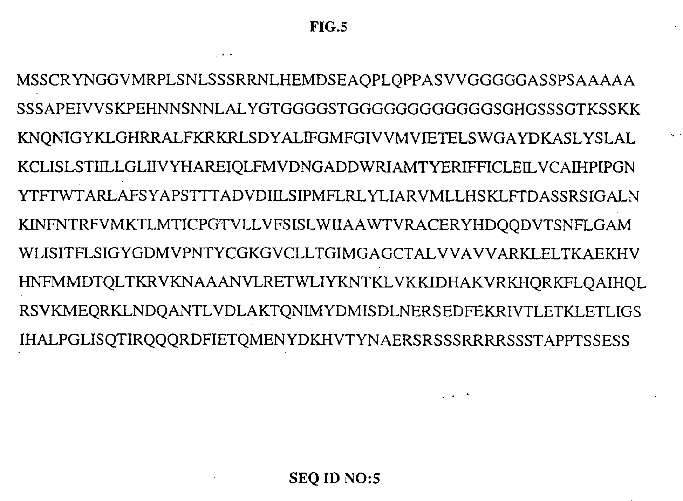

The IK channel is expressed in hematopoetic and endothelial cells, certain cells of epithelial origin and in red blood cells. In the red blood cells, where the channel has been denominated the Gardos channel, a rise in the concentration of intracellular Ca²⁺ opens the channel and causes potassium loss and cell dehydration, a condition which is exacerbated in sickle cell anemia. Promising therapeutic approaches for sickle cell anemia involve specifically blocking the IK channel. IK channels have also been implicated in the microvasculature of the kidney, where they may be responsible for the vasodilatory effects of bradykinin. The decrease in blood pressure during septic shock is caused by an increased NO production by the endothelial cells, and the IK channels in these cells are responsible for maintaining the Ca²⁺ influx activating the Ca²⁺-sensitive NO-synthase. In brain capillary endothelial cells, IK channels, activated by endothelin that is produced by neurons and glia, shunt excess K⁺ into the blood. Neutrophil granulocytes, mobile phagocytic cells that defend against microbial invaders, undergo large depolarization subsequent to agonist stimulation, and IK channels have been implicated in repolarizing the stimulated granulocyte. The IK channel is sensitive to a number of peptide toxins as well as organic blockers. Charybdotoxin is the most potent peptide blocker (IC₅₀ = 5-10 nM on cloned hKCa4 (SEQ ID NO:3, *infra*)) and clotrimazole is the most potent of the antimycotics (IC₅₀ = 100-300 nM) (Figs 5-6). Clotrimazole is the compound which is being evaluated for the treatment of sickle cell anemia.

### Brain-derived calcium activated potassium channels

Köhler, M. *et al.,* describe the molecular structures of members of a previously unidentified family of calcium activated potassium channels with six transmembrane domains cloned from rat and human brain. Three full-length coding sequences were isolated and translated into their respective amino acid residue sequences, one from human, HSK1 (SEQ ID NO:4) (561 residues), and two from rat brain, rSK2 (SEQ ID NO:5) (580 residues), and rSK3 (SEQ ID NO:6) (553 residues). Expression of the respective mRNAs in *Xenopus* oocytes resulted in calcium-activated, voltage-independent potassium channels. Science. 273:1709 (1996). Hydrophobicity analysis predicts six transmembrane segments of each with the NH2- and COOH-termini residing inside the cell. The sequences are highly conserved across their transmembrane cores (80 to 90% homology) but diverge in sequence and length within their NH2- and COOH-terminal domains. The cloned channels show no significant amino acid homology to other cloned potassium channel subunits except for a 12-residue stretch within the putative pore domain. Hydrophobicity plots predicted that these subunits contain six transmembrane domains, a topology shared with members of the voltage-gated class of K+ channels. Jan, L.Y., et al., Nature. 345:672 (1990). Although related in topology to voltage-dependent K⁺ channels, including a P region and S4 segment, the Köhler, M. *et al.* clones reside on a distinct evolutionary branch within the K⁺ channel superfamily. Science, 273:1709 (1996).

### Novel human lymph node derived calcium activated potassium channel (IK) polypeptide

Potassium channels play a critical role in modulating calcium signaling of lymphocytes. Verheugen, J. A., *et al.,* Cell Calcium, 17: 287 (1995). Human T lymphocytes express at least two types of potassium channels: those that open in response to changes in membrane potential (Kv channels) and those that are activated following elevations of intracellular calcium levels (KCa channels). Lewis, R. S., *et al.,* Annu. Rev. Immunol., 13:623 (1995). The predominant Kv channel in human T cells is encoded by Kv1.3, a *Shaker*-related voltage-gated potassium channel gene. Kv1.3 has been extensively characterized at the molecular and physiological level and plays a vital role in controlling T cell proliferation, mainly by maintaining the resting membrane potential of the cell. Chandy, K. G., *et al.,* Sem. Neurosci., 5:125 (1993). Upon T cell activation, there is at most a 2-fold increase in Kv1.3 currents. The predominant KCa channel in human T lymphocytes is of intermediate conductance, is voltage-insensitive and is potently blocked by the scorpion venom peptide, charybdotoxin (CTX; 4). Unlike Kv1.3, KCa channel currents are up-regulated dramatically (10-25 fold) following mitogenic or antigenic stimulation, and are thought to play a significant role in post-activation and secondary immune phenomena. Grissmer, S., *et al.,* 102:601 (1993); Verheugen, J. A., *et al.,* Cell Calcium. 21:1 (1997). KCa channels with very similar biophysical and pharmacological properties to the T lymphocyte channel have also been identified in red blood cells, e.g., Gardos channel, macrophages, neutrophils, B lymphocytes as well as in other non-immune peripheral tissues. Grygorczyz. R., et al., Biophys. J., 45:693 (1984); Gallin, E. K., Am. J. Physiol., (Cell Physiol. 26) 257:C77-C-85 (1989); Karl-Heinz, *et al.,* J. Clin. Invest., 85:491 (1990); Mahaut-Smith. M. P., *et al.,* J. Physiol., 415:69 (1989). However, the molecular identity of this channel type has hitherto been unknown. The cloning and characterization of an intermediate-conductance, CTX-sensitive KCa channel, called hKCa4 (SEQ ID NO:2: SEQ ID NO:3) from a human lymph node cDNA library is described herein. Convergent molecular, biophysical, and pharmacological evidence that the novel sequences presented herein (e.g.. SEQ ID NO:2. SEQ ID NO:3, SEQ ID NO:1, and SEQ ID NO:7) correspond to the the predominant KCa channel in human T cells is presented.

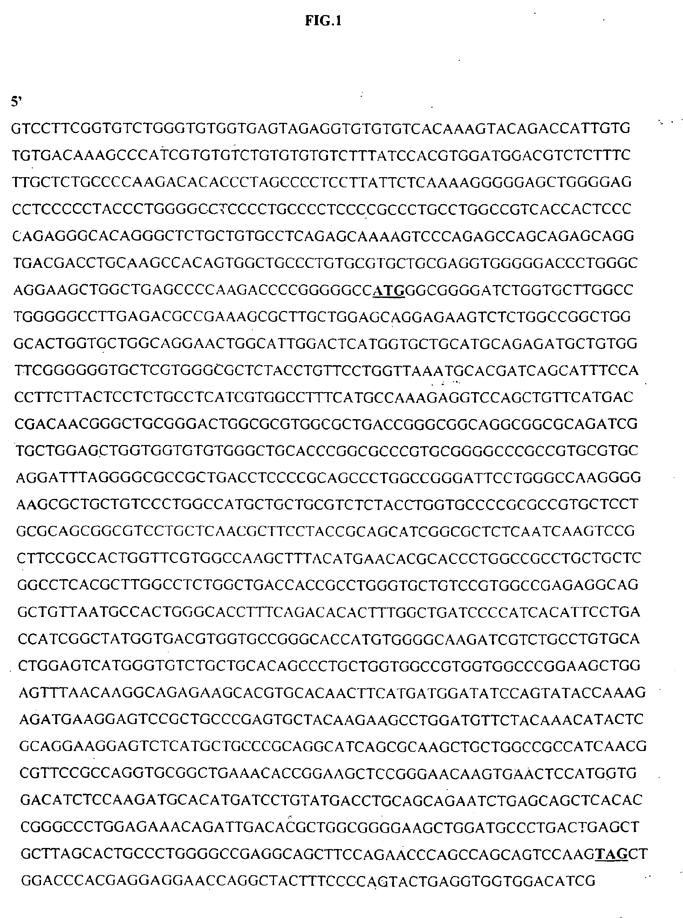

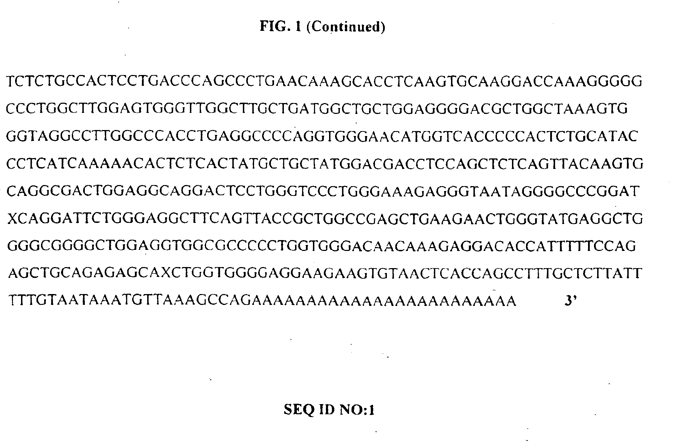

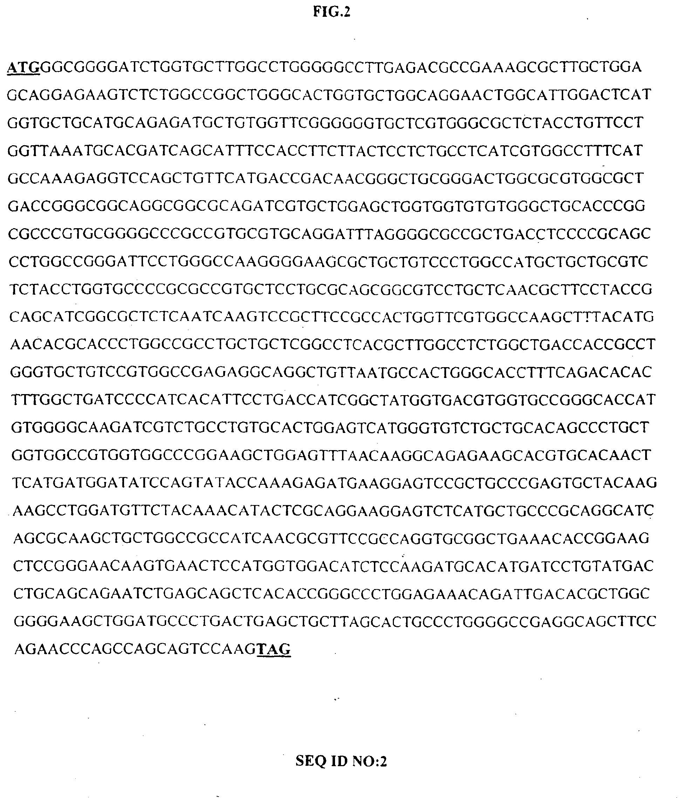

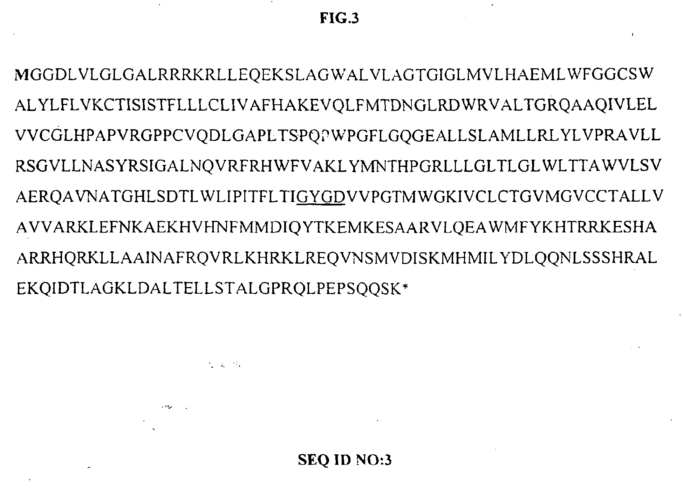

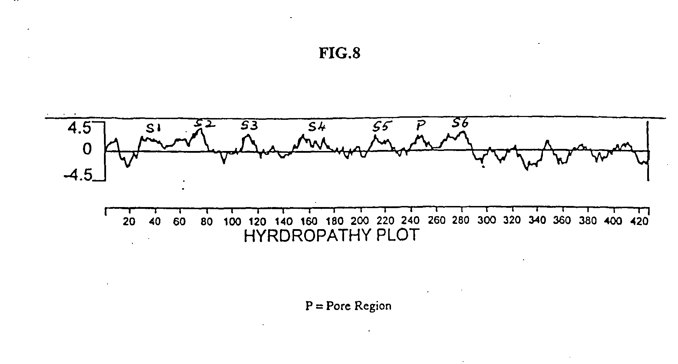

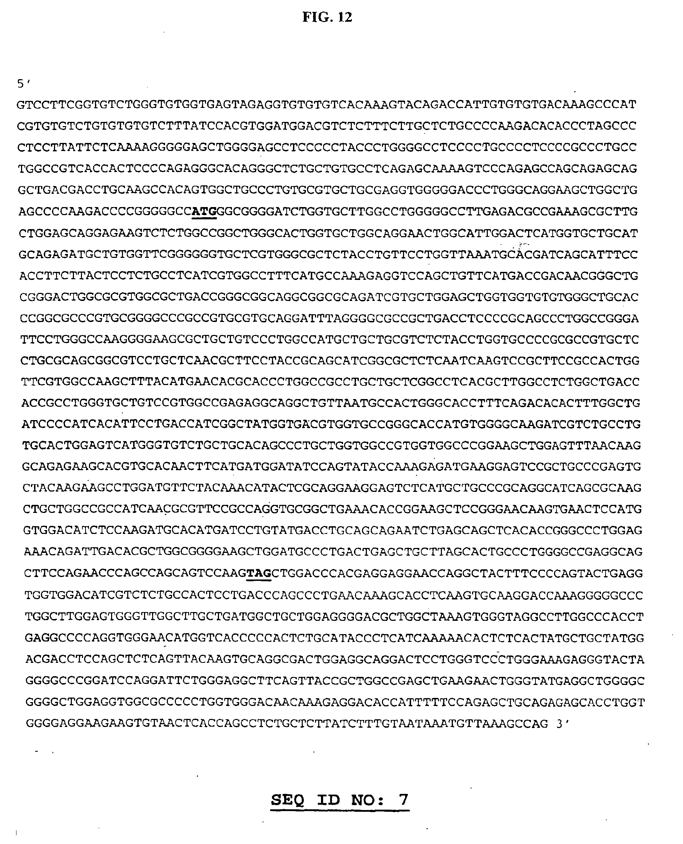

Example full-length cDNAs from human lymph node which comprises the coding region for the novel calcium activated potassium channel polypeptide described herein is set forth in FIG.1 (SEQ ID NO:1) and FIG.12 (SEQ ID NO:7). The native structural coding region for the subject polypeptide is set forth in FIG.2 (SEQ ID NO:2). The amino acid residue sequence of the native calcium activated potassium channel polypeptide described herein is set forth in FIG.3 (SEQ ID NO:3). The translated protein SEQ ID NO:3 is comprised of 427 amino acid residues FIG.3 and appears to have 6 transmembrane regions, i.e. residues 25-42. 64-79, 105-120, 150-174, 205-223, and 265-285, S 1 through S6, respectively, revealed by hydropathy analysis FIG.8. Moreover the SEQ ID NO:3 polypeptide displays a pore region of the calcium activated potassium channel, i.e., residues 245-260 which contain the consensus signature sequence of potassium channels. Heigenbothams, L., *et al.,* Biophys. J., 66(4):1061 (1994).

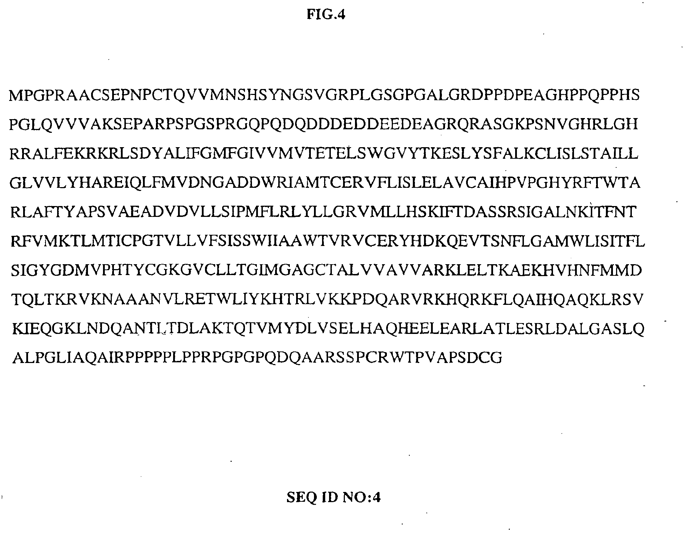

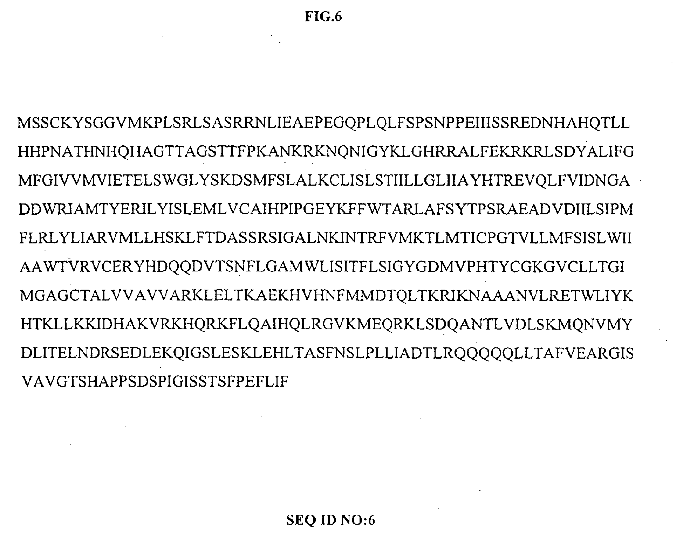

The amino acid sequence of the novel calcium activated potassium channel (SEQ ID NO:3) is about 41 % homologous to the calcium activated potassium channels (hSK1, rSK2 and rSK3) cloned from brain. Kohler, M., *et al.,* Science, 273:1709 (1996). FIG.4 shows SEQ ID NO:4 which is the amino acid residue sequence of the HSK I brain derived small-conductance calcium activated potassium channel polypeptide. FIG.5 shows SEQ ID NO:5 which is the amino acid residue sequence of the RSK2 brain derived small-conductance calcium activated potassium channel polypeptide. FIG.6 shows SEQ ID NO:6 which is the amino acid residue sequence of the RSK3 brain derived small-conductance calcium activated potassium channel polypeptide. FIG. 7 shows a comparison between the amino acid residue sequence of the novel human lymph node derived calcium activated potassium channel polypeptide described herein (SEQ ID NO:3) (designated HKCA4), and the amino acid residue sequences of the brain derived small-conductance calcium activated potassium channel polypeptides HSK1 (SEQ ID NO:4), RSK2 (SEQ ID NO:5), and RSK3 (SEQ ID NO:6). Conserved amino acid residues are boxed. Dashes represent gaps introduced to optimize the alignment. Sequences shown in this figure were produced using the multisequence alignment program of DNASTAR software (DNASTAR Inc, Madison WI).

The pore region, between residues 245-260 of SEQ ID NO:3 show significant amino acid differences from its homologs, HSK1 (SEQ ID NO:4), RSK2 (SEQ ID NO:5) or RSK3 (SEQ ID NO:6), see FIG.7, predicting different phamacology and biophysics from the brain derived calcium activated potassium channels. Kohler, M.. *et al.,* Science, 273:1709 (1996). A splice variant of the subject gene has also been identified as discussed *infra* at the region corresponding to the S2-S3 regions of SEQ ID NO:3.

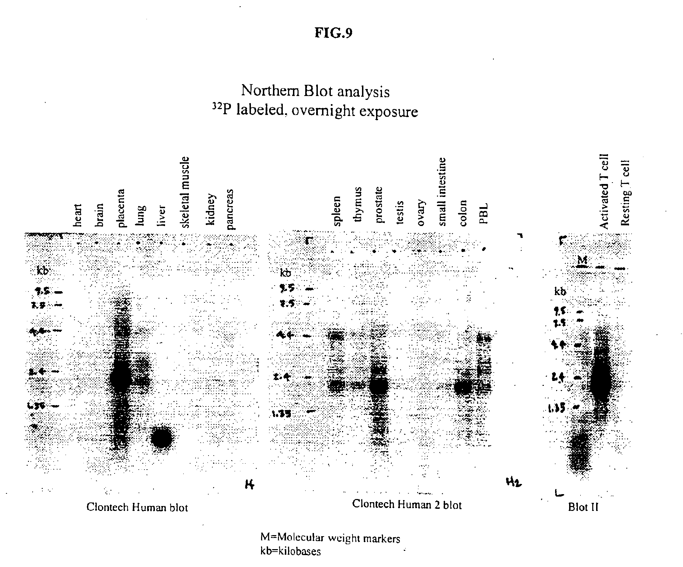

Similar to the previous description by Grissmer. S., *et al.,* wherein expression of the charybdotoxin (CTX) -sensitive calcium activated potassium channel increases dramatically after T-cell activation. J. Gen. Physiol, 102:601 (1993). The novel human lymph node derived calcium activated potassium channel polypeptide described herein (SEQ ID NO:3) is shown have high level expression, demonstrated via Northern analysis FIG.9, in activated T-cells relative to resting T-cells. FIG.9 shows a strong mRNA signal of the the novel human lymph node derived calcium activated potassium channel described herein upon T-cell activation in Northern blot analysis.

The observation of dramatic up-regulation of the novel calcium activated potassium channel upon T-cell activation provides evidence that this channel plays a crucial role in lymphocyte signalling events. In addition, high expression of the calcium activated potassium channel in prostrate, colon and placenta suggests an important role in the physiology of these tissues as well. Transcripts of the novel calcium activated potassium channel described herein are not detected in the brain. The expression of the subject channel in cells of the myeloid, lymphoid and erythroid lineages indicates that they could play a major role in modulating immune responses as well as in red blood cell and platelet physiology. Accordingly, due to the evidence of very strong mRNA signal in activated T-cells and the fact that SEQ ID NO: 1 (SEQ ID NO:7) was isolated as descibed *infra* from a lymph node library which is enriched for activated T-cells the polypeptide of the current invention appears to be an ideal pharmacological target for modulating the activity of human leukocytes and other cells of hematopoetic orign and T-cells in particular.

It is therefore probable that compounds (e.g., small-molecules, peptides, analogs, mimetics) that modulate the lymph node derived calcium activated potassium channel described herein could be used in the treatment of a variey of disease conditions including acute and chronic inflammation, asthma, allergies, graft rejection, proliferative disorders, anemias. neurodegenerative diseases with immunological components, as well as autoimmune diseases including rheumatoid arthritis, type-1 diabetes mellitus, multiple sclerosis, myasthenia gravis, systemic lupus erythematosus. Sjogren's syndrome, mixed connective tissue disease, and experimental allergic encephalomyelitis (EAE).

A gene sequence described herein (e.g., SEQ ID NO:7) which encodes an intermediate-conductance, calcium-activated potassium channel, has been isolated from a human lymph node library. The translated protein is 427 amino-acids long, has 6 transmembrane segments, S1-S6. and a pore-motif between S5 and S6. The novel protein (SEQ ID NO:3) shares 40-42% similarity at the amino acid level with three small-conductance calcium-activated potassium channels cloned from brain. SEQ ID NO:7 maps to human chromosome 19q 13.1-13.2. Northern blot analysis of primary human T lymphocytes reveals a 2.2 Kb transcript that is highly upregulated in activated compared to resting cells, concomitant with an increase in KCa currents. Transcripts of the novel gene presented herein are detected by PCR and by Northerns in placenta, prostate, thymus, spleen, colon and many cell lines of hematopoietic origin. Patch-clamp recordings of SEQ ID NO:2 -transfected HEK 293 cells reveal a large voltage-independent, inwardly rectifying potassium current with a single channel conductance of 33±2 pS in symmetrical potassium solutions. The channel is activated by intracellular calcium (Kd=270±8 nM) with a highly cooperative interaction of ~3 calcium ions per channel. hKCa4 currents are blocked by externally applied tetraethylammonium (Kd= 30±6 mM), charybdotoxin (Kd=10±1 nM) and clotrimazole (Kd=380±34 nM), but are resistant to apamin, iberiotoxin, kaliotoxin and scyllatoxin (Kd>1 µM). These properties of the cloned channel are very similar to those reported for the KCa channel in activated human T lymphocytes (Grissmer *et al,* J. Gen. Physiol. 102: 601. 1993), sequences described herein (e.g., SEQ ID NO:7; SEQ ID NO:1; SEQ ID NO:2; and SEQ ID NO:3) are example embodiments which encode this native channel type.

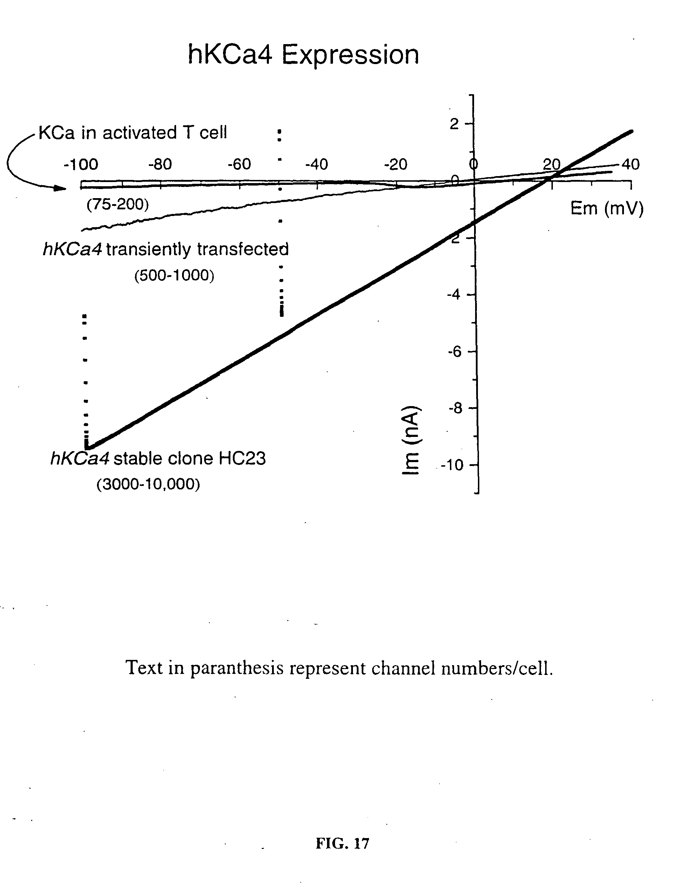

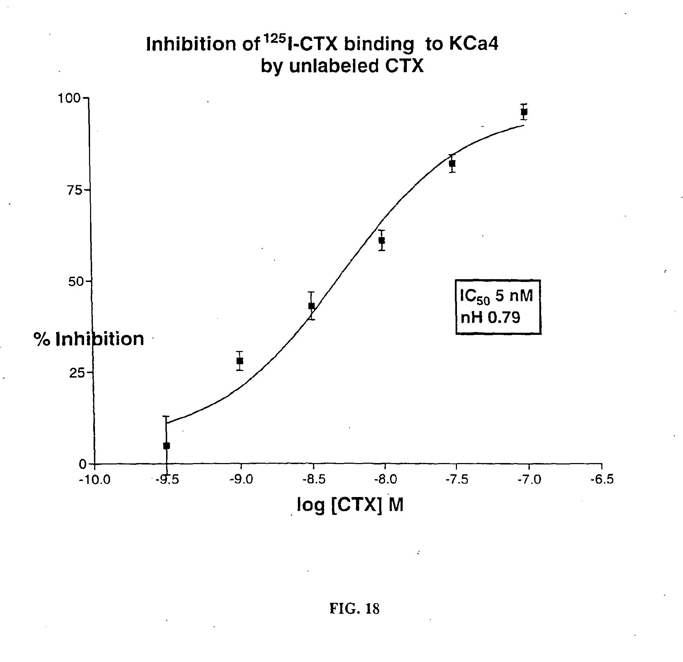

A 2.2 Kb cDNA clone described herein (e.g., SEQ ID NO: 7) has been identified from a human lymph node lambda library using a probe derived from an EST sequence that was identified from a database search for novel potassium channels. The clone has 400 bp of 5' UTR, 1.3 Kb of coding region and 540 bp of 3' UTR (fig. 17A). The entire transcript was mapped of the predominant 2.2 Kb band seen in Northerns from various tissues (see fig 18). Four independent lambda clones were identified which started with the same 5' UTR sequence (± 15 bp) and the 3' UTR ended with a poiy-adenylation signal followed by a poly-A tail. We also detected an in-frame stop codon upstream of the initiator ATG in all the 4 clones, ruling out the existence of alternate upstream initiator ATG's. The novel gene (e.g., SEQ ID NO:2) maps to chromosome 19q13.1-13.2. The translated protein comprises 427 amino acids. Hydropathy plots reveal a short intracellular N-terminus, followed by 6 transmembrane segments (S1-S6) and a long intracellular C-terminus (Fig. 17 B). The loop between S5 and S6 contains the highly conserved GYG sequence characteristic of all cloned potassium channel pores (10). The channel protein (SEQ ID NO:3) has one consensus N-linked glycosylation site between S5 and the pore and several sites for serine-threonine phosphorylation. There are no consensus E-F hand motifs in SEQ ID NO:3. A comparison of the amino acid sequence of the hKCa4 with other representative members of the K⁺ channel super-family reveals that it is most similar to the small-conductance KCa channels (hSK1, rSK2, rSK3; SEQ ID NO: 4, SEQ ID NO: 5 and SEQ ID NO: 6) recently cloned from brain, although it shares only 40-42% amino acid identity to them, warranting placement within a distinct sub-family (fig.15 C).

The novel gene described herein (e.g., SEQ ID NO:2) maps to human chromosome 19q13.1-13.2. This region of the chromosome 19 has several disease susceptibility loci, including B-cell lymphoma and Diamond Black Fan Syndrome. Abnormalities of the gene described herein may be associated with any disorders which map to this general area including disorders that are currently being mapped as part of the human genome project.

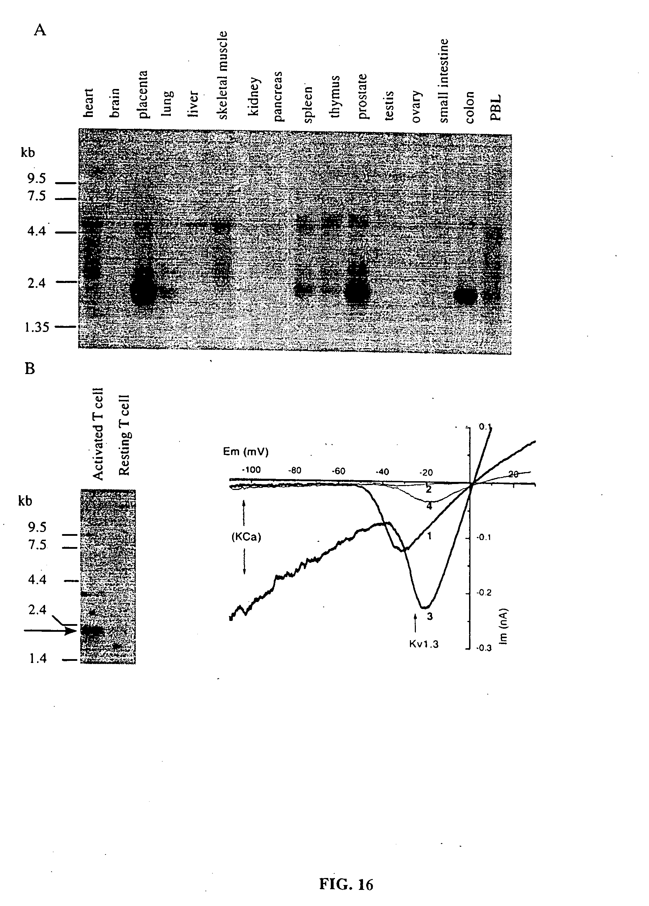

Northern blot analysis reveals a strong signal at ~2.2 Kb in activated human T lymphocytes. placenta, prostate, and colon (fig. 16A and B). Moderate signal was observed in spleen. thymus and peripheral blood leukocytes (fig. 16 A); there was no detectable signal in brain. Minor transcripts of larger sizes were also seen in some tissues (fig. 16 A).

The novel gene described herein (SEQ ID NO:2) when co-transfected with a reporter gene for GFP into HEK 293 cells, produced a calcium-dependent K⁺ current with strong inward rectification in symmetrical K⁺ solutions upon perfusion of 1 µM ionomycin (Fig. 13 A). Current induced by ionomycin was completely blocked by 100 nM CTX, but unaffected (i.e., <10% change in amplitude) by I µM apamin (n = 2). Internal dialysis with a solution containing I µM [Ca²⁺]_{free} also activated a stable large current (15.4 ± 2.4 nS slope conductance between -100 and -30 mV; n = 11) with similar characteristics. Pharmacological evaluation (Fig. 13 B) revealed that this current was potently inhibited by CTX (K_{d} = 10 ± 1 nM) and also blocked by TEA (K_{d} = 30 ± 7 mM) and Clotrimazole (K_{d} = 387 ± 34 nM), but insensitive to apamin, iberiotoxin, and kaliotoxin, and scyllatoxin (K_{d} > 1 µM, n=3 for each experiment). Control current during ramps in symmetrical 160 mM K⁺ converged with current elicited during CTX perfusion close to 0 mV (e.g., Fig. 13 A), supporting K⁺ selectivity of the channel. K⁺ selectivity was evaluated further by examining reversal potentials of control vs. CTX currents over a range of different K⁺₍ₒᵤₜ₎ concentrations (Fig. 13. C). Reversal potential shifted -57 mV per e-fold increase in K⁺₍ₒᵤₜ₎ in close agreement with the predicted Nernstian value for a K⁺-selective channel. Untransfected HEK 293 cells, or cells transfected with GFP alone, showed small currents in response to voltage ramps during dialysis with 1 µM Ca²⁺_{(free)} (conductance < 0.1 nS: n = 8), and these currents were not measurably affected by 100 nM CTX. Cell-attached recordings revealed single channel openings during perfusion with Ringer solution containing 1 µM ionomycin. Channels showed a unitary conductance (measured during voltage ramps between -120 and -30 mV of 33 + 2 pS (n = 3; Fig. 14A) with pipettes containing 160 mM K⁺, and 9 ± 1 pS with pipettes containing 4.5 mM K⁺. Pipette potentials between -100 and +20 mV had no apparent effect on the probability of channel openings (Pₒ) during ramps (e.g., Fig. 16A) or steps. The inside-out configuration in symmetrical K⁺ also revealed single channels of similar conductance with gating that clearly depended on the [Ca²⁺]_{free} at the cytoplasmic face of the patch (Fig. 14 B). The Pₒ with different [Ca²⁺]_{free} varied considerably between cells, but never exceeded ~0.5. Fitting the open probability vs. [Ca²⁺]_{free} curve revealed an activation K_{d} = 270 ± 8 nM Ca²⁺ with a Hill-coefficient of 2.7 ± 0.2, indicative of a highly cooperative interaction between calcium ions and the channel (Fig. 14 C).

The evidence demonstrated herein that the novel gene (e.g., SEQ ID NO:2) encodes the calcium-activated potassium channel in T lymphocytes is summarized as follows: (a) the clone was identified from a lymph node library which is enriched for activated T cells (b) Northern blot analysis of resting and activated T cells reveals ~ 10-fold up-regulation of transcript levels, corresponding to an increase in current levels (FIG.16 B) (c) the biophysical and pharmacological properties of the currents are very similar to those reported for T cells (Grissmer, S., et al., 102:601 (1993); Verheugen, J. A. H., *et al.,* Cell Calcium 21:1 (1997)), including inward rectification in high K solution, block by CTX and TEA, single-channel conductance, and Kd for calcium-dependence (FIG.14 D). Furthermore, SEQ ID NO:2 quite possibly may encode the KCa channels found in other cells of hematopoietic origin, including erythrocytes, monocytes, B cells, neutrophils and eosinophils. The reported electrophysiological properties of the channel from these cells is compatible with the data presented herein, for example SEQ ID NO:2 heterologously expressed in HEK 293 cells. Moreover, a 1 Kb band was amplified using SEQ ID NO:7-specific primers, for example, and confirm its sequence from many cell-lines of hematopoietic origin (e.g.: U937, HL-60, fetal liver cells, Jurkats). Subtle changes in reported properties is attributable to differences in post-translational modifications and/or association with accessory subunits in different cell-types.

The significant up-regulation of the novel transcript levels during T cell activation highlights the importance of these channels in early-activation and post-activation immune responses. Engagement of the T cell receptor by mitogen or antigen evokes an increase in intracellular calcium concentrations, leading to membrane hyperpolarizations (rather than depolarization) caused by opening of KCa channels. This event in turn maximizes the electrical driving force for calcium influx through calcium-release activated channels, facilitating sustained calcium oscillations required for cell proliferation , cytokine production and the expression of immune function. Blockade of both KCa and Kv channels have been reported to cause profound inhibition of T cell proliferation (Rader, R. K. , *er al.,* J. Immunol. 156:1425 (1996)) while blockade of KCa channels is sufficient to prevent secondary immune responses (Verheugen, J. A. H., *et al.,* Cell Calcium, 21: 1 (1997)).

### The calcium activated potassium channel subunit (SEQ ID NO:3) (hKCA4) transcipt is present in microglial cells

Microglia are resident macrophages in the brain that play vital roles in neuroinflammatory responses and in neurodegenerative diseases (Wood, P.L., *Role of CNS macrophages in neurodegenerarion in Neuroinflammation: mechanism and management,* edited by PL Wood, Human Press Inc., Totowa, NJ). In view of SEQ ID NO:2 expressed in several cells of hematopoietic origin, including monocytes and macrophages, we asked whether it could be present in microglia. A predicted 800 bp band was obtained from microglial cDNA, and sequence was confirmed, indicating the presence of the transcript in brain microglial cells. *See* EXAMPLE XIX.

### Calmodulin is an interaction partner for the novel calcium activated potassium channel subunit (hkca4) and is possibly the calcium sensor

The small- and intermediate-conductance calcium-activated potassium channels KCa2, KCa3, KCa4 belong to a distinct sub-family of K⁺ channels that play crucial roles in hyperpolarization of excitable and non-excitable cells. Despite their exquisite calcium sensitivity, the protein coding region of these channels do not contain any consensus calcium-bowl or EF-hand motifs. To investigate whether an accessory protein is involved in calcium sensing, we used the 146-a.a. C-tail of hKCa4 as a bait to screen an activated T-cell library in a yeast-two-hybrid system (discussed *infra*). Several of the positive clones encoded for calmodulin. See. Vogel. H.. The Merck Frosst Award Lecture 1994, *Calmodulin: a versatile calcium mediator protein.,* Biochem Cell Biol., 72(9-10):357 (1994).

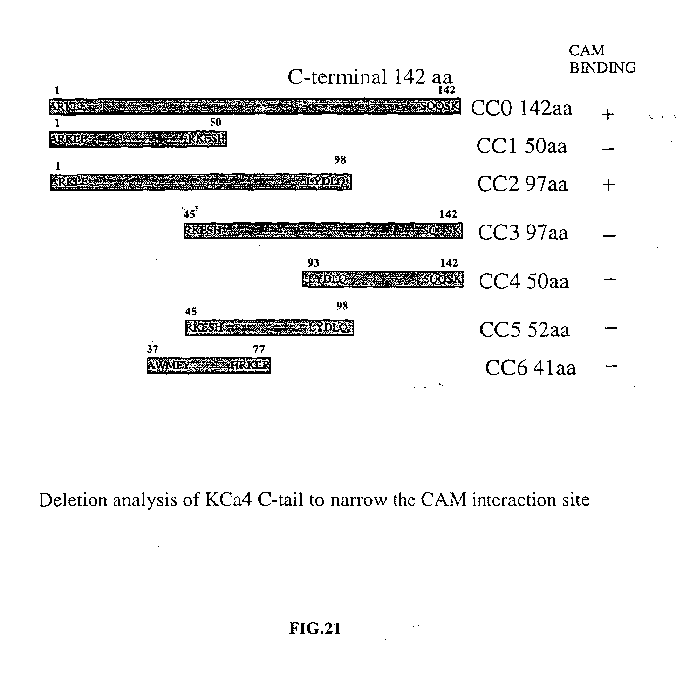

GST-fusion pull-down experiments have confirmed the interaction and deletion analysis have identified the first 97 amino acids in the C-tail of hKCa4 as being required for calmodulin binding (FIG.21). This region is highly conserved among all SKCa channels and appears to contain hydrophobic and positively charged amino acids representative of calmodulin binding domains. The interaction of calmodulin with the C-tail does not appear to require calcium and suggests that calmodulin could be pre-bound to the channel on the membrane. The SEQ ID NO:3 calcium activated potassium channel (hKCa4) currents in HEK 293 cells are inhibited (50-60% at 10 uM) by the calmodulin antagonist W7. Taken together, our data point to the fact that calmodulin quite possibly may serve as the calcium sensor for this class of channels.

### Embodiments

The present invention relates to nucleic acid (e.g., SEQ ID NO:2) and amino acid sequences (SEQ ID NO:3) of a novel human lymph node derived calcium activated potassium channel and variations thereof and to the use of these sequences to identify compounds that modulate the activity of human leukocytes, as described *infra.*

The invention further relates to the use of the novel calcium activated potassium channel in expression systems as assays for agonists or antagonists of calcium-activated potassium channels. The invention also relates to the diagnosis, study, prevention, and treatment of disease related to dysfunctional leukocytes.

Polynucleotide sequences which encode the human leukocyte calcium activated potassium channel SEQ ID NO:3 or a functionally equivalent derivative thereof may be used in accordance with the present invention which comprise deletions, insertions and/or substitutions of the SEQ ID NO:2 nucleic acid sequence. Biologically active and/or pharmacologically active variants of the calcium activated potassium channel subunit of the present invention may also be comprised of deletions, insertions or substitutions of SEQ ID NO:3 amino acid residues. A purified polynucleotide comprising a nucleic acid sequence encoding the polypeptide having the sequence substantially as depicted in SEQ ID NO:3 or a biologically active fragment thereof is a particularly preferred embodiment of the present invention.

Amino acid substitutions of SEQ ID NO:3 may be made, for instance, on the basis of similarity in polarity, charge, solubility, hydrophobicity, hydrophilicity, and/or the amphipathic nature of the residues as long as the biological activity of the calcium activated potassium channel is retained. For example, negatively charged amino acids include aspartic acid and glutamic acid; positively charged amino acids include lysine and arginine; and amino acids with uncharged polar head groups having similar hydrophilicity values include leucine, isoleucine, valine; glycine, alanine; asparagine, glutamine: serine, threonine phenylalanine, and tyrosine.

Nucleic acid sequences which encode the amino acid sequence of the calcium activated potassium channel described herein are of an exponential sum due to the potential substitution of degenerate codons (different codons which encode the same amino acid). The oligonucleotide sequence selected for heterologous expression is therefore preferably tailored to meet the most common characteristic tRNA codon recognition of the particular host expression system used as well known by those skilled in the art.

Suitable conservative substitutions of amino acids are known to those of skill in this art and may be made without altering the biological activity of the resulting polypeptide, regardless of the chosen method of synthesis. The phrase "conservative substitution" includes the use of a chemically derivatized residue in place of a non-derivatized residue provided that such polypeptide displays the desired binding activity. D-isomers may also be substituted for the naturally occurring amino acids. Substitutions are preferably, although not exclusively, made in accordance with those set forth in TABLE 1 as follows:

**TABLE 1**

| **Original residue** | **Example conservative substitution embodiments** |
|---|---|
| Ala (A) | Gly; Ser; Val; Leu; Ile; Pro |
| Arg (R) | Lys; His: Gln; Asn |
| Asn (N) | Gln; His; Lys; Arg |
| Asp (D) | Glu |
| Cys (C) | Ser |
| Gln (Q) | Asn |
| Glu (E) | Asp |
| Gly (G) | Ala, Pro |
| His (H) | Asn; Gln; Arg; Lys |
| Ile (I) | Leu; Val; Met: Ala; Phe |
| Leu (L) | Ile; Val; Met; Ala; Phe |
| Lys (K) | Arg; Gln; His; Asn |
| Met (M) | Leu; Tyr; Ile; Phe |
| Phe (F) | Met; Leu; Tyr; Val; Ile; Ala |
| Pro (P) | Ala; Gly |
| Ser (S) | Thr |
| Thr (T) | Ser |
| Trp (W) | Tyr; Phe |
| Tyr (Y) | Trp; Phe; Thr; Ser |
| Val (V) | Ile; Leu; Met; Phe; Ala |

The nucleotide sequences of the present invention may also be engineered in order to alter a coding sequence for a variety of reasons, including but not limited to, alterations which modify the cloning, processing and/or expression of the gene product. For example, mutations may be introduced using techniques which are well known in the art, eg, site-directed mutagenesis to insert new restriction sites, to alter glycosylation patterns, to change codon preference, etc.

Dominant negative mutant embodiments of the channel subunit are contemplated herein (*see,* FIG.15 for site change embodiments (sites for glycosylation are indicated by a triangle, serine-threonine phosphorylation sites are circled) (e.g., change to D or R), *any* changes in the pore region are preferred embodiments as dominant negative mutants) as pharmacologically valuable agents in order to attenuate the corresponding channel biological activity *in vivo.*

Generally mutated versions, or site directed mutagenesis species embodiments of the pore region of SEQ ID NO:2, for instance, can be used to produce non-functional calcium activated potassium channels to be subsequently over-expressed in heterologous cells, preferrably ones of hematopoetic origin, to knock-out endogenous channels by dominant-negative suppression of the channel. Ribera, A.B., J. Neurosci., 16:1123, (1996); Babila, T., *et al.,* Neuron, 12:615 (1994).

Included within the scope of the present invention are alleles of the calcium activated potassium channel gene of the present invention. As used herein, an allele or allelic sequence is an alternative form of the subunit herein. Alleles result from nucleic acid mutations and mRNA splice-variants or longer transcripts which comprise SEQ ID NO:2 which produce polypeptides whose structure or function may or may not be altered.

Particularly preferred embodiments of the present invention are host cells transformed with a purified polynucleotide comprising a nucleic acid sequence encoding the polypeptide having the sequence substantially as depicted in SEQ ID NO:3 or a biologically active fragment thereof. Cells of this type_or preparations made from them may be used to screen for pharmacologically active modulators of the novel calcium activated potassium channel activity using methods which are well known in the art. U.S. Patent No. 5,397,702, *Assay For and Treatment of Autoimmune Diseases,* issued March 14, 1995; U.S. Patent No. 5.637,470, *Screening array using cells expressing recombinant alpha and beta subunits of the mammalian large-conductance (maxi-K) potassium channel,* issued June 10, 1997; U.S. Patent No. 5,607,843, *Nucleic Acid Sequence Encoding Apamin Binding Protein.* issued March 4, 1997; and U.S. Patent No. 5,602,169, *3-substituted oxindole derivatives as potassium channel modulators,* issued Feb. 11, 1997.

Such modulators are potentially useful in treating disease which is manifested by dysfunctional leukocytes including but not limited to acute and chronic inflammation, asthma, allergies, graft rejection, proliferative disorders, anemias, neurodegenerative diseases with immunological components, as well as autoimmune diseases including rheumatoid arthritis, type-1 diabetes mellitus, multiple sclerosis, myasthenia gravis, systemic lupus erythematosus, Sjogren's syndrome, mixed connective tissue disease, and experimental allergic encephalomyelitis (EAE).

As used herein, a "functional derivative" of the calcium activated potassium channel disclosed herein is a compound that possesses a biological activity (either functional or structural) that is substantially similar to SEQ ID NO:3. The term "functional derivatives" is intended to include the "fragments," "variants," "degenerate variants," "analogs" and "homologues", and to "chemical derivatives". The term "variant" is meant to refer to a molecule substantially similar in structure and function to either the entire calcium activated potassium channel molecule or to a fragment thereof. A molecule is "substantially similar" to the calcium activated potassium channel polypeptide if both molecules have substantially similar structures or if both molecules possess similar biological activity. The term "analog" refers to a molecule substantially similar in function to either the entire calcium activated potassium channel polypeptide, or to a fragment thereof.

The cloned calcium activated potassium channel DNA obtained through the methods described herein may be recombinantly expressed by molecular cloning into an expression vector containing a suitable promoter and other appropriate transcription regulatory elements, and transferred into prokaryotic or eukaryotic host cells to produce the calcium activated potassium channel polypeptide. Techniques for such manipulations are fully described in Sambrook. J., et al.. Molecular Cloning Second Edition, 1990, Cold Spring Harbor Press and are well known in the art.

Expression vectors are defined herein as DNA sequences that are required for the transcription of cloned copies of genes and the translation of their mRNAs in an appropriate host cell. Such vectors can be used to express nucleic acid sequences in a variety of hosts such as bacteria, bluegreen algae, plant cells, insect cells, fungal cells, human, and animal cells. Specifically designed vectors allow the shuttling of DNA between hosts such as bacteria-yeast, or bacteria-animal cells, or bacteria-fungal cells, or bacteria-invertebrate cells.

### In vitro synthesis of capped mRNA

The full-length cDNA (SEQ ID NO: 1), may be used in standard procedures to synthesize biologically active mRNA for functional expression in heterologous cells, in *Xenopus* oocytes, for instance, or in various types of mammalian cells. See, e.g., Goldin A, Methods Enzymol. 207:279 (1992).

The coding region for the novel calcium activated potassium channel described herein (e.g., a region which comprises SEQ ID NO:2) which encodes the polypeptide having a bio-active sequence substantially as depicted in SEQ ID NO:3 or a biologically active fragment thereof may be cloned 3', for example, to a bacteriophage promoter, e.g., an SP6, T7 or T3 promoter. The PGEM vectors from Promega, Madison, WI, are examples of preferred vectors which may be used with the present invention. Standard vectors known in the art, such as pSP64T or pBSTA, which enhance stabililty of the message and increase specific expression in *Xenopus* oocytes, are especially preferred. Kreig and Melton , Nucleic Acids Res., 12:7057 (1984). These particular preferred vectors contain the Xenopus beta-globin 5' and 3' untranslated mRNA regions flanking the 5'end and a poly-A tail on the 3' end of the gene.

A plasmid vector DNA construct which contains an insert which encodes the novel calcium activated potassium channel described herein or a biologically active fragment thereof (e.g., a SEQ ID NO: 1 or SEQ ID NO:7 region which comprises SEQ ID NO:2, or a truncated version thereof) may be then cut with a restriction enzyme to linearize the construct 3' to the structural coding region. The linearized vector should be extracted with phenol-chloroform-isoamyl alcohol, precipitated with ethanol and re-suspended in RNAse-free water for use as a transcription template. The transcription reaction, for example, may be carried out as described *infra* to synthesize biologically active mRNA for subsequent *in vivo* or *in vitro* translation by methods which are well-known in the art. The mMessage mMachine^{™} kit, AMBION, Austin, TX, is especially preferred for synthesizing biologically active mRNA. Alternately, the mRNA transcripts may be used as probes for analysis of tissue distribution ny Northern analyses and or RNAse protection assays.

### mRNA SYNTHESIS

| PGEM, Promega, Madison, WI | |
|---|---|
| 10x SP6/T7 buffer | 5 ul |
| 10X ATP, CTP. UTP (5 mM each) | 5 ul |
| 10x GTP (5 mM) | 1 ul |
| 10x GpppG (Cap; 5 mM) | 5 ul |
| DTT (1M) | 0.5 ul |
| Rnase inhibitor (40U/ul) | 1.25 ul |
| Water | 27 ul |
| Linearized DNA (1 ug/ul) | 1-5 ul |
| SP6 or T7 RNA polymerase (20U/ul) | 1-3 ul |
| TOTAL REACTION VOLUME | 50 ul |

Incubate at 37 degrees for 1-2 hours. Add 1 ul of RNAse-free Dnase to degrade template DNA. Digest for 10 minutes. Add 75 ul of water. Extract with phenol/CHCl3. Ethanol precipitate RNA. Store in aliqots at -70 degrees.

### Functional expression

Biologically active mRNA can be introduced into heterologous cells for functional expression and analyses by methods well-known in the art. Synthetic mRNA from example constructs described *supra,* for example, may be injected into *Xenopus* oocytes for functional expression and analyses. Goldin, A., Methods Enzyml.,207:266, (1992). Hererologous calcium-activated potassium channels may be examined using standard two-electrode voltage clamp techniques. *See, e.g.,* Stuhmer, W., Methods in Enzymol., 207:319, (1992); Kohler, *et al.,* Science, 273:1709 (1996). Calcium concentrations inside the cell may be altered, for example, by adding Ionomycin, a calcium-ionophore, co-expression with a G-protein coupled receptor that causes a rise in intracellular calcium. Calcium concentrations may alternately be altered by pulling inside-out patches and changing calcium concentrations in the bath medium. Grissmer, S., *et al.. Calcium-activated Potassium Channels in Resting and Activated Human T Lymphocytes,* J. Gen. Physiol, 102:601 (1993). Standard biophysical parameters, such as activation, calcium dependence, single-channel conductance, inactivation, tail currents, potassium selectivity, and thorough pharmacology of various K channel blockers including TEA. Charybdotoxin, Apamin, and others may also be tested. Grissmer, S., *et al., Calcium-activated Potassium Channels in Resting and Activated Human T Lymphocytes,* J. Gen. Physiol, 102:601 (1993).

Alternatively, cRNA (synthetic mRNA from a cDNA construct) can be introduced into heterologous mammalian cells, for example, RBL cells (ATCC # CRL 1378), and 293 cells (ATCC # CRL 1573), may be transformed using standard art-methods. For example, the Eppendorf microinjection system may be used (Micromanipulator 5171 and Transjector 5242). Transformed cells may be analysed for calcium-activated K currents about 4 hours later using patch-clamp techniques which are well-documented. *E.g.,* Ikeda, *et al.,* Pfueg. Arch. Eur. J. Physiol., 422:201 (1992); Grissmer, *et al..* J. Gen. Physiol., 102:601 (1993).

### Over-expression of the novel channel in cell-lines

Transient and/or stable eucaryotic transfectant cells comprised of the coding region(s) described herein are contemplated for high-level expression of the novel calcium activated potassium channel.

Eucaryotic transfectants are preferred embodiments of the present invention for employment in pharmacological target binding studies for the identification molecules which block the novel channel described herein *in vivo.* HEK cells are particularly preferred.

Transient expression of coding regions for the novel channel can be achieved by straight transfection into mammalian cells, by standard techniques. Omari, K. *et al.,* J. Physiol., 499:369, (1997); Panyi, G. *et al.,* J. Gen. Physiol.. 107(3):409 (1996). High level transient expression may be achieved using standard viral systems, e.g.. Baculovirus. Adenovirus, or Vaccinia virus. Channel numbers resulting from these systems are typically 5-500K per cell. Kamb, A., Methods Enzymol. 207:423 (1992); Sun, T. *et al.,* Biochemistry, 33(33):9992 (1994); Spencer, R.H., *et al.,* J. Biol. Chem., 272:2389 (1997). See, EXAMPLE XV, Baculovirus expression system for hKCa4 (SEQ ID NO:2).

Stable transfection of heterologous cells using sequences which encode the novel calcium activated potassium channel described herein (SEQ ID NO:3) or biologically active variations or fragments thereof can be generated using, for example. NIH-3t3, L929, COS, HEK, or CHO cells. *See, e.g.,* EMBO, 11 (6):2033 (1992), Grissmer, *et al.,* Mol. Pharm., 45:1227 (1994).

A preferred vector for use with the present invention is pcDNA/Neo, which is commercially available from INVITROGEN, Carlsbad, CA.

Cells, NIH-3t3. for example, are grown to 50% confluency in 60mm plates (media, and conditions are according to requirements of the particular cell line) and transfected with 5 ug of pure DNA comprising a coding region for the novel calcium activated potassium channel, e.g. SEQ ID NO:2, in pCDNA/Neo using the Lipofection reagent, as described by the supplier (LIFE TECHNOLOGIES Gibco BRL, Bethesda, MD). After transfection, the cells are incubated at 37°C, conditions for 3 days in medium with 10% FCS. Cells are trypsinized seeded onto 100mm dishes, and then selected with 300ug/ml of G418 (Neomycin). Only cells that have stable integration of the heterologous coding region will grow in the presence of G418, which is confered by the Neomycin-resistance gene in the plasmid. Isolated clones are processed for 2-3 rounds of purification and subjected to patch-clamp analysis for Kca currents.

A variety of mammalian expression vectors may be used to express the recombinant calcium activated potassium channel polypeptide disclosed herein in mammalian cells. Commercially available mammalian expression vectors which are suitable for recombinant expression, include but are not limited to, pcDNA3 (Invitrogen), pMC I neo (Stratagene), pXT 1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVept (ATCC 37199), pRSVneo (ATCC 37198), pSV2-dhfr (ATCC 37146), pUCTag (ATCC 37460), and IZD35 (ATCC 37565).

A variety of insect cell expression vectors may be used to express recombinant calcium activated potassium channel polypeptide in insect cells along with the alpha subunit. Commercially available insect cell expression vectors which may be suitable for recombinant expression of beta subunit include but are not limited to pBlue Bac III (INVITROGEN).

Eukaryotic recombinant host cells are especially preferred. Examples include but are not limited to yeast, mammalian cells including but not limited to cell lines of human, bovine, porcine, monkey and rodent origin, and insect cells including but not limited to Drosophila and silkworm derived cell lines. Cell lines derived from mammalian species which may be suitable and which are commercially available, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616),BS-C-1 (ATCC CCL 26) and MRC-5 (ATCC CCL 171).

The calcium activated potassium channel may also be expressed as a recombinant protein with one or more additional polypeptide domains added to facilitate protein purification. Such purification facilitating domains include, but are not limited to, metal chelating peptides such as histidine-tryptophan modules that allow purification on immobilized metals (Porath, J., Protein Expr Purif., 3:263 (1992)), protein A domains that allow purification on immobilized immunoglobulin, and the domain utilized in the FLAGS extension/affinity purification system (Immunex Corp, Seattle WA). The inclusion of a cleavable linker sequences such as Factor XA or enterokinase (Invitrogen, San Diego CA) between the purification domain and TMP is useful to facilitate purification.

### Glutathione S-Transferase (GST) Fusion Proteins, in vitro Translation of Calmodulin, and Binding of Calmodulin to GST Fusion Proteins

GST fusion plasmids were based on PGEX-6P-1 (Pharmacia, Piscataway, NJ) and were constructed by conventional means using standard PCR with primer to the C-terminal of KCa4 sequence with the appropriate restriction site, EcoRI site and XhoI site in this case. Positions 1252-1272 of SEQ ID NO: 1 is the sense oligonucleotide and SEQ ID NO: I Positions 1660-1680 is the antisense oligonucleotide for the full length hKCa4 C-terminal GST construct. After transfection into *Escherichia coli* BL21, synthesis of the fusion proteins was induced with 0.2 mM isopropyl b-D-thiogalactoside (IPTG) in a liquid culture grown to OD of 1.0. After 2-3 hr at 37°C, the cells were collected by centrifugation, resuspended in NETN lysis buffer (0.5% Nonidet P-40/1 mM EDTA/20 mM Tris-HCl, pH 8.0/100 mM NaCl; 1.0 ml per 20 ml of culture) and lysed by sonication. The lysate was cleared by centrifugation at 10,000 *x* g for 10 min at 4 °C. The GST fusion proteins in the supernatant were adsorbed for 30 min at room temperature to glutathione-agarose beads in NETN [1 volume of lysate: 1 volume of 50% (vol/vol) slurry of agarose-GSH beads (Pharmacia) in NETN]. The last wash was with binding buffer [1% (vol/vol) Polyoxyethylene 9 Lauryl Ether/2 mM EDTA/100 mM NaCI/20 mM Tris-HCI, pH 8.01 instead of NETN]. Slurries [50% (vol/vol)] slurries of washed agarose-GSH beads with GST fusion proteins adsorbed to them (agarose-GSH::GST fusions) were then incubated for 30 min at room temperature in an equal volume of binding buffer with ³⁵S-labeled calmodulin, synthesized by coupled transcription-translation (Cat#L4610, Promega, Madison, WI), as described (Pragnell, *et al.,* Nature, 368:67 (1994). After a 50-fold dilution in binding buffer, the beads were centrifuged and washed three times with binding buffer and resuspended in an equal volume of 4x Laemmli's sample buffer. Proteins released from the beads were analyzed by 12% SDS/PAGE followed by autoradiography to detect retention of calmodulin by the fragments fused to GST. *In vitro*-translated luciferase was used to test for specificity in the interaction of calmodulin with the KCa4 C-terminal fragments.

### Stable mammalian cell expression of hkca4 for assay development

Heterologous expression of cloned channels is a pre-requisite for assay development, particularly preferred binding assays which require high density of pure channels. The first step towards assay development for the novel channel subunit (hKCa4) (SEQ ID NO:3) was therefore the generation of a high-level expression system. A stable transfectant of the novel gene described herein was developed in HEK 293 cells.

SEQ ID NO:2 was subcloned into mammalian cell expression vectors to generate both stably integrated and episomally replicating lines. For stable lines, the (pGEN IRES-neo) construct and the pcDNA3 construct is used (described in JBC. 272 (52): 32723). The pGEN IRES-neo vector was made by cleaving a 1.3 kb SmaI/Scal fragment containing the coding region (SEQ ID NO:2) of the calcium activated potassium channel This fragment was subcloned into the vector at the SmaI site. This cloning strategy results in a construct that uses the authentic initiator methionine.

For the episomal lines, SEQ ID NO:2 was first subcloned into pFastBac 1 from the pcDNA3 construct using a strategy that eliminates the three upstream fusion amino acids. A 1101 bp NcoI fragment was cleaved from the pcDNA3 construct and cloned into the NcoI site of pBlueBac III (Invitrogen, Carlsbad, CA). A colony was identified whose orientation contained the BamHI site from the pBlueBac III multiple cloning region on the 5' end of the cDNA (hKCa4) fragment. The 5' end of the cDNA was then cleaved from pBlueBac III using BamHI (5') and EcoRV (internal to hKCa4). The 3' end of the gene was provided by cleaving the pcDNA3 construct with EcoRV and XhoI (3'). The reconstructed coding region was cloned into the BamHI (5') and Xhol (3') sites of the pFastBac 1 vector (Life Technologies, Gaithersburg, MD). This cloning strategy eliminated the N-terminal MGA created by cloning into pcDNA3. The coding region of the calcium activated potassium channel subunit was then cleaved from this pFastBac 1 construct using KpnI (5') and NotI (3'), and cloned into the KpnI and NotI sites of pCEP4 (Invitrogen, Carlsbad, CA).

HEK293 cells were cultured in DMEM medium containing 10% fetal bovine serum, 100 units per mL of penicillin and 100 µg, per mL of streptomycin. CHO-K1 cells were cultured in Ham's F12 medium containing 10% fetal bovine serum. Transfections into HEK293 cells were achieved using the LipoTAXI mammalian transfection kit (cat # 204110) from Stratagene (La Jolla, CA), and, separately, a modified calcium phosphate transfection protocol (see EXAMPLE XIII). LipoTAXI transfections were according to the protocol, with the following notes: 1 mL of serum-free DMEM was mixed with 70 µL transfection reagent, and 15 µg of either (pGEN IRES-neo) or pcDNA3 - hKCa4 construct was added. The DNA/lipid complexes were allowed to form for 20 minutes at room temperature. The cells were incubated with the DNA/lipid complexes for four hours at 37°C, 5% CO₂. Ten mL of DMEM containing 20% fetal bovine serum (FBS) were added and the cells were grown overnight. Calcium phosphate transfections followed the attached protocol (EXAMPLE XIII). The episomal lines in HEK293 cells were generated using the pCEP4/SEQ ID NO:2 construct and the modified calcium phosphate method according to the protocol (EXAMPLE XIII).

Transfections into CHO-K1 cells were achieved using Cellfectin reagent from Life Technologies, Gaithersburg, MD, (cat # 10362-010), according to EXAMPLE XIV.

For all lines, media was removed and replaced with fresh the morning after the transfection. Forty eight hours later (i.e., three days after the transfection), cells were split as follows: HEK293 stable lines, 1:10; HEK293 episomal lines, 1:4; CHO-K1 stable lines, 1:4. Twenty four hours after the split (i.e., four days after the transfection), stable lines were subjected to medium containing 1 mg/mL G418 sulfate (MediaTech, Inc., Herndon, VA, cat # 30-234-CR; or Life Technologies, Gaithersburg, MD, cat # 10131-035) and the HEK293 episomal lines were subjected to medium containing 250µg/mL hygromycin B (Calbiochem, San Diego, CA, cat # 400051). The HEK293 episomal lines were not cloned, but rather were maintained as four separate populations. For all stable lines, colonies were picked after 10 days in selective medium using sterile cloning discs (Research Products International, Mount Prospect, IL, cat. # 248710, according to their protocol (see attached protoctol #3) Clones were expanded as per protocol and characterized by Northern blot analysis and ¹²⁵I-CTX binding.

### RNA dot-blot assay for screening positive clones

Initial characterizations were done by seeding HEK293 stable or episomal lines in two duplicate 96 well plates and growing to confluency. One plate was processed for crude total RNA by rinsing the cells with 250 µL of phosphate buffered saline and lysing the cells directly in the well with 100 µL per well of RLT buffer plus β-mercaptoethanol from Qiagen, Inc., Chatsworth, CA (cat # 79216). The lysates were vacuumed onto Hybond N+ positively charged nylon membrane (Amersham Life Science, Inc., Arlington Heights, IL, cat # RPN203B) that had been soaked in 2 X concentrated SSC solution (20 X SSC is 0.3 M Na₃ citrate and 3 M NaCl at pH 7.0). The wells were rinsed 2 to 3 times in 2 X SSC and the blot was crosslinked on a Spectrolinker XL1500 (Spectronics Corp., Westbury, NY) under the optimal setting. The vacuum apparatus used was the Bio-dot 96 well apparatus, Bio-Rad Laboratories, Hercules, CA, cat. # 170-6545. The probe used to hybridize the blot was a PCR product corresponding to nucleotide # 658-1098 of hKCa4. Fifty ng of fragment was labeled using α³²P-dCTP (NEN Life Science Products, Inc.. Boston, MA, cat. # NEG-013H) and Ready-To-Go labeling beads (-dCTP) (Pharmacia Biotech, Piscataway, NJ, cat # 27-9240-01) according to the manufacturer's recommendations. Labeled product was purified from unincorporated radioactive nucleotide using Probe Quant G-50 Micro Columns (Pharmacia Biotech, Piscataway, NJ, cat # 27-5335-01) according to the manufacturer's recommendations. The blot was prehybridized in ExpressHyb solution (Clontech, Palo Alto, CA, cat # 8015-1) for 1.5 hours at 68°C. Twenty five ng (28 X 10⁶ cpm) of probe was denatured by boiling and added to 10 mL of fresh prewarmed ExpressHyb. This replaced the prehybridization solution for one hour at 68°C. The blot was washed twice for twenty minutes at room temperature in 2 X SSC and 0.1% SDS, followed by two fifteen-minute washes at 50°C in 0.1 X SSC and 0.1 % SDS. The blot was then exposed to film overnight with two intensifying screens.

### CTX-Binding Assay For Screening Positive Clones

The second plate was processed for ¹²⁵I-CTX binding by rinsing each well in 250 µL of phosphate buffered saline and detaching the cells using 50 µL per well of versene (Life Technologies, Gaithersburg, MD, cat # 15040-066). The cells were pipetted to break up clumps and to ensure they were detached, and the cells were then centrifuged five minutes at 1000 rpm to bring the cells to the bottom of the wells. Supernatant was carefully removed and the cell pellets were stored at -20°C until the binding assay was performed. To begin the experiment, plates were allowed to thaw to room temperature and 200 ul of assay buffer (5mM NaCl, 5mM KCl, 10mM HEPES , 6mM glucose, pH 8.4) containing 0.02% bovine serum albumin was added to each well. 50ul of ¹²⁵I-charybdotoxin (Dupont New England Nuclear, Boston, MA, Cat # NEX-276) was added to each well for a final concentration of 50 pM. The plates were incubated for 1 hour at room temperature with gentle agitation on a Titertek plate mixer. After one hour, bound ligand was separated from free by filtration onto GF/C Unifilters (Packard, Meriden, CT) that had been pre-soaked in 0.6% polyethylenimine. The samples were washed twice rapidly, using ice cold wash buffer (200mM NaCl, 20mM HEPES, pH 8.0). The Unifilter plates were air-dried overnight. When dry, 25 ul of Microscint-20 was added to the Unifilters, and the plates were counted in a Top Count scintillation counter (Packard, Meriden, CT).

Cell lines that appeared positive for both SEQ ID NO:2 RNA transcript and ¹²⁵I-CTX binding were characterized further by whole cell patch clamp analysis.

### Patch clamp recording of stable transfectants:

Currents were recorded with an Axopatch 200A amplifier (Axon Instr., Foster City, CA) using the whole cell and inside-out configurations (Hammill, *et al.,* Pfugers Arch., 391:85 (1981)). Thin-wall borosilicate glass pipettes were fabricated, sylgarded, and fire-polished to a DC resistance of 2 - 8 MΩ. The resistance of patch seals was >10 GΩ. Liquid junction potentials were corrected for in all experiments, and series resistance compensation of >70% was used where maximal current was >0.5 nA. Voltage clamp protocols were implemented and data acquisition performed with pClamp 6.0 software (Axon Instr., CA). Currents were low-pass filtered at (-3 db at 1 kHz) and then digitized at 3-8 kHz as computer files with a TL-1 interface (Scientific Solutions, Solon, OH). Currents were measured with p-Clamp software, and iterative curve fittings were performed with either p-Clamp or Origin software (Version 3.73; Microcal Inc., Northampton, MA).

For *whole cell recording,* the pipette solution contained (in mM) 160 K aspartate. 2 MgCl₂, 5 HEPES, and 1.6 EGTA with either 0.8 CaCl₂ (calculated [Ca²⁺] _{free} = 100 nM; Eqcal software, Biosoft Corp, Cambridge. UK) or 1.6 CaCl₂ (calculated [Ca²⁺ ]_{free} = 1 µM) at pH 7.2 (by KOH) and osmolality ~315 mOsm (by sucrose). Cells were perfused locally with a solution containing 160 KCl, 2 CaCl₂, 1 MgCl₂, 5 HEPES, and 5 glucose at pH 7.4 (by KOH) and osmolality -325 mOsm. In K⁺ selectivity experiments, equimolar Na⁺ was substituted for K⁺. Whole-cell voltage clamp protocol evoked current by 273 ms voltage ramp from -100 to +40 mV. Slope conductance was measured during voltage ramp between -99 to -50.1 mV. Slope conductance of Charybdotoxin block at 100 or 300 nM was subtracted from control current as leak.

Channel numbers were calculated using the formula :
(Whole-cell conductance / Unitary conductance) /(open probability of 0.5). Cell-attached voltage clamp protocol evoked single-channel current by 500 ms voltage ramp from -120 to +180 mV. Unitary conductance was measured during voltage ramp between -99 to +20 mV. Several stable transfectant clones expressed huge KCa currents. A current trace from an example clone (HC23) is shown in FIG. 17. This current was blocked by CTX (¹²⁵I charybdotoxin binding assay for KCa4 (SEQ ID NO:3) *(See* examples I, II and III)). An example of a dose-response curve for CTX-binding on KCa4 (SEQ ID NO:3) channels is shown in FIG.18.

### Human calcium activated potassium channel subunit (hKCa4)

### expression in anergic T-cells

In the event a T-lymphocyte interacts with a specific antigen and results in signaling through the T-cell antigen receptor, yet does not lead to T cell proliferative response, the cell enters a state of nonresponsiveness, or *anergy.* Johnson, J.G., *et al.,* Life Sciences, 55: 1767 (1994). For normal T cell activation, IL-2 synthesis and proliferation, two signals are required: one through the T cell receptor (TCR) complex and another through a co-stimulatory molecule on the T cell known as CD28. Anergy induction can result from a number of different situations, including antigen presentation in the absence of costimulation, pharmacological blockade of T-cell proliferation or chronic stimulation of the TCR by antigen. Anergy is a long-lived state characterized by a profound inability of the T-cell to produce IL-2. An understanding of the mechanism of anergy induction and maintenance will most certainly lead to beneficial clinical applications, including improving graft acceptance and avoidance of such deleterious immune responses such as autoimmunity and allergy. See EXAMPLE XVII.

We have shown dramatic upregulation of the novel calcium activated potassium channel subunit RNA and currents when T-cells are activated. There appears to be be a correlation between induction of hKCa4 (SEQ ID NO:3) currents and the anergic state.

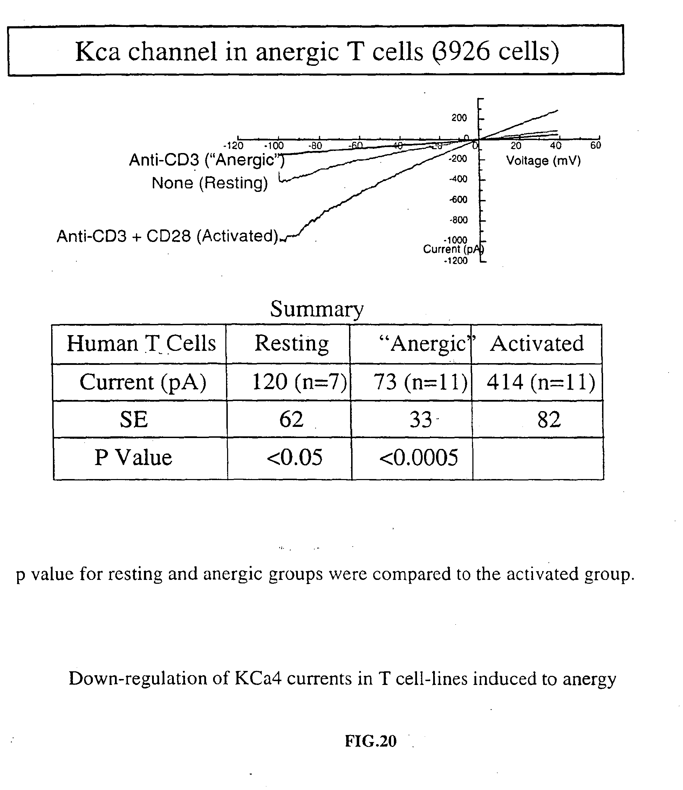

Resting (no stimulus), activated (anti-CD3+CD28) and T-cells induced to anergy (anti-CD3 alone) were patch clamped, to examine relative KCa4 channel activities. Dramatic downregulation of KCa4 current is herein demonstrated in T-cells receiving TCR occupancy alone compared to T cells receiving TCR occupancy plus costimulation. (p<0.001). Results are summarized in FIG.20. Our data point to the importance of the costimulatory signal in upregulation of this channel during T cell activation.

### Antibodies

Three peptides were designed to SEQ ID NO:3 (hKCa4) for antibody generation. These peptides were chosen based on specificity for KCa4 over KCal-3 as well as high antigenicity index.
A: Positions 415-427 + C of SEQ ID NO:3 (COOH-terminus with an added cysteine)
B: Positions 415-427 of SEQ ID NO:3 (bare COOH-terminus)
C: Positions 135-148 of SEQ ID NO:3 (S3-S4 loop)

The peptides were ordered from Research Genetics, Huntsville. AL. The peptides were conjugated to KLH and to BSA using the Imject Immunogen EDC Conjugation kit (Cat#77101, Pierce Inc., IL). The KLH-conjugated peptides were sent to VETERINARY MEDICINE for antibody generation in rabbits. 300 ug of the KLH-peptide was used per injection. The immunization protocol was as follows:

| | |
|---|---|
| Aug 19 | Prebleed |
| Aug 20 | 1^{st} Injection(Complete adj.) |
| Aug 27 | 2^{nd} Injection(Incomplete adj.) |
| Sept. 10 | 3^{rd} Injection(Incomplete adj.) |
| Sept. 17 | 1^{st} Bleed |
| Sept. 24 | 4^{th} Injection(Incomplete adj.) |
| Oct. 1 | 2^{nd} Bleed |
| Oct. 8 | 5^{th} Injection(Incomplete adj.) |
| Oct 15 | 3^{rd} Bleed |
| Oct. 22 | 6^{th} Injection (Incomplete adj.) |
| Oct. 29 | Terminal Sample |

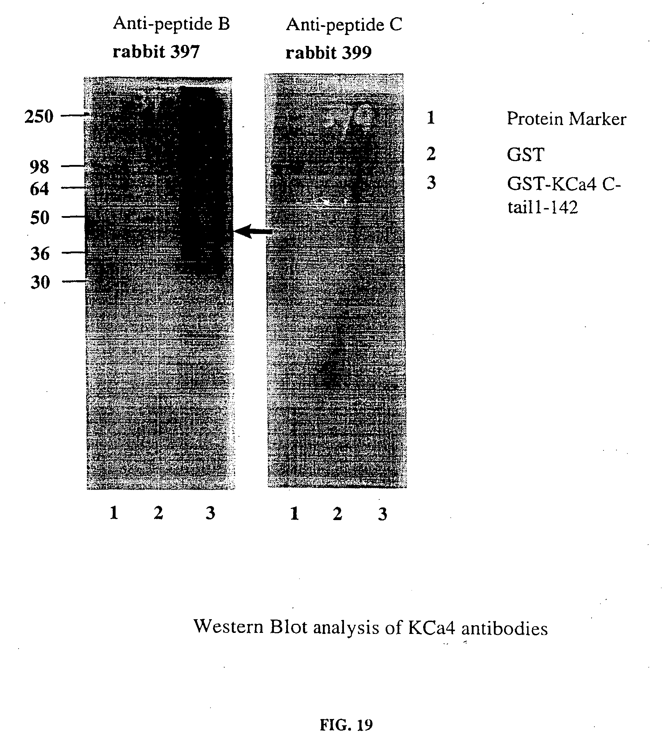

An ELISA (ELISAmate kit. Kirkegaard and Perry Laboratories Inc. MD) using the BSA-peptide as the antigen, and HRP-conjugated goat anti-rabbit antibody as the secondary reagent, gave very good titers in immune sera for all three peptide antibodies tested. Western blot analysis (using the ECL detection kit, Amersham., England) of a C-terminus tail GST fusion protein (made in bacteria, see elsewhere in this document under *two yeast hybrid*) picked up a strong band at the predicted size when peptide B antiserum was used but not with peptide C antiserum (which is directed to S3-S4 loop). *See,* FIG.19.

In addition, recombinant the calcium activated potassium channel polypeptide can be separated from other cellular proteins by use of an immunoaffinity column made with monoclonal or polyclonal antibodies specific for full length nascent calcium activated potassium channel polypeptide, or polypeptide fragments of the calcium activated potassium channel.

Calcium activated potassium channel polypeptides described herein may be used to affinity purify biological effectors from native biological materials, e.g., disease tissue. Affinity chromatography techniques are well known to those skilled in the art. A calcium activated potassium channel peptide described herein or an effective fragment thereof, is fixed to a solid matrix, e.g. CNBr activated Sepharose according to the protocol of the supplier (Pharmacia, Piscataway, NJ), and a homogenized/buffered cellular solution containing a potential molecule of interest is passed through the column. After washing, the column retains only the biological effector which is subsequently eluted, e.g., using 0.5M acetic acid or a NaCl gradient.

Monospecific antibodies to the calcium activated potassium channel polypeptide are purified from mammalian antisera containing antibodies reactive against the polypeptide or are prepared as monoclonal antibodies reactive with calcium activated potassium channel polypeptide using the technique of Kohler and Milstein, Nature 256, 495-497 (1975). Monospecific antibody as used herein is defined as a single antibody species or multiple antibody species with homogenous binding characteristics for the novel calcium activated potassium channel. Homogenous binding as used herein refers to the ability of the antibody species to bind to a specific antigen or epitope, such as those associated with the novel calcium activated potassium channel, as described. Novel calcium activated potassium channel specific antibodies are raised by immunizing animals such as mice, rats, guinea pigs, rabbits, goats, horses and the like, with rabbits being preferred, with an appropriate concentration of calcium activated potassium channel polypeptide either with or without an immune adjuvant.

Preimmune serum is collected prior to the first immunization. Each animal receives between about 0.1 mg and about 1000 mg of calcium activated potassium channel polypeptide associated with an acceptable immune adjuvant. Such acceptable adjuvants include, but are not limited to, Freund's complete, Freund's incomplete, alum-precipitate, water in oil emulsion containing Corynebacterium parvum and tRNA. The initial immunization consists of calcium activated potassium channel polypeptide in, preferably, Freund's complete adjuvant at multiple sites either subcutaneously (SC), intraperitoneally (IP) or both. Each animal is bled at regular intervals, preferably weekly, to determine antibody titer. The animals may or may not receive booster injections following the initial immunization. Those animals receiving booster injections are generally given an equal amount of the antigen in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about - 20o C.

Monoclonal antibodies (mAb) reactive with the calcium activated potassium channel polypeptide are prepared by immunizing inbred mice, preferably Balb/c, with the calcium activated potassium channel polypeptide. The mice are immunized by the IP or SC route with about 0.1 mg to about 10 mg, preferably about I mg, of calcium activated potassium channel polypeptide in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 0.1 to about 10 mg of calcium activated potassium channel polypeptide in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1; MPC-11; S-194 and Sp 2/0, with Sp 2/0 being preferred. The antibody producing cells and myeloma cells are fused in polyethylene glycol, about 1000 molecular weight, at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatant fluids are collected from growth positive wells on about days 14, 18, and 21 and are screened for antibody production by an immunoassay such as solid phase immunoradioassay (SPIRA) using calcium activated potassium channel polypeptide as the antigen. The culture fluids are also tested in the Ouchterlony precipitation assay to determine the isotype of the mAb. Hybridoma cells from antibody positive wells are cloned by a technique such as the soft agar technique of MacPherson, Soft Agar Techniques, in Tissue Culture Methods and Applications, Kruse and Paterson, Eds., Academic Press, 1973.

Monoclonal antibodies are produced in vivo by injection of pristane primed Balb/c mice, approximately 0.5 ml per mouse, with about 2 x 10⁶ to about 6 x 10⁶ hybridoma cells about 4 days after priming. Ascites fluid is collected at approximately 8-12 days after cell transfer and the monoclonal antibodies are purified by techniques known in the art.

In vitro production of anti- calcium activated potassium channel polypeptide mAb is carried out by growing the hydridoma in DMEM containing about 2% fetal calf serum to obtain sufficient quantities of the specific mAb. The mAb are purified by techniques known in the art.

Antibody titers of ascites or hybridoma culture fluids are determined by various serological or immunological assays which include, but are not limited to, precipitation, passive agglutination, enzyme-linked immunosorbent antibody (ELISA) technique and radioimmunoassay (RIA) techniques. Similar diagnostic assays are used to detect the presence of the novel calcium activated potassium channel polypeptide in body fluids or tissue and cell extracts.

Diagnostic assays using calcium activated potassium channel polypeptide specific antibodies are useful for the diagnosis of conditions, disorders or diseases characterized by abnormal expression of the calcium activated potassium channel polypeptide or expression of genes associated with abnormal cell growth. Diagnostic assays for calcium activated potassium channel polypeptide include methods utilizing the antibody and a label to detect calcium activated potassium channel polypeptide in human body fluids, cells, tissues or sections or extracts of such tissues. The polypeptides and antibodies of the present invention may be used with or without modification. Frequently, the polypeptides and antibodies will be labeled by joining them, either covalently or noncovalently, with a reporter molecule, a myriad of which are well-known to those skilled in the art.

A variety of protocols for measuring calcium activated potassium channel polypeptide, using either polyclonal or monoclonal antibodies specific for the respective protein are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on calcium activated potassium channel polypeptide is preferred, but a competitive binding assay may be employed. These assays are described, among other places, in Maddox. DE et al (1983. J Exp Med 158:1211).

In order to provide a basis for the diagnosis of disease, normal or standard values for calcium activated potassium channel polypeptide expression must be established. This is accomplished by combining body fluids or cell extracts taken from normal subjects, either animal or human, with antibody to calcium activated potassium channel polypeptide under conditions suitable for complex formation which are well known in the art. The amount of standard complex formation may be quantified by comparing it with a dilution series of positive controls where a known amount of antibody is combined with known concentrations of purified calcium activated potassium channel polypeptide. Then, standard values obtained from normal samples may be compared with values obtained from samples from subjects potentially affected by a disorder or disease related to calcium activated potassium channel polypeptide expression. Deviation between standard and subject values establishes the presence of the disease state.

It is readily apparent to those skilled in the art that the above described methods for producing monospecific antibodies may be utilized to produce antibodies specific for calcium activated potassium channel polypeptide fragments, or the full-length nascent calcium activated potassium channel polypeptide. Specifically, it is readily apparent to those skilled in the art that monospecific antibodies may be generated which are specific for the fully functional receptor or fragments thereof.

Calcium activated potassium channel polypeptide antibody affinity columns are made by adding the antibodies to Affigel-10 (Biorad), a gel support which is activated with N hydroxysuccinimide esters such that the antibodies form covalent linkages with the agarose gel bead support. The antibodies are then coupled to the gel via amide bonds with the spacer arm. The remaining activated esters are then quenched with IM ethanolamine HCl (pH 8).

The column is washed with water followed by 0.23M glycine HCI (pH 2.6) to remove any non-conjugated antibody or extraneous protein. The column is then equilibrated in phosphate buffered saline (pH 7.3) with appropriate detergent and the cell culture supernatants or cell extracts containing calcium activated potassium channel polypeptide made using appropriate membrane solubilizing detergents are slowly passed through the column. The column is then washed with phosphate buffered saline/detergent until the optical density falls to background, then the protein is eluted with 0.23M glycine-HCl (pH 2.6)/detergent. The purified calcium activated potassium channel polypeptide is then dialyzed against phosphate buffered saline/detergent.

### Screening methods

The present invention is also directed to methods for screening for compounds which modulate the expression of DNA or RNA encoding calcium activated potassium channel polypeptide, as well as the function of calcium activated potassium channel polypeptide *in vivo*. Compounds which modulate these activities may be DNA, RNA, peptides, proteins, or non-proteinaceous organic molecules. Compounds may modulate by increasing or attenuating the expression of DNA or RNA encoding calcium activated potassium channel polypeptide, or the function of calcium activated potassium channel polypeptide. Compounds that modulate the expression of DNA or RNA encoding calcium activated potassium channel polypeptide or the function of calcium activated potassium channel polypeptide may be detected by a variety of assays. The assay may be a simple "yes/no" assay to determine whether there is a change in expression or function. The assay may be made quantitative by comparing the expression or function of a test sample with the levels of expression or function in a standard sample.

The calcium activated potassium channel polypeptide described herein, its immunogenic fragments or oligopeptides can be used for screening therapeutic compounds in any of a variety of drug screening techniques. The fragment employed in such a test may be free in solution, affixed to a solid support, borne on a cell surface, or located intracellularly. The abolition of activity or the formation of binding complexes, between calcium activated potassium channel polypeptide and the agent being tested, may be measured. Accordingly, the present invention provides a method for screening a plurality of compounds for specific binding affinity with calcium activated potassium channel polypeptide or a fragment thereof, comprising providing a plurality of compounds; combining the calcium activated potassium channel polypeptide of the present invention or a fragment thereof with each of a plurality of compounds for a time sufficient to allow binding under suitable conditions; and detecting binding of the calcium activated potassium channel polypeptide, or fragment thereof, to each of the plurality of compounds, thereby identifying the compounds which specifically bind the calcium activated potassium channel polypeptide.

Since the novel gene, e.g., SEQ ID NO:1 is highly expressed in activated T-cells, and since T-cell calcium activated potassium channels are potently blocked by the scorpion toxin Charybdotoxin (CTX), various cell-lines that heterologously over-expresses the novel channel structural coding regions described herein can be used in radio-labeled binding assays to screen for pharmacologically active molecules which block the novel channel using ¹²⁵I-CTX as the ligand.

### CTX-binding assay for high-throughput screening for modulators of the novel channel

Particularly preferred embodiments of the present invention are cell-lines that heterologously over-expresses the calcium activated potassium channel described herein (e.g., SEQ ID NO:3 or a biologically active truncated version thereof or a chimeric fusion) and their use in binding assays to identify pharmacologically active molecules which block or otherwise modulate biological activity of the novel channel.

For example, a radio-labeled binding assay using ¹²⁵I-CTX as the ligand may be used as previously decribed by Hill, R.J., Mol. Pharm., 48:98 (1995), and Deutsch, C., *et al.,* J. Biol. Chem., 266:3668 (1991). Membrane preparations of cell-lines which over-express the novel calcium activated potassium channel described herein are made by homogenizing the cells using a Polytron for 25 seconds at 13,000 RPM and spun at low speed (100 g) for 2 minutes. The supernatant is spun at high speed (50,000 g) for 10 minutes. The pellet is suspended in 1 ml of assay buffer (5 mM Nacl, 5 mM KCL, 10 mM HEPES, 6 mM glucose, pH 8.4) and diluted to 50 ug/ml.

To each well of a 96-well microtiter plate, 130 ul of assay buffer is added, as well as 20 ul of test molecule compound (test drug may be, for instance, a small molecule, peptide, analog or mimetic compound) /control assay buffer /non-specific (10 nM cold CTX) (not-labelled) 50 ul of membranes from cells which over-express the novel calcium activated potassium channel at 50 ug/ml and 50 ul of ¹²⁵ I-CTX (25 pM: NEN. 2200 Ci/mmol) are incubated for 20 minutes at 21°C with mixing. Bound radiolabeled CTX is separated from free radiolabeled CTX in solution by filtering over pre-soaked GF/C Unifilters (Packard Instruments) and washing rapidly in ice-cold wash buffer Upon drying, the filter plates are scintillation counted. Data from saturation experiments are subject to Scatchard analysis and linear regression. Deutsch, *et al.,* J. Biol. Chem., 266:3668 (1991). Compounds that compete with the radio-labeled CTX for binding the novel calcium activated potassium channel are identified which produce a reduction in specific counts.

### Scintillation proximity assay

In another embodiment, a scintillation proximity assay (SPA) that eliminates the need for filters, can be easily adapted for high throuput screening (HTS) assays. Hoffman, R., *et al.,* Anal. Biochem., 20370 (1992): Kienuis, C.B.M., *et al.,* J. Recept. Res.. 12:389 (1992). *See,* EXAMPLE XVI.

### Flurometric imaging plate reader (FLIPR)

The FLIPR system is most useful as a high-throughput primary screen for ion channels, which influence the cell membrane potential (e.g: KCa4 (SEQ ID NO:3) described herein). FLIPR is a commercial device (Molecular Devices, Sunnyvale, CA) which is designed to measure fluorescent signals simultaneously from 96 wells in a microtiter plate using an argon ion laser and a semi-confocal optical system. It is equipped with a 96-tip pipettor allowing simultaneous drug administration to all wells. The time resolution is down to 1 second. The fluorescent dyes which are most useful are DiBAC4(3) for membrane potential (Epps, D.E., *et al.,* Chem Phys Lipids., 69(2):137 (1994)) and Fluo-3 for calcium (Rijkers, G.T., *et al.,* Cytometry, 11(8):923 (1990). Cells stably expressing SEQ ID NO:2 are plated onto 96-well plates at a density of 0.5×10⁴ cells/well and allowed to form a uniform monolayer. Next day, cell media is removed and incubated with EBSS buffer containing 20 mM HEPES buffer and 5uM DiBAC (cat# B-438, Molecular Probes) with or without test-compounds and incubated at 37 degrees for 1.5 hours. Cells are then washed in a non-fluorescent salt-buffer such as EBSS. Fluorescence is measured using FLIPR laser optics. (Molecular Devices, Sunnyvale, CA)

Methods of identifying compounds that modulate the activity of a human leukocyte calcium-activated potassium channel polypeptide are generally preferred, which comprise
(a) combining a candidate compound modulator of a human leukocyte calcium-activated potassium channel activity with a polypeptide of a human calcium-activated potassium channel having the sequence of SEQ ID NO:3, and
(b) measuring an effect of the candidate compound modulator on the channel.

Methods of identifying compounds that modulate the activity of a human leukocyte calcium-activated potassium channel, are especially preferred which comprise
(a) combining a candidate comound modulator of a human leukocyte calcium-activated potassium channel activity with a host-cell expressing the polypeptide of a human calcium-activated potassium channel having the sequence of SEQ ID NO:3, and
(b) measuring an effect of the candidate comound modulator on the channel.

Compounds which are identified generally according to methods descibed and referenced herein that modulate the activity of a calcium-activated potassium channel comprised of the sequence of SEQ ID NO:3 are especially preferred embodiments of the present invention.

### Two hybrid assays

In another embodiment of the invention, a nuleic acid sequence which encodes a calcium activated potassium channel substantially as depicted in SEQ ID NO:3 or a biologically active fragment thereof may be ligated to a heterologous sequence to encode a fusion protein. For example, for screening compounds for modulation of biological activity, it may be useful to encode a chimeric calcium activated potassium channel as described herein for expression in hererologous host cells. Chimeric constructs may also be used to express a 'bait', according to methods well known using a yeast two-hybrid system, to identify accessory native peptides that may be associated with the calcium activated potassium channel polypeptide described herein. Fields, S., *et al.,* Trends Genet.. 10:286 (1994); Allen, J.B., *et al..* TIBS, 20:511 (1995). A yeast two-hybrid system has been described wherein protein:protein interactions can be detected using a yeast-based genetic assay via reconstitution of transcriptional activators. Fields, S., Song, O., Nature 340:245 (1989). The two-hybrid system used the ability of a pair of interacting proteins to bring a transcription activation domain into close proximity with a DNA-binding site that regulates the expression of an adjacent reporter gene. See also, Mendelsohn, A.R., Brent. R., Curr. Op. Biotech.. 5:482 (1994); Phizicky. E.M., Fields, S., Microbiological Rev., 59(1):94 (1995): Yang, M., et al., Nucleic Acids Res.. 23(7):1152 (1995): Fields, S., Sternglanz, R., TIG, 10(8):286 (1994); and US Patents 5,283,173. *System to Detect Protein-Protein Interactions,* and 5,468,614.

### Yeast 2-Hybridization experiments (implication of calmodulin)

The MATCHMAKER two-hybrid system from Clontech, Palo Alto CA, (Cat # K1604-1) from is a complete GAL4-based system that provides transcriptional assay for detecting protein-protein interactions in yeast. The two-hybrid system utilizes a powerful growth selection-the conditional expression of a nutritional reporter gene-to screen large numbers of yeast transformants with a specially constructed fusion library for interacting proteins. hKCa4 (SEQ ID NO:2) C-terminal was subcloned into GAL4 DNA-BD vector (pAS2-1, Clontech, Palo Alto, CA) by conventional means using standard PCR (Clontech, PT3061-1). The restriction sites used in the subcloning are EcoRI and XhoI. This construct was then confirmed by sequencing and used as the bait in the library screening. The library used is human leukocyte MATCHMAKER cDNA (HL4021AB, Clontech). All the screening procedures were performed based on manufacturer's recommendations (Clontech manual #PT3061-1). Ten thousand putative positive clones were identified after first round screening. Tow thousand of these were then subjected to colony-lift lacZ assay, which identified seven positives. Positive colonies are then sequenced for further analysis, such as comparison with the sequence database. Six of the seven clones coded for calmodulin.

### Antisense

The cDNA sequences SEQ ID NO: 1 and SEQ ID NO:7 provided herein, may be used in another embodiment of the invention to study the physiological relevance of the novel calcium activated potassium channel in cells, especially cells of hematopoietic origin, by knocking out the endogenous gene by use of anti-sense constructs that target, for example, the region of the initiator methionine or the pore-region decribed *supra. See, e.g.,* Shirihai, O. *et al.,* Pfugers Arch. 431:632 (1996); Chung, S., *et al.,* PNAS, 92:5955 (1995).

U.S. Patent No. 5,639,595, *Identification of Novel Drugs and Reagents,* issued Jun. 17, 1997, wherein methods of identifying oligonucleotide sequences that display *in vivo* activity are thoroughly described. Expression vectors containing random
oligonucleotide sequences derived from previously known polynucleotides are transformed into cells.
The cells are then assayed for a phenotype resulting from the desired activity of the oligonucleotide.
Once cells with the desired phenotype have been identified, the sequence of the oligonucleotide having the desired activity can be identified. Identification may be accomplished by recovering the vector or by polymerase chain reaction (PCR) amplification and sequencing the region containing the inserted nucleic acid material.

Nucleotide sequences that are complementary to the calcium activated potassium channel polypeptide encoding polynucleotide sequence can be synthesized for antisense therapy.These antisense molecules may be DNA, stable derivatives of DNA such as phosphorothioates or methylphosphonates, RNA, stable derivatives of RNA such as 2'-O-alkylRNA, or other oligonucleotide mimetics. Calcium activated potassium channel polypeptide antisense molecules may be introduced into cells by microinjection, liposome encapsulation or by expression from vectors harboring the antisense sequence. Calcium activated potassium channel polypeptide antisense therapy may be particularly useful for the treatment of diseases where it is beneficial to reduce calcium activated potassium channel polypeptide activity.

Calcium activated potassium channel polypeptide gene therapy may be used to introduce the polypeptide into the cells of target organs. Conversely, calcium activated potassium channel polypeptide *antisense* gene therapy may be used to reduce the expression of the polypeptide in the cells of target organs. The calcium activated potassium channel polypeptide coding region can be ligated into viral vectors which mediate transfer of the calcium activated potassium channel polypeptide DNA by infection of recipient host cells. Suitable viral vectors include retrovirus, adenovirus, adeno-associated virus, herpes virus, vaccinia virus, polio virus and the like. Alternatively, calcium activated potassium channel polypeptide DNA can be transferred into cells for gene therapy by non-viral techniques including receptor-mediated targeted DNA transfer using ligand-DNA conjugates or adenovirus-ligand-DNA conjugates, lipofection membrane fusion or direct microinjection. These procedures and variations thereof are suitable for *ex vivo,* as well as *in vivo* calcium activated potassium channel polypeptide gene therapy.

### Compositions

Pharmaceutically useful compositions comprising calcium activated potassium channel polypeptide DNA, calcium activated potassium channel polypeptide RNA, or calcium activated potassium channel polypeptide, or modulators of calcium activated potassium channel polypeptide activity, may be formulated according to known methods such as by the admixture of a pharmaceutically acceptable carrier. Examples of such carriers and methods of formulation may be found in *Remington's Pharmaceutical Sciences.* To form a pharmaceutically acceptable composition suitable for effective administration, such compositions will contain an effective amount of the protein, DNA, RNA, or modulator.

Therapeutic or diagnostic compositions of the invention are administered to an individual in amounts sufficient to treat or diagnose calcium activated potassium channel polypeptide related disorders. The effective amount may vary according to a variety of factors such as the individual's condition, weight, sex and age. Other factors include the mode of administration.

The pharmaceutical compositions may be provided to the individual by a variety of routes such as subcutaneous, topical, oral and intramuscular.

The term "chemical derivative" describes a molecule that contains additional chemical moieties which are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as *Remington's Pharmaceutical Sciences.*

Compounds identified according to the methods disclosed herein may be used alone at appropriate dosages defined by routine testing in order to obtain optimal modulation of a calcium activated potassium channel, or its activity while minimizing any potential toxicity. In addition, co-administration or sequential administration of other agents may be desirable. The present invention also has the objective of providing suitable topical, oral, systemic and parenteral pharmaceutical formulations for use in the novel methods of treatment of the present invention. The compositions containing compounds identified according to this invention as the active ingredient for use in the modulation of calcium activated potassium channels can be administered in a wide variety of therapeutic dosage forms in conventional vehicles for administration. For example, the compounds can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as a calcium activated potassium channel modulating agent.

The daily dosage of the products may be varied over a wide range from 0.01 to 1,000 mg per adult human/per day. For oral administration, the compositions are preferably provided in the form of scored or unscored tablets containing 0.01. 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. Even more particularly, the range varies from about 0.05 to about I mg/kg. Of course the dosage level will vary depending upon the potency of the particular compound. Certain compounds will be more potent than others. In addition, the dosage level will vary depending upon the bioavailability of the compound. The more bioavailable and potent the compound, the less compound will need to be administered through any delivery route, including but not limited to oral delivery. The dosages of the calcium activated potassium channel modulators are adjusted when combined to achieve desired effects. On the other hand, dosages of these various agents may be independently optimized and combined to achieve a synergistic result wherein the pathology is reduced more than it would be if either agent were used alone.

### EXAMPLES

### EXAMPLE I

### Membrane Preparation of Transfected HEK Cells for Screening Compounds

Membranes are prepared when the HEK cells in each T-150 (or T-175 or T-225) flask are confluent.
The preparation of membranes from each flask of cells is as follows:
1. Pour off the cell culture media and save (since it always contains some cells that have just been dislodged from handling the flask).
2. Rinse the confluent layer of cells with about 8 mls of phosphate buffered saline (PBS). 37°C. This removes residual fetal calf serum which can inhibit the trypsin used in step 3. Save the PBS from the rinse, too, to capture any other cells that are dislodged.
3. Add about 5 ml of trypsin-EDTA, 37°C. Slowly rock the flask back and forth to let the trypsin wash over the whole layer of cells. In about 30 sec, some of the cells will come loose; tap the flask firmly on the lab bench to dislodge the rest of the cells. Quickly add fresh medium to the flash to stop the trypsin reaction. Add about 10ml medium for every 5ml of trypsin. Pipet the trypsin/cells/medium into a centrifuge tube. Rinse the flask with about 5 ml more medium and combine with other tubes.
4. Wash the cells by spinning at 250xg, 8 min. Resuspend the individual cell pellets in a small volume of assay buffer, and combine them into one tube.
5. Homogenize the cells using a Polytron, 25 sec., 13000 RPM
6. LOW SPEED SPIN: 800 RPM (~100xg) 2 min.; save the supernatant; discard the pellet
7. HIGH SPEED SPIN: transfer the sup into high speed tubes, and spin 20,000 RPM (50,000xg), 10 min. Discard the supernatant: add 1 ml of assay buffer on top of pellet
8. Freeze on dry ice; cap the tubes and store at -80 C.

Dilute the membranes to 50ug/ml, and use 2.5ug in each well (50ul of 50ug/ml)

### EXAMPLE II

### ¹²⁵-I Charybdotoxin binding assay for (SEQ ID NO:3) HKca4

### (A) FILTRATION METHIOD

### Materials:

| Assay Buffer: | Wash Buffer: |
|---|---|
| 5mM NaCl | 200mM NaCl |
| 5mM KCl | 20mM HEPES |
| 10mM HEPES | pH 8.0 with Tris base |
| 6mM glucose | |
| 230mM sucrose | |
| bring to pH 8.4 with TRIS base | |
| add BSA to a [final] of 0.02% | |

### SEQ ID NO:2 stably transfected whole MEL cells 500,000 cell per well - or - SEQ ID NO:2 stably transfected HEK cells

- misc.: 96 well microtiter plates, Falcon, round bottom, non tissue culture treated Phosphate buffered saline (PBS) no Ca++ or Mg++
96 well Unifilters, GF/C, (Packard, Meriden, CT).
Microscint-20 scintillation fluor, Packard
Top Count scintillation counter, Packard
Polyethlyenimine, 0.6% for soaking Unifilters
¹²⁵I-Charybdotoxin, (NEN, Boston, MA, NEX-276) 2200Ci/mmol nonspecific binding defined with 100nM cold ChTX (Bachem, King of Prussia, PA).
- **Assay:**: 130ul assay buffer
20ul test compound or buffer or cold CTX (nonspecific)
50ul cells at 1E⁶/ml (500,000 per well)
50ul ¹²⁵I-ChTX [final] 50 pM
250ul total volume

### Assay Method:

Place 130ul assay buffer per well in 96well plate. Add 20ul of test compound, or control solution (more buffer or test compound diluent), or nonspecific (defined with 100nM cold CTX). Wash the cells twice in PBS. resuspend in assay buffer, and add 50ul of cells to the assay plate. Add 50ul ¹²⁵I-ChTX, [final] 50 pM. and incubate for 45 min. 21° C. mixing on the plate mixer. Separate bound from free ¹²⁵I-ChTX by filtering over the presoaked GF/C Unifilters: wash twice rapidly, using ice cold wash buffer. Let Unifilter plates dry overnight. When dry, add 25ul Microscint-20 to Unifilters, and count in Top Count scintillation counter.

An example of a dose-response curve for CTX-binding/novel calcium activated potassium channel subunit (hKCa4) is shown in FIG. 18.

### EXAMPLE III

### 125I-ChTX Binding Assays

Lymphocytes are incubated in 12 x 75-mm polystyrene tubes with ¹²⁵I-ChTX (1-2 x 10³ Ci/mmol). Unless otherwise noted, cells are suspended in isotonic sucrose medium (Medium I) containing 10 mM NaHepes, 5mM KCl. 5 mM NaCl, and 6 mM glucose, pH 8.4, and incubated with ¹²⁵I-ChTX for 1 h at room temperature on a rotary shaker. Nonspecific binding is determined in the presence of 10 nM native ChTX. Stock cell suspensions are diluted to give a final cell concentration of 2.5 x 10⁵ cells/ml in a total volume of 400 µl. At the end of the incubation period, samples are diluted with 4 ml of ice-cold Quench solution, which contain 200 mM NaCl, 20 mM Hepes (free acid), titrated to pH 8.0 with Trisbase. Quenched samples are filtered through GF/C glass microfiber filters, that had been presoaked in 0.6% polyethylenimine, and washed twice with ice-cold Quench solution. Triplicate samples are run for each experimental point. Standard deviation of the mean is typically less than 5%. Different cell preparations may produce somewhat different ratios of nonspecific/total ¹²⁵I-ChTX binding. Stock solutions of ChTX are prepared in 100 mM NaCl, 20 mM Tris-HCl, pH 7.4. 0.1% bovine serum albumin.

*Analysis of Data-*Data from saturation experiments are subjected to Scatchard analysis, and linear regression is performed to yield to equilibrium dissociation constant (K₄) and maximum receptor concentration (Bₘₐₓ). Correlation coefficients for these determinations are typically greater than 0.95. Data from competition experiments are analyzed by the standard method of Cheng and Prusoff to determine Kᵢ values. The dissociation rate constant (κ₁) is determined directly from a first order plot of ligand dissociation *versus* time. The rate of ligand association (*k*₁) is determined from the equation *k*₁ =k_{obe}([LR]_{c}/([L][LR]ₘₐₓ)), where [L] is the concentration of ligand, [LR]_{c} is the concentration of the complex at equilibrium, [LR]ₘₐₓ is the maximum number of receptors present, and k_{obe} is the slope of the pseudo-first order plot In ([LR]ₑ/{[L]_{c} - [LR]ₜ}] *versus* time. Association and,dissociation rate of ¹²⁵I-ChTX are also determined by measuring the kinetics of radiolabeled toxin binding at different ligand concentrations, determining k_{obe} at each concentration of toxin from semilogarithmic representations of these data, and determining *k*₁ and *k*-₁ from the slope and y intercept, respectively, of the plot of k_{obe} *versus* ChTX concentration.

125I-ChTX Binding Assays may be used with the present invention substantially as described by Deutsch. C., *et al.,* J. of Biological Chemistry, 266: No. 6, 3668-3674 (1991).

### EXAMPLE IV

### Database search

A pore motif of voltage-gated potassium channels, SEQ ID NO: 10:
PASFWWATITMTTVGYGDIYP, was derived from hKv2.1, a Shab-related K channel (Chandy and Gutman, Handbook of Receptors and Channels, *Ligand and Voltage-gated Ion Channels,* Edited by R. Alan North, CRC Press, Inc., Chapter 1 (1995)). SEQ ID NO: 10 was used to query via *tblastn* search of an un-anotated proprietary database. *Basic Local Alignment Search Tool,* Altschul, S.F.. *et al.,* J. Mol. Biol., 215:403 (1990). SEQ ID NO:8 (FIG.8) was identified in this manner. Seq ID NO: 8 was then used as a query sequence in a *blastn* search against a proprietary database to identify SEQ ID NO:9 (FIG.9) through overlapping clones.

Both SEQ ID NO:8 and SEQ ID NO:9 clones were isolated from a male and female donor cDNA library of adherent mononuclear cells. Library was constructed using 2 micrograms of polyA RNA isolated from adherent mononuclear cells, which came from a pool of male and female donors. The cells were cultured for 24 hours following Ficoll Hypaque centrifugation. CDNA synthesis was initiated using a Notl-oligo(dT) primer. Double-stranded cDNA was blunted, ligated to EcoRI adaptors, digested with NotI, size-selected, and cloned into the NotI and EcoRI sites of the pSPORT vector. The SEQ ID NO:8 and SEQ ID NO:9 clones were sequenced (ABI PRISM^{™} Dye Terminator Cycle sequencing on ABI PRISM^{™} 377 automated sequencer). SEQ ID NO:8 is 384 bp shorter than SEQ ID NO:9 and is identical in all but the 5'- end, wherein they differ by fifty five (55) nucleotides. This evidence suggests the existence of an alternate splice variant of the native gene. SEQ ID NO:9 was used to identify the full-length clone SEQ ID NO: described herein, by screening a human lymph-node cDNA library, as described below.

### EXAMPLE V

### cDNA library screening

A human lymph-node lambda gt 10 cDNA library, oligo-dT plus random primed, from mRNA obtained from 6 donor male/female Caucasians, aged 26-29 whole lymph-nodes was used.

Clontech Labortatories, Palo Alto, CA (Cat # HL5000a: Lot # 46005). Standard methodology was followed as described in the Clonetech Lambda Library protocol handbook (PT1010-1).

### Primary screen

About 30,000 plaque-forming units (pfu's) of lambda phage were plated onto 150 mm plates containing LB agar + 10 mM MgSO4+ 200 ul of an overnight culture of the bacterical host strain E.coli C600 Hfi. Twenty such plates were made, a total of 600,000 plaques for primary screening.

Plates were inverted and incubated at 37°C overnight. The plaque diameters had just begun to make contact with one another. Plates were chilled for 1 hour.

A 150 mm nylon membrane filter (Amersham) was marked in pencil and placed onto a LB agarose plate containing the plaques. The filter was further marked in 3 asymmetric peripheral locations by puncturing the filter into the agar with a 16 gauge needle. The bottom of the plate corresponding to the hole was marked with a marker. After I minute, the filter was carefully peeled off and floated on top of DNA-denaturing solution (1.5 M NaCl, 0.5 M NaOH), plaque-side up, for 30 sec. The filter was removed and immersed in Neutralizing solution (1.5 M NaCl, 0.5 M Tris-HCl, pH 8) for 5 minutes, rinsed in 2x SSC (0.3 M NaCl, 0.03M Na-citrate) and placed on a Whatman 3M paper to dry. The DNA was fixed onto the filter by UV-crosslinking for 45 seconds. This procedure was repeated for all 20 plates. Second, duplicate filters were placed onto the original plates and similarly processed.

### Hybridization

The 40 filters prepared were pre-hybridized in 6X SSPE. 5X Denhart's and 0.25% SDS and 100 ug/ml of salmon-sperm DNA, for 4 hour at 42°C in a water bath shaker.

### Probe for library screening

Primers were designed based on SEQ ID NO:9 to generate a probe via PCR. The primer sequences used were: 5' (forward) corresponding to SEQ ID NO: 1 nucleotides 658-676. and 5' (reverse) corresponding to SEQ ID NO: 1 nucleotides 1643-1661. The target sequence was amplified from 1 ng of SEQ ID NO:9 plasmid (in pSport 1 vector, Life Technologies, Gaithersburg, MD) in a 50 µL reaction using Advantage^{™} KlenTaq polymerase (Clontech # 8417-1, lot 7020348) according to the manufacturer's recommendations. Cycling parameters were employed according to the Clontech Marathon-Ready kit conditions (touchdown PCR with three minute extensions). The target nucleic acid product was purified using Qiagen's Qiaquick PCR Purification kit, cat # 28104) according to the manufacturer's protocol and subsequently used to screen the human lymph node cDNA library. The probe was radiolabeled using ³²P-dCTP (Pharmacia Inc., Piscataway. NJ, cat# 27-9240 B).

The radiolabelled probe was added to the pre-hybridization mix containing the filters and allowed to hybridize at 42°C overnight. The filters were washed twice in buffer 2 (1X SSC, 0.5% SDS) at 65°C for 1 hour, placed on a Whatman filter paper and exposed to X-ray film overnight. Thirty eight positive plaques were identified. The plaques were picked and eluted in 1ml of dilution buffer using a cut P1000 pipet tip .

### Secondary and tertiary screens

Of the 38 positive clones, 12 were [randomly] chosen for secondary screening. The clones were plated at dilutions of 1: 10,0000, grown overnight, lifted on nylon filters, denatured, neutralized and probed with the 1 Kb SEQ ID NO:9 probe using a non-radioactive ECL method (Amersham, Inc., Cat# RPN 3001). Ten positive clones were picked (one from each plate) and processed for tertiary screening as described *supra.* At least 80% to 100% of the tertiary plaques were positive. One positive plaque was picked from each of the 10 plates.

### Amplifying Lambda clone inserts

The 10 lambda inserts were amplified from 1 µl of phage supernatant in a 50 µl reaction using Advantage^{™} KlenTaq polymerase (Clontech # 8417-1, lot 7020348) according to the manufacturer's recommendations, with the addition of 5% final concentration DMSO. The primers were vector primers corresponding to λgt10 nucleotides 201-231 (forward) and 267-298 (reverse), which surround the EcoRI cloning site (Appendix C. Clontech Lambda Library Protocol Handbook, manual # PT1010-1). Cycling parameters were according to Clontech Marathon-Ready cDNA User Manual (PT1156-1) p. 19, program 1 (briefly, touchdown PCR with three minute extensions). Product was purified using Qiagen's Qiaquick PCR Purification kit (cat # 28104) according to the manufacturer's protocol. Four PCR products were sequenced directly in the presence of DMSO (ABI PRISM^{™} Dye Terminator Cycle sequencing on ABI PRISM™377 automated sequencer). One clone was identified which contained the full-length cDNA: SEQ ID NO:1.

### EXAMPLE VI

### Northern blots

In order to analyze tissue-distribution and size of the transcripts corresponding to SEQ ID NO:1, Northern blot analyses were performed using two separate probes. Pre-made mRNA blots were used from Clontech which contained approximately 2ug poly A+ RNA per lane.

Probe I was generated by digesting SEQ ID NO:8 (in pSport1, Life Technologies) with PstI and ScaI restriction enzymes according to manufacturer's recommendations (Promega), and gel-isolating the 444 bp fragment using standard molecular biology techniques. This fragment corresponds to 3' UTR, SEQ ID NO: 1 nucleotides 1716-2163, an area of exact match between SEQ ID NO:8 and SEQ ID NO:9. The restriction fragment was purified from agarose using Qiagen's Qiaquick Gel Extraction kit according to the manufacturer's recommendations and quantitated by 260 nm OD measurement. Twenty ng of fragment was labeled using α³²P-dCTP (Dupont NEN) and Pharmacia's Ready-To-Go™ labeling beads (-dCTP) (cat # 27-9240-01) according to the manufacturer's recommendations. Labeled product was purified from unincorporated radioactive nucleotide using Pharmacia's ProbeQuant™ G-50 Micro Columns (cat # 27-5335-01) according to the manufacturer's recommendations.

Twenty ng (17 x 10⁶ cpm) of labeled restriction fragment was used to probe Clontech Multiple Tissue Northerns (catalogue # 7760-1 and 7759-1) and a prepared blot (descrbed *infra*) according to Clontech's recommendations (User Manual #PT1200-1). The blots were prehybridized for 45 minutes at 68°C in Clontech's ExpressHyb™ solution (cat # 8015-1). Denatured probe was added to 10 ml fresh pre-warmed ExpressHyb™. This replaced the prehybridization solution for 1.5 hours at 68°C. The blots were washed three times, ten minutes each, in 2X SSC + 0.1 % SDS at room temperature, followed by two washes, twenty minutes each, in 0.1X SSC + 0.1% SDS at 50°C. Blots were exposed to film overnight using two intensifying screens.

Probe 2 was generated by PCR using the primers: 5' (forward) corresponding to SEQ ID NO: nucleotides 658-676. and 5' (reverse) corresponding to SEQ ID NO: nucleotides 1079-1098. The 440 bp product is unique to the SEQ ID NO:9 clone except for the reverse primer sequence, which is also found in the SEQ ID NO:8 clone. PCR reagents, template, and cycling conditions were the same as those used for generating cDNA library probe except that extension times were 1.5 minutes. Product was purified in the same manner as the library probe. Forty ng of Northern probe 2 was labeled exactly as Northern probe 1 and used to screen the Clontech Multiple Tissue Northerns as described *supra* for Northern probe 1.

### EXAMPLE VII

### Northern blot II

Since calcium activated potassium channels are known to be dramatically up-regulated upon T-cell activation (e.g., Grissmer, *et al.,* J. Gen. Physiol. 102:601 (1993)), the expression pattern of SEQ ID NO: 1 (SEQ ID NO:7) in resting versus activated human peripheral blood T-lymphocytes was determined.

Mononuclear cells were isolated from whole blood (obtained from healthy donors) on Ficoll-Hypaque density gradients as in Current Protocols in Immunology, vol.1, ed. by John E. Coligan, *et al.,* John Wiley and Sons. (1996). Red blood cells were removed by hypotonic lysis for 45 seconds in 0.2% saline followed by an equal volume of 1.6% saline to bring the saline back to physiological concentration. After an additional wash in Hank's Balanced Salt Solution (Life Technologies cat # 14175-095), CD14+ (monocyte and macrophage) and CD19+ (B cell) populations were removed using Dynal's Dynabeads® M450 Pan-B (CD19, cat # 111.03) and M450 CD 14 (cat # 111.11) according to the standard Dynal protocol. Remaining cells were T cells 80-90% as assessed by FACS analysis.

The T cells were cultured overnight at 37°C, 5% CO₂, in complete RPMI 1640 medium (RPMI 1640, Life Technologies, cat # 11875-093, containing: 10% fetal bovine serum, Life Technologies, cat # 26140-087, heat inactivated 30 minutes at 56°C; 1X penicillin/streptomycin, Life Technologies, cat # 15140-122). The cells were then activated with 10 µg/mL final concentration phytohemagglutinin (PHA-P, Sigma cat # L9132) in complete RPMI for 48-72 hours at 37°C, 5% CO₂. Messenger RNA was isolated using Invitrogen's FastTrack® 2.0 kit (cat # K1593-02). 2.8 µg of each mRNA was run on a 1% agarose/formaldehyde/formamide gel according to Sambrook *et al.,* Molecular Cloning Lab Manual. Second Edition. Cold Spring Harbor Press (1989), section 7:43, with 3µg of RNA ladder as size markers (Life Technologies, cat # 15620-016). RNA was transferred overnight to positively charged nylon membrane (Hybond™-N+, Amersham # RPN203B), according to the standard Amersham protocol, and then UV crosslinked to the membrane using Stratagene's Stratalinker on the automatic setting.

### EXAMPLE VIII

### PCR of SEQ ID NO:1 from various human cDNAS of hematopoietic origin

SEQ ID NO: I (SEQ ID NO:7) message is determined to be present in other cells of hematopoietic orign by PCR with cDNA's isolated from different cell-lines of hematopoietic orign.

Target product was amplified from 2-2.5 ng of reverse transcribed mRNAs in a 20 µL reaction using Advantage^{™} KlenTaq polymerase (Clontech # 8417-1, lot 7020348) according to the manufacturer's recommendations. Primer set 1 sequences are: 5' (forward) corresponding to SEQ ID NO: nucleotides 1462-1479, and 5' (reverse), corresponding to SEQ ID NO: I nucleotides 1904-1922. Primer set 2 was the same set used to generate probe for the cDNA library screening. Primer set 2 reactions included DMSO at a final concentration of 5%. Cycling parameters were according to Clontech Marathon-Ready cDNA User Manual (PT1156-1) p. 19, program 1 (briefly, touchdown PCR with 1.5-2 minute extensions). 10 µL of each reaction was analyzed on a 1% agarose gel containing 0.5 µg/mL final concentration ethidium bromide in TAE buffer as in Sambrook *et al.,* Molecular Cloning Lab Manual, Second Edition. Cold Spring Harbor Press (1989), using 600 ng of 1 kb DNA ladder as markers (Life Technologies, cat # 15615-016).

### EXAMPLE IX

### SEQ ID NO:7

*Library screening:* A ³²P-dCTP labeled fragment from a clone (corresponding to SEQ ID NO:7 positions 658-1661) was used as a probe to screen ~ 600,000 recombinant plaques from a human lymph-node lambda gt10 cDNA library (Clontech Laboratories, Inc., Palo Alto, CA). Hybridizations were at 42°C overnight. Filters were washed twice in 1X SSC, 0.5% SDS at 65°C for 1 hour, and exposed to X-ray film overnight. Of 38 doubly-positive clones, 10 were subjected to two rounds of plaque purification and rescreening. Inserts were amplified using lambda-specific primers and amplicons were sequenced directly by automated sequencing as above. Six clones had SEQ ID NO:7 sequence information, and three were full-length. One full-length clone was subcloned and sequenced entirely in both directions, and used for subsequent expression constructs.

Computer analysis of the structural sequence was done using Lasergene software (DNAstar. Inc, Madison. WI). Alignments with other potassium channel sequences were performed using the CLUSTAL algorithm (Megalign program, Lasergene) and these were utilized to create a dendrogram. The gap penalty and the gap length penalty were 10 each. Hydropathy plots were according to Kyte-Doolittle criteria, averaging over a 9-residue window (Protean program. Lasergene). Post-translational modification sites were identified using pattern searches within the Protean program (Lasergene). Patterns were derived from the Prosite database, and the threshold for matching was 100 percent.

### EXAMPLE X

### Transient transfections

A~1.3 Kb SmaI/ScaI fragment containing the coding region (Nucleotides 390-1718 of **SEQ ID NO:7** and containing entire sequence for **SEQ ID NO:2)** was cloned into pcDNA3 vector (Invitrogen) at the EcoRV site. This cloning strategy introduced an additional methionine and two amino acids (G, A) upstream and in-frame with the authentic initiator methionine. Approximately 3X10⁵ HEK 293 cells (ATCC, Rockville, MD) were transfected with 5 µg of the novel gene in pcDNA3 vector along with 1 µg of green fluorescent protein (GFP) in pEGFP-C 1 vector (Clontech) using the LipoTAXI^{™} transfection kit (Stratagene, La Jolla. CA) as per manufacturer's instructions. Currents were recorded 24-72 hours later. GFP was used to track fluorescent cells for patch-clamping.

### EXAMPLE XI

### Chromosomal Localization of the Novel Gene

Two (2) ng of human genomic DNA (Sigma #D-4642 lot 41019) was used to amplify a 2 KB genomic DNA PCR fragment in a 20 µL reaction using 1X Clontech's Advantage KlenTaq polymerase in 1X buffer (Clontech # 8417-1) and 200 µM dNTPs (final concentration) plus 0.2 µM each primer (final concentration). Forward primer corresponds to nucleotides 1462-1479 of SEQ ID NO: 7; Reverse primer corresponds to nucleotides 1904-1922 of SEQ ID NO: 7. The cycling parameters were as follows: one cycle of 94°C, 1 min. 30 sec.; five cycles of 94°C, 30 sec., 72°C, 3 min.; five cycles of 94°C, 30 sec., 70°C, 3 min.; twenty five cycles of 94°C, 30 sec.. 68°C, 3 min.

The 2 kb product obtained from the above PCR reaction was subcloned into pT7Blue (Novagen # 69820-1). Two clones were sequenced with vector-specific primers. PCR primers (Forward primer: 5' GTGGACATCTCCAAGGTACTAGGA 3' (SEQ ID NO:11) & Reverse primer: 5' TTTACTGACAGGCTGTGTGTGCCA 3' (SEQ ID NO:12)) were designed from the 2 Kb genomic sequence described *supra,* to reproducibly amplify a single 139bp product from 25ng of Genomic DNA via the polymerase chain reaction (PCR) under the following conditions: 2mM MgCl₂, 100µM dNTPs, & 1µM of each primer, for 35 cycles of 94°C for 30s, 65°C for 30s and 72°C for 30s. These primers and conditions were subsequently used to screen the "Research Genetics" BAC library in a 3D fashion by PCR (Bentley, *et al.,* 1992), using 10ng of pool DNA in a 50µl reaction volume. BAC 107 D 14 was identified and streaked to give single colonies after overnight,growth @ 37°C on LB-agar plates +chloramphenicol (12.5µg/ml). Colonies were screened by PCR (Huxley, *et al.,* 1990) to confirm the correct BAC had been isolated. One of the colonies was used as an innoculum for 500mls of LB+ chloramphenicol which was grown @37°C overnight with shaking @ 200rpm. This culture was used to prepare DNA by the alkaline-lysis method (Birnboim & Doly, 1979) and purified by banding on caesium chloride. The DNA was used as a probe for chromosomal-localization by fluroscent in-situ hybridization (Trask B, 1995), described below.

Chromosome spreads were prepared from PHA stimulated human lymphocytes which had been cultured in BrdU (200mg/ml) supplemented medium for 16 hours and in BrdU-free medium for a further 5 hours prior to colcemid treatment. Slides were stained for 30 minutes at room temperature in Hoecsht 33258 (5mg/ml in 2xSSC), then placed face down in 2xSSC on a uv transilluminator for 30 minutes and dehydrated through an ethanol gradient. The chromosomes were denatured by incubation in 70% formamide, 2xSSC at 75°C for 5 minutes.

DNA from BAC 107d14 was biotinylated by nick translation using Bionick Kit according to the manufacturers instructions (BRL). 200ng labelled BAC and 10mg Cot-1 DNA (BRL) were dissolved in 20ml hybridization buffer (50% formamide, 2xSSC, 10% dextran sulphate), denatured, and incubated at 37°C for 15 minutes before being hybridised to the denatured chromosomes at 37°C for 72 hours. Slides were washed for 3x5 minutes in 50% formamide/2xSSC, 2xSSC at 42°C and 0.1 xSSC at 60°C. Slides were blocked for one hour by incubation at room temperature in 4xSSC/5% dried milk/0.05% Triton X-100. Probe detection was with FITC-avidin (5mg/ml) with one round of signal amplification using biotinylated anti-avidin (5mg/ml) (Vector Laboratories) in 4xSSC/ 5% dried milk/ 0.05% Triton X-100. Washes between treatments were 3x 3minutes in 4xSSC/0.05% Triton X-100. Slides were counterstained with 0.1 mg/ml DAPI and analysed on an Axioskop microscope (Zeiss) fitted with a Nu200 CCD camera (Photometrics) and SmartCapture software (Digital Scientific).

For the colocalization of BAC 107d14 and YAC 798E5, DNA (total yeast plus YAC) was labelled with digoxigenin dUTP by nick translation (Boehringer Mannheim kit). 300mg labelled DNA and 3mg Cot-1 DNA were added to the above mix and processed as above: Detection of the BAC was as described above but with rhodamine-avidin. YAC detection used 1 mg/ml anti digoxigenin monoclonal antibody (Boehringer Mannheim), followed by 1/100 anti mouse IgG FITC conjugate, followed by 1/500 anti rabbit IgG FITC cojugate (Sigma). Signal was visible by eye. Paired signals were obtained on Chromosome 19 with BAC 107d14. Co-localization of BAC 107d14 and YAC 798E5 gave signals superimposed on each other indicating a localization to 19q 13.1-13.2.

### References for chromosomal localization

Birnboim, H.C. & Doly, J. (1979). A rapid alkaline extraction procedure for screening recombinant plasmid DNA. *Nucleic Acids Research* 7 1513.
Huxley, C., Green, E.D., & Dunham, I. (1990). Rapid assessment of S.cerevisiae mating type by PCR. *Trends Genet.* 6 236.
Bentley, D.R.,Todd, C., Collins, J., Holland, J., Dunham, I., Hassock, S., Bankier, A. & Gianelli, F. (1992). The development and application of automated gridding for efficient screening of yeast and bacterial ordered libraries. *Genomics* 12, 534 - 541.

Trask, B (1995) In genome analysis: A laboratorory manual. Eds. B. Birren, E. Green and R. Myers, CSHL Press Inc.

### EXAMPLE XII

### Patch Clamp Recording

Currents were recorded with an Axopatch 200A amplifier (Axon Instr., Foster City, CA) using the conventional whole cell, cell-attached, and inside-out configurations. Christian, E.P., *et al.,* J. Membr. Biol., 150:63 (1996). Transfected HEK cells were selected for recording by the presence of GFP labeling during epiflourescent illumination with a Nikon mercury arc light source and FITC filter block (excitation: 485/22 nm, emission: 505 nm). Thin-wall borosilicate glass pipettes were fabricated, sylgarded and fire-polished to a DC resistance of 2 - 8 MΩ. The resistance of patch seals was > 10 GΩ. Liquid junction potentials (14) were corrected for in all experiments examining reversal potentials. Series resistance compensation of>70% was used in all experiments where maximal current was >0.5 nA. Voltage clamp protocols were implemented and data acquisition performed with pClamp 6.0 software (Axon Instr.). Currents were low-pass filtered at (-3 db at 1 or 2 kHz), and then digitized at 3-8 kHz as computer files with a TL-1 interface (Scientific Solutions, Solon, OH).

For seal formation, cells were bathed in normal Ringer solution containing (in mM) 160 NaCl, 4.5 KCl, 1 MgCl₂, 5 HEPES and 2 CaCl₂ (except for excised inside-out patch formation, in which the bathing solution contained 0 CaCl₂) at pH 7.4 (by NaOH) and osmolality ~325 mOsm (by sucrose). For *whole cell recording,* the pipette solution contained (in mM): 160 K aspartate , 2 MgCl₂, 5 HEPES and 1.6 EGTA with either 0.8 CaCl₂ (calculated [Ca²⁺] _{free} = 100 nM; Eqcal software, Biosoft Corp, Cambridge, UK), or 1.6 CaCl₂ (calculated [Ca²⁺] _{free} = 1 µM) at pH 7.2 (by KOH) and osmolality ~315 mOsm (by sucrose). Cells were perfused locally with a solution containing 160 KCl, 2 CaCl₂ 1 Mg Cl₂, 5 HEPES, and 5 glucose at pH 7.4 (by KOH) and osmolality ~325 mOsm. In K⁺ selectivity experiments equimolar Na⁺ was substituted for K⁺. For *cell-attached patches,* the pipette solution contained 160 KCl, 2 CaCl₂, 1 MgCl₂, and 10 HEPES at pH 7.4 (by NaOH) and osmolality -325 mOsm (for determination of unitary conductance at physiological [K⁺]ₒ the pipette contained Ringer). Cells were perfused with Ringer solution +/- 1 µM ionomycin. For *excised inside-out patches,* the pipette solution contained 160 KCl , 2 CaCl₂ 1 MgCl₂ and 10 HEPES at pH 7.4 (by KOH) and osmolality ~325 mOsm. The local perfusion solution (cytoplasmic face) contained 160 K aspartate, 2 MgCl₂, 5 HEPES and 10 EGTA at pH 7.2 (by KOH) and osmolality -329 mOsm with CaCl₂ added at concentrations that yielded the calculated [Ca²⁺]_{free} specified. Perfusion solutions were delivered locally using a solenoid valve system (13). Toxins were purchased from Sigma Chemicals (St. Louis. MO) and were diluted in solution containing 0.01% bovine serum albumin.

### EXAMPLE XIII

### CaP04 transfection protocol for HEK293 cells

### REAGENTS

| Prepare | 1) 2 x HBS | | |
|---|---|---|---|
| | Reagent | Final | grams, dry |
| | HEPES pH7.05 | 50mM | 5.0 |
| | KCl | 10mM | 0.37 |
| | dextrose | 12mM | 1.0 |
| | NaCl | 280mM | 8.0 |
| | Na,HP0₄ (MW 141.96) | 1.5mM | 0.1065g |
| | 500ml total, pH to 7.05 (**NOTE pH is important**). | | |
| | 2) CaCl2 2M | 29.4g | |
| | 100ml total | | |

Stock solutions may be frozen at -20°C. Once opened stocks may be stored at 4°C for 6 weeks.

**METHOD** (for each T75 plate):
1. Plate cells 2-3 days prior to transfection to ensure ~75% confluence immediately prior to transfection. This is really important, it is vital that there are still some gaps on the plate before you start.
2. On day of transfection, prepare DNA in water to a final vol. of 876 ml in 15ml (polystyrene) Falcon tube. 20 mg of hKCa4 plasmid was used (pcDNA3 or pGEN IRES-neo) and 10 µg of pEGFP-C1.
3. Add 124 ml of 2M CaC12 to DNA and mix.
4. Remove media from cells very carefully.
5. Add 8 mls of fresh media very slowly down the side of the plate to ensure cells *are* not dislodged.
6. Add chloroquine to final concentration of 25 mM (0.1 ml of 100x stock (2.5mM)) and swirl gently.
7. Add 1 ml of 2X HBS onto DNA dropwise while bubbling (or mix the solution) for 30 secs.
8. Immediately, add mixture onto cells dropwise covering entire plate, and leave at 37°C for 6-10 hours (usually 6)
9. **NOTE: Leave chloroquine on cells for a MAX of IO hours.**
10. At the end of the day, replace with fresh media and again the following morning. The cells should be ready to check for GFP at that point, and up to 2 days after the transfection.

For stables: On day 4, (48 hours after the last media change), split cells into 150 mm dishes (or T150s for episomal, if you're not going to clone individual colonies) at ~ 1:10 in complete medium.
On day 5, (24 hours after splitting), put cells into selective media; HEK293 take 1 mg/mL G418 and 250 µg/mL hygromycin B.

### EXAMPLE XIV

### Transfection of cho-k1 cells with cellfectin

1. Split cells 2-3 days ahead of time so that they are 50-70% confluent in a T75 flask (e.g., grow CHO-K1 cells in Ham's F12 plus 10% FBS, no antibiotics).
2. Place 30 µg hKCa4 DNA (*either* pGEN IRES-neo or pcDNA3) plus 15 µg pEGFP-Cl in a 15 mL Falcon tube (polystyrene).
3. Add 2 mL of media without serum or antibiotics (in this case Ham's F12 (called SFM (for short)).
4. In a separate polystyrene tube, mix 2 mL of SFM with Cellfectin reagent (50 µL per T75 (LTI# 10362-010).*
5. Combine the contents of the two tubes, mix, and let sit at room temperature 15 minutes.
6. Wash the cells in SFM, then add 5 mL SFM per T75.
7. Add 11 mL SFM to the DNA/Cellfectin mix (bringing the volume to 15 mL).
8. Immediately overlay the DNA/Cellfectin mix onto the cells.
9. Incubate at 37°C in 5% CO₂ for 5 hours.
10. Remove DNA/Cellfectin mix and replace with Ham's F12 plus 10% FBS.
11. Next day change media and check for EGFP.

For stables: On day 4, (48 hours after the last media change), split cells into 150 mm dishes (or T150s for episomal, if clone individual colonies are not required) at ~1:10 in complete medium.

On day 5, (24 hours after splitting), put cells into selective media; CHO-K1 take 1 mg/mL G418 and 300 µg/mL hygromycin B.

### EXAMPLE XV

### Baculovirus expression system for hKCa4 (SEQ ID NO:2)

A recombinant virus bearing the coding region of hKCa4 was constructed using the Bac-To-Bac Baculovirus Expression System (Life Technologies, Gaithersburg, MD, cat. # 10359-016). The coding region of hKCa4 was subcloned into the donor vector pFastBac 1 by cleaving the pcDNA3 construct (described in JBC 272 (52): 32723-32726) with EcoRI (5') and XhoI (3') sites, releasing the 1356 bp insert, and cloned into these sites within pFastBac1. This strategy retains the additional methionine and two amino acids (G. A) upstream and in-frame with the authentic initiator methionine. Transposition was as detailed in the protocol from Life Technologies. Gaithersburg, MD, with the following notes: transformation plates were incubated 48 hours at 37°C for transposition to occur. Restreaked colony plates were also incubated 48 hours at 37°C to ensure white phenotype. For transfecting Sf9 cells, 8 µL of bacmid miniprep DNA and 5 µL Cellfectin was used. The DNA plus Cellfectin mixes were allowed to incubate 20 minutes before applying to cells.

Passage two stocks of the virus were obtained by infecting 3 X 10⁶ Sf9 cells in a T25 flask with 100 µL of the passage one virus (the transfection supernatant produced above) in a total volume of 5 mL. The infection was allowed to proceed for three days at 27°C in a humidified chamber, and the cell culture supernatants were collected. This passage two virus was quantitated using the BacPAK Baculovirus Rapid Titer Kit (Clontech, Palo Alto, CA, cat # K1599-1) or by conventional plaque assay (Life Technologies, Gaithersburg, MD, Baculovirus Expression Training course manual, 3.54-3.58).
High-titer passage three viral stocks were generated from passage two virus by infecting Sf9 cells in Sf900II medium in suspension at a MOI of 0.1 and a cell density of 4.0 X 10⁶/mL. After allowing virus to adhere to cells for one hour at room temperature with constant agitation, cells were harvested and resuspended in fresh medium to a cell density of 1.0 X 10⁶/mL. The infection was allowed to continue for two days in suspension at 27°C and 110 rpm. The supernatant (passage three virus) was harvested and quantitated as for the passage 2 virus. Infections for protein production were performed at various MOIs and times of harvest as per the Bac-To-Bac manual. Cells were examined by patch clamp analysis and/or ¹²⁵I-charybdotoxin (CTX) binding.

### EXAMPLE XVI

### Scintillation proximity assay (SPA)

This method has been successfully applied to receptor binding assays by immobilizing receptors directly on to SPA beads via a number of coupling methods (Bosworth N and Towers P, Nature, 342: 167-168, 1989; Undenfriend S et. al., Anal. Biochem. 161: 494-500. 1987). The most commonly used beads are those coupled to Wheat Germ Agglutinin (WGA), which bind to N-acetyl b-D glucosamine residues in membrane glycolipids and glycoproteins.. (Nagata Y and Burger MM, JBC, 249: 3116-3122, 1974). Once immobilized, the receptor is close enough to the bead so that, should a suitably radiolabelled ligand bind to the receptor, it will be in close enough proximity to stimulate the bead to emit light. Any unbound radioligand is too distant from the bead to transfer energy and goes undetected. The bead therefore only detects the population of ligand molecules which are receptor-bound. The discrimination of binding by proximity means that no separation of bound and free ligand is required, as in traditional methods. SPA is an extremely powerful technique when large numbers of samples are required to be assayed simultaneously, as during a high-throughput assay for compounds (News, 27: November 1996, Amersham Life Sciences).

All reagents for the SPA assay (except buffer solutions), were ordered from Amersham Life Sciences, Arlington Heights, IL.

| Assay Buffer: | Wash Buffer: |
|---|---|
| 5mM NaCl | 200mM NaCl |
| 5mM KCl | 20mM HEPES |
| 10mM HEPES | pH 8.0 with Tris base |
| 6mM glucose | |
| bring to pH 8.4 with TRIS base | |

assay run in 96-well SPA microtiter plates
0.5-3 mg per well of wheat germ agglutinin-coated SPA beads (Amersham, Cat# RPNQ0001).
5-30 ug of KCa4-HEK cell membranes per well
50 pM of 125I charybdotoxin (CTX, NEN, Boston, MA)
non specific defined with 50 nM cold charybdotoxin (Bachem, King of Prussia, PA).

### ASSAY:

50 ul beads + membranes
50 ul assay buffer
50 ul experimental compound or nonspecific or buffer (defines total binding)
50 ul ligand

Membranes are allowed to pre-incubate with SPA beads for 1-2 hours; unbound membranes are washed away from beads with centrifugation.

Assay buffer is added to each well, followed by compounds, bead-membranes and ligand

The assay incubates with shaking for 1 hour at room temperature

The beads are allowed to settle overnight, then the plate is counted in a Top Count scintillation counter (Packard, Meriden. CT).

### EXAMPLE XVII

### Anergic T-cell line

3926 alloT is one of a number of HLA-DR4Dw4-specific alloreactive T cell lines which were produced according to standard methodology. Twenty-five million peripheral blood mononuclear cells from a donor not carrying the HLA-DR4 allotype were mixed with the same number of irradiated blood mononuclear cells from a donor carrying the HLA-DR4Dw4 allotype in 5ml of RPMI 1640 cell growth medium supplemented with glutamine and 5% human serum. After 14 days culture, viable cells were isolated by centrifugation over a Lymphopaque density gradient, and 5 million cells added to a further twenty-five million -irradiated blood mononuclear cells from the same HLA-DR4Dw4-positive donor:
Recombinant hIL-2 (2 Units/ml) was added on day 4, and every 3 days afterwards. The line was maintained by periodic restimulation and IL-2 addition as described. The HLA-DR4Dw4 allospecificity of the lines was confirmed by demonstrating that they were able to proliferate in response to HLA-DR4Dw4 homozygous EBV B cells (JAH cell line) and HLA-DR4Dw4-expressing L cell transfectants.

### maintenance

A frozen vial of the 3926 T cell line from UK was thawed and maintained in RPMI medium with 10% FCS and glutamine. The cells were maintained on a "rest-stimulation" protocol by stimulating them with soluble anti-CD3 (2.5 mg/ml catalog # 30100D plus 2.5 mg/ml catalog #30110D from PharMingen, San Diego, CA) plus mitomycin-c-treated untyped human PBMC to provide the costimulatory signal. The PBMC would also act to crosslink the soluble CD3 on the TCR. After two days, 5 Units/ml of human recombinant IL-2 (Boehringer Mannheim. Indianapolis, IN) was added to the cells. During this stimulation step, the T cells were seen to clump and proliferate. T cells used in experiments were rested at least one week after addition of IL-2 and were separated from debris and dead PBMC by Histopaque 1077 (Sigma, St. Louis, MO) density gradient centrifugation.

### pretreatment conditions for resting, activated and anergic cells

Purified 3926 allo T cells (4 x 105 cells/well) were incubated in 24-well plates which were uncoated ("resting" cells) or coated with anti-CD3 plus anti-CD28 (2.5 mg/ml each of the two anti-CD3 antibodies plus 20 mg/ml catalog # 33740D- "two signal"); or in order to induce anergy, with 2.5 mg/ml each of the two human anti-CD3 antibodies in a 6 well plate ("one signal"). All antibodies were diluted in PBS. The cells were washed away from the antibodies after one day and rested in fresh wells for two more days (total of three days following exposure to the antibodies) at which time the T cells were patch-clamped for KCa channels as described elsewhere in the document.

### EXAMPLE XVIII

### Cloning of murine KCa4

An effort to clone a rodent KCa4 gene was initiated because there are several rodent models of autoimmunity available, and it was important to verify that the rodent genes are essentially the same as the human gene, for these animal models to be used. An earlier publication had already shown that the KCa channel in rodent lymphocytes behaves very similar to its human counterpart by electrophysiology; it is low in resting cells and is dramatically upregulated upon lymphocyte activation (Mahaut-Smith and Mason J. Physiol. 1991).

The amino acid sequence of human KCa4 (SEQ ID NO:3) was used as a query sequence in a TBLASTN search against the embl est database. One mouse EST, accession number W30402, showed 79% identity to hKCa4 at the amino terminus (N-teem.) (over an 89 amino acid region) and 63% identity to hKCa4 at the carboxy terminus (C-term.) (over only a 20 amino acid region). The conceptual translation of the EST resulted in a truncated protein compared to that expected for a 6 transmembrane ion channel subunit, wherein the portion of the molecule corresponding to the third transmembrane region through all but the last 20 amino acids are deleted. However, because of the high homology to the human sequence, primers were designed upstream of the putative start methionine and downstream of the putative stop codon to PCR "full-length" mouse KCa4. The forward primer sequence is corresponds to W30402 nucleotides 20-42. The reverse primer sequence corresponds to W30402 nucleotides 413-436.

cDNA was made from the mouse erythroleukemic cell line MEL-C88 (Deisseroth, A., *et al.,* Cell 15: 55 (1978)). Cells were cultured in αMEM medium containing 10% fetal bovine serum, 100 units per mL of penicillin and 100 µg per mL of streptomycin. Total RNA was isolated using the RNeasy Midi prep Kit from Qiagen, Inc., Chatsworth, CA, cat. # 75142 and treated with DNAse I, Life Technologies, Gaithersburg, MD, cat. # 18068-015, according to manufacturer's instructions. cDNA synthesis was with SuperScript II reverse transcriptase (Life Technologies, Gaithersburg, MD, cat. # 18064-014) using the Marathon cDNA synthesis primer (Marathon cDNA Amplification kit, Clontech, Palo Alto, CA, cat. # K 1802-1), according to the SuperScript II protocol. First strand cDNA was digested with RNAse H (Life Technologies, Gaithersburg, MD, cat. # YD1220) and used at ~1 ng per PCR.

mKCa4 products were amplified from the cDNA in 25 µL reactions using Advantage^{™} KlenTaq polymerase (Clontech, Palo Alto, CA # 8417-1) according to the manufacturer's recommendations, with the addition of 5% final concentration DMSO. Cycling parameters were as follows: one cycle at 94°C for 1.5 minutes; thirty cycles of: 94°C thirty seconds, 65°C forty five seconds, 68°C one minute. Ten µL of each reaction was analyzed on a 1% agarose gel containing 0.5 µg/mL final concentration ethidium bromide in TAE buffer as in Sambrook et al, Molecular Cloning Lab Manual, Second Edition, Cold Spring Harbor Press 1989. using 600 ng of 1kb DNA ladder as marker (Life Technologies, Gaithersburg, MD. cat # 15615). The remaining 15 µL of the reactions were purified using the Qiaquick PCR purification kit according to the manufacturer's recommendations (Qiagen, Inc., Chatsworth, CA, cat # 28104), quantitated by Optical Density measurements at 260 nm, and subjected to automated sequencing (ABI PRISM Dye Terminator Cycle sequencing on ABI PRISM 377 automated sequencer).

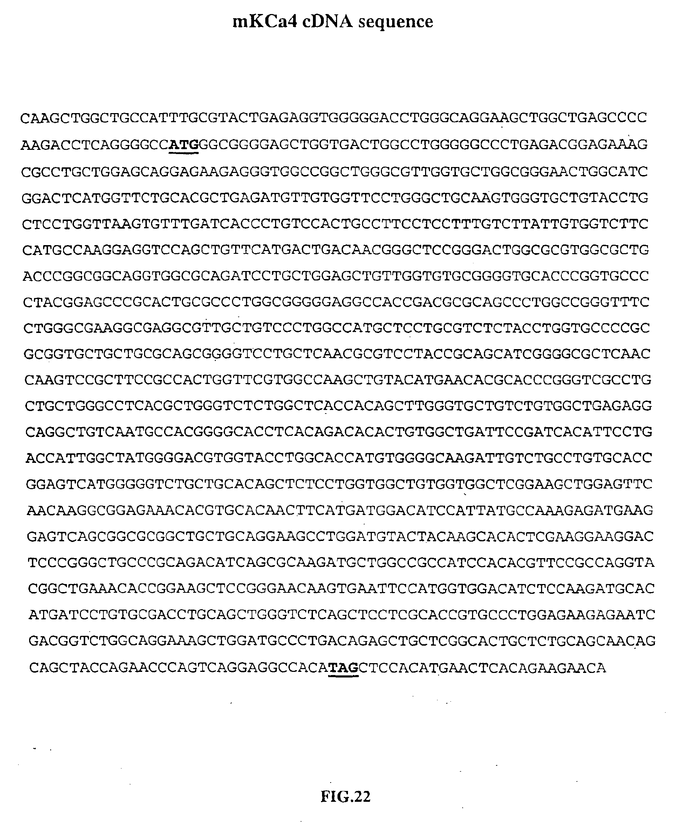

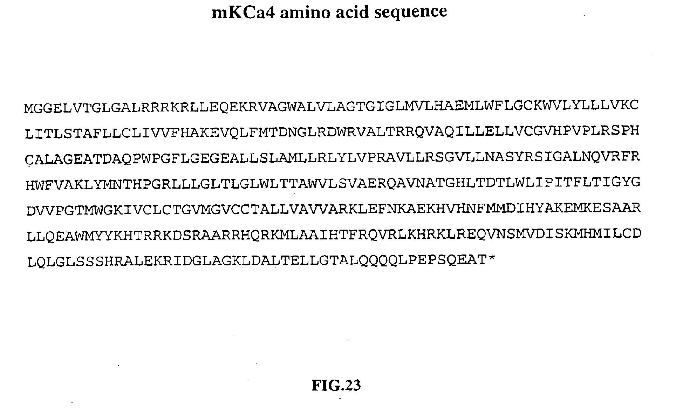

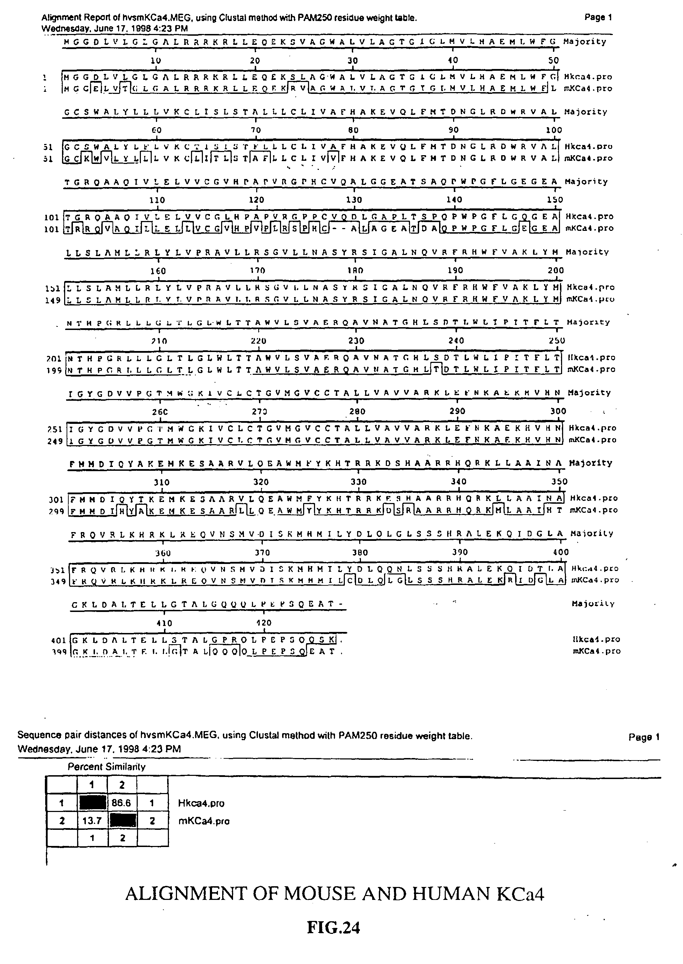

The full-length coding region nucleotide sequence, translated amino acid sequence and amino acid alignment of the mouse ortholog and human KCa4 sequences are shown in FIG.22 (SEQ ID NO: 13), FIG.23 (SEQ ID NO: 14) (murine homolog (ortholog of human Kca4, SEQ ID NO:3)), and FIG.24, respectively.

### EXAMPLE XIX

### Isolation of microglia & transcripts

Human fetal brain tissue (from 16- to 22-week-old fetuses) was obtained commercially from the Anatomic Gift Foundation (Woodbine. GA). Cerebral hemispheres were placed in chilled, sterile, calcium-free, magnesium-free Hanks Balanced Salt Solution (HBSS, # 14170-112, Gibco-BRL. Gaithersburg, MD). Meninges were removed with sterile forceps and tissue was dissociated initially by repeated trituration through sterile pipets. Tissue was incubated with 0.05% Trypsin/0.53 mM EDTA (# 25300-054, Gibco-BRL, Gaithersburg, MD) and 0.15 mg/ml DNAse (# D-5025, Sigma Chemical Co.. St. Louis, MO) at 37°C for 45 minutes with gentle shaking. 10% Fetal Bovine Serum (FBS, # 160001-036, Gibco-BRL, Gaithersburg, MD) was added to the suspension to stop trypsinization. The cells were then passed through 210 µm and 149 µm polypropylene mesh (# 08-670-188 and # 08-670-189. Fisher Scientific, Pittsburgh, PA) sequentially. The filtrate was washed twice and resuspended in complete media [DMEM (with high glucose, L-glutamine and HEPES), Gibco # 12430-054; 100 U-µg/ml penicillin/streptomycin, Gibco # 15070-030; 10% FBS, Gibco # 160001-036]. Cells were plated at density of 80 million/75 cm² flask (# 12-565-52, Fisher Scientific, Pittsburgh, PA). Cultures were incubated at 37°C, 5% CO₂ for 2 weeks (with one media change after one week in culture to remove cellular debris). Mature cultures consisted of astrocytes, neurons, microglia and oligodendrocytes. The microglial population is enriched in the supernatant, which was harvested (astrocytes and oligodendrocytes stick to the plate). Cells were lysed in Buffer RLT (QIAGEN) with 0.01% b-ME (Sigma Cat.# M7154) and frozen at -80°C until use. Lysed cells were thawed on ice, and processed through a QIAshredder (QIAGEN Cat.# 79654) to homogenize.

Total RNA was then prepared with the RNeasy Mini Kit (QIAGEN Cat.#74103) according to manufacturer's directions. Concentration was determined by absorbance at 260 nm. CDNA was prepared from 3.2 ug of microglial RNA by standard reverse transcription using SuperScript II reverse transcriptase kit (Life Technologies, Gaithersburg, MD, cat. # 18064-014). PCR was performed at 65 degrees annealing. Human KCa4 specific primers used were: Forward primer: positions 862-883 of SEQ ID NO:1; and reverse primer corresponding to positions 1659-1680 of SEQ ID NO: 1.

Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### SEQUENCE LISTING

(I) GENERAL INFORMATION
   (i) APPLICANT:
   (ii) TITLE OF THE INVENTION: HUMAN LYMPH NODE DERIVED CALCIUM ACTIVATED POTASSIUM CHANNEL
   (iii) NUMBER OF SEQUENCES: 14
   (iv) CORRESPONDENCE ADDRESS:
      (A) ADDRESSEE: ZENECA Pharmaceuticals, Inc.
      (B) STREET: 1800 Concord Pike
      (C) CITY: Wilmington
      (D) STATE: DE
      (E) COUNTRY: USA
      (F) ZIP: 19850-5437
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: (UK)9714760.7
      (B) FILING DATE: 15-JUL-1997

      (A) APPLICATION NUMBER: (UK)9721366.4
      (B) FILING DATE: 09-OCT-1997
   (viii) ATTORNEY/AGENT INFORMATION:
      (A) NAME: Higgins, Patrick H.
      (B) REGISTRATION NUMBER: 39.709
      (C) REFERENCE/DOCKET NUMBER: PHM.70235
   (ix) TELECOMMUNICATION INFORMATION:
      (A) TELEPHONE: 302.886.4889
      (B) TELEFAX: 302.886.8221
      (C) TELEX:
(2) INFORMATION FOR SEQ ID NO: 1:
   (i) SEQUENCE CHARACTERISTICS:
      (A) LENGTH: 2261 base pairs
      (B) TYPE: nucleic acid
      (C) STRANDEDNESS: single
      (D) TOPOLOGY: linear
   (xi) SEQUENCE DESCRIPTION: SEQ ID NO: 1:
(2) INFORMATION FOR SEQ ID NO:2:
   (i) SEQUENCE CHARACTERISTICS:
      (A) LENGTH: 1284 base pairs
      (B) TYPE: nucleic acid
      (C) STRANDEDNESS: single
      (D) TOPOLOGY: linear
   (xi) SEQUENCE DESCRIPTION: SEQ ID NO:2:
   ATGGGCGGGG ATCTGGTGCT TGGCCTGGGG GCCTTGAGAC GCCGAAAGCG CTTGCTGGAG   60
   CAGGAGAAGT CTCTGGCCGG CTGGGCACTG GTGCTGGCAG GAACTGGCAT TGGACTCATG  120
   GTGCTGCATG CAGAGATGCT GTGGTTCGGG GGGTGCTCGT GGGCGCTCTA CCTGTTCCTG  180
   GTTAAATGCA CGATCAGCAT TTCCACCTTC TTACTCCTCT GCCTCATCGT GGCCTTTCAT  240
   GCCAAAGAGG TCCAGCTGTT CATGACCGAC AACGGGCTGC GGGACTGGCG CGTGGCGCTG  300
   ACCGGGCGGC AGGCGGCGCA GATCGTGCTG GAGCTGGTGG TGTGTGGGCT GCACCCGGCG  360
   CCCGTGCGGG GCCCGCCGTG CGTGCAGGAT TTAGGGGCGC CGCTGACCTC CCCGCAGCCC  420
   TGGCCGGGAT TCCTGGGCCA AGGGGAAGCG CTGCTGTCCC TGGCCATGCT GCTGCGTCTC  480
   TACCTGGTGC CCCGCGCCGT GCTCCTGCGC AGCGGCGTCC TGCTCAACGC TTCCTACCGC  540
   AGCATCGGCG CTCTCAATCA AGTCCGCTTC CGCCACTGGT TCGTGGCCAA GCTTTACATG  600
   AACACGCACC CTGGCCGCCT GCTGCTCGGC CTCACGCTTG GCCTCTGGCT GACCACCGCC  660
   TGGGTGCTGT CCGTGGCCGA GAGGCAGGCT GTTAATGCCA CTGGGCACCT TTCAGACACA  720
   CTTTGGCTGA TCCCCATCAC ATTCCTGACC ATCGGCTATG GTGACGTGGT GCCGGGCACC  780
   ATGTGGGGCA AGATCGTCTG CCTGTGCACT GGAGTCATGG GTGTCTGCTG CACAGCCCTG  840
   CTGGTGGCCG TGGTGGCCCG GAAGCTGGAG TTTAACAAGG CAGAGAAGCA CGTGCACAAC  900
   TTCATGATGG ATATCCAGTA TACCAAAGAG ATGAAGGAGT CCGCTGCCCG AGTGCTACAA  960
   GAAGCCTGGA TGTTCTACAA ACATACTCGC AGGAAGGAGT CTCATGCTGC CCGCAGGCAT 1020
   CAGCGCAAGC TGCTGGCCGC CATCAACGCG TTCCGCCAGG TGCGGCTGAA ACACCGGAAG 1080
   CTCCGGGAAC AAGTGAACTC CATGGTGGAC ATCTCCAAGA TGCACATGAT CCTGTATGAC 1140
   CTGCAGCAGA ATCTGAGCAG CTCACACCGG GCCCTGGAGA AACAGATTGA CACGCTGGCG 1200
   GGGAAGCTGG ATGCCCTGAC TGAGCTGCTT AGCACTGCCC TGGGGCCGAG GCAGCTTCCA 1260
   GAACCCAGCC AGCAGTCCAA GTAG                                       1284
(2) INFORMATION FOR SEQ ID NO:3:
   (i) SEQUENCE CHARACTERISTICS:
      (A) LENGTH: 427 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3.
(2) INFORMATION FOR SEQ ID NO:4:
   (i) SEQUENCE CHARACTERISTICS:
      (A) LENGTH: 561 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Met Pro Gly Pro Arg Ala Ala Cys Ser Glu Pro Asn Pro Cys Thr Gln
    1               5                  10                  15
   Val Val Met Asn Ser His Ser Tyr Asn Gly Ser Val Gly Arg Pro Leu
               20                  25                  30
   Gly Ser Gly Pro Gly Ala Leu Gly Arg Asp Pro Pro Asp Pro Glu Ala
           35                  40                  45
   Gly His Pro Pro Gln Pro Pro His Ser Pro Gly Leu Gln Val Val Val
       50                  55                  60
   Ala Lys Ser Glu Pro Ala Arg Pro Ser Pro Gly Ser Pro Arg Gly Gln
   65                  70                  75                  80
   Pro Gln Asp Gln Asp Asp Asp Glu Asp Asp Glu Glu Asp Glu Ala Gly
                   85                  90                  95
   Arg Gln Arg Ala Ser Gly Lys Pro Ser Asn Val Gly His Arg Leu Gly
               100                 105                 110
   His Arg Arg Ala Leu Phe Glu Lys Arg Lys Arg Leu Ser Asp Tyr Ala
           115                 120                 125
   Leu Ile Phe Gly Met Phe Gly Ile Val Val Met Val Thr Glu Thr Glu
       130                 135                 140
   Leu Ser Trp Gly Val Tyr Thr Lys Glu Ser Leu Tyr Ser Phe Ala Leu
   145 150 155 160
   Lys Cys Leu Ile Ser Leu Ser Thr Ala Ile Leu Leu Gly Leu Val Val
   165 170 175
   Leu Tyr His Ala Arg Glu Ile Gln Leu Phe Met Val Asp Asn Gly Ala
   180 185 190
   Asp Asp Trp Arg Ile Ala Met Thr Cys Glu Arg Val Phe Leu Ile Ser
   195 200 205
   Leu Glu Leu Ala Val Cys Ala Ile His Pro Val Pro Gly His Tyr Arg
   210 215 220
   Phe Thr Trp Thr Ala Arg Leu Ala Phe Thr Tyr Ala Pro Ser Val Ala
   225 230 235 240
   Glu Ala Asp Val Asp Val Leu Leu Ser Ile Pro Met Phe Leu Arg Leu
   245 250 255
   Tyr Leu Leu Gly Arg Val Met Leu Leu His Ser Lys Ile Phe Thr Asp
   260 265 270
   Ala Ser Ser Arg Ser Ile Gly Ala Leu Asn Lys Ile Thr Phe Asn Thr
   275 280 285
   Arg Phe Val Met Lys Thr Leu Met Thr Ile Cys Pro Gly Thr Val Leu
   290 295 300
   Leu Val Phe Ser Ile Ser Ser Trp Ile Ile Ala Ala Trp Thr Val Arg
   305 310 315 320
   Val Cys Glu Arg Tyr His Asp Lys Gln Glu Val Thr Ser Asn Phe Leu
   325 330 335
   Gly Ala Met Trp Leu Ile Ser Ile Thr Phe Leu Ser Ile Gly Tyr Gly
   340 345 350
   Asp Met Val Pro His Thr Tyr Cys Gly Lys Gly Val Cys Leu Leu Thr
   355 360 365
   Gly Ile Met Gly Ala Gly Cys Thr Ala Leu Val Val Ala Val Val Ala
   370 375 380
   Arg Lys Leu Glu Leu Thr Lys Ala Glu Lys His Val His Asn Phe Met
   385 390 395 400
   Met Asp Thr Gln Leu Thr Lys Arg Val Lys Asn Ala Ala Ala Asn Val
                       405                    410                    415
   Leu Arg Glu Thr Trp Leu Ile Tyr Lys His Thr Arg Leu Val Lys Lys
                   420                    425                    430
   Pro Asp Gln Ala Arg Val Arg Lys His Gln Arg Lys Phe Leu Gln Ala
               435                    440                    445
   Ile His Gln Ala Gln Lys Leu Arg Ser Val Lys Ile Glu Gln Gly Lys
           450                    455                    460
   Leu Asn Asp Gln Ala Asn Thr Leu Thr Asp Leu Ala Lys Thr Gln Thr
   465                    470                    475                    480
   Val Met Tyr Asp Leu Val Ser Glu Leu His Ala Gln His Glu Glu Leu
                       485                    490                    495
   Glu Ala Arg Leu Ala Thr Leu Glu Ser Arg Leu Asp Ala Leu Gly Ala
                   500                    505                    510
   Ser Leu Gln Ala Leu Pro Gly Leu Ile Ala Gln Ala Ile Arg Pro Pro
               515                    520                    525
   Pro Pro Pro Leu Pro Pro Arg Pro Gly Pro Gly Pro Gln Asp Gln Ala
           530                    535                    540
   Ala Arg Ser Ser Pro Cys Arg Trp Thr Pro Val Ala Pro Ser Asp Cys
   545                    550                    555                    560
   Gly
(2) INFORMATION FOR SEQ ID NO:5:
   (i) SEQUENCE CHARACTERISTICS:
      (A) LENGTH: 579 amino acids
      (B) TYPE: amino acid
      (C) STRANDEDNESS: single
      (D) TOPOLOGY: linear
   (xi) SEQUENCE DESCRIPTION: SEQ ID NO:5:
   Met Ser Ser Cys Arg Tyr Asn Gly Gly Val Met Arg Pro Leu Ser Asn
   1               5                   10                  15
   Leu Ser Ser Ser Arg Arg Asn Leu His Glu Met Asp Ser Glu Ala Gln
   20 25 30
   Pro Leu Gln Pro Pro Ala Ser Val Val Gly Gly Gly Gly Gly Ala Ser
   35 40 45
   Ser Pro Ser Ala Ala Ala Ala Ala Ser Ser Ser Ala Pro Glu Ile Val
   50 55 60
   Val Ser Lys Pro Glu His Asn Asn Ser Asn Asn Leu Ala Leu Tyr Gly
   65 70 75 80
   Thr Gly Gly Gly Gly Ser Thr Gly Gly Gly Gly Gly Gly Gly Gly Gly
   85 90 95
   Gly Gly Gly Ser Gly His Gly Ser Ser Ser Gly Thr Lys Ser Ser Lys
   100 105 110
   Lys Lys Asn Gln Asn Ile Gly Tyr Lys Leu Gly His Arg Arg Ala Leu
   115 120 125
   Phe Lys Arg Lys Arg Leu Ser Asp Tyr Ala Leu Ile Phe Gly Met Phe
   130 135 140
   Gly Ile Val Val Met Val Ile Glu Thr Glu Leu Ser Trp Gly Ala Tyr
   145 150 155 160
   Asp Lys Ala Ser Leu Tyr Ser Leu Ala Leu Lys Cys Leu Ile Ser Leu
   165 170 175
   Ser Thr Ile Ile Leu Leu Gly Leu Ile Ile Val Tyr His Ala Arg Glu
   180 185 190
   Ile Gln Leu Phe Met Val Asp Asn Gly Ala Asp Asp Trp Arg Ile Ala
   195 200 205
   Met Thr Tyr Glu Arg Ile Phe Phe Ile Cys Leu Glu Ile Leu Val Cys
   210 215 220
   Ala Ile His Pro Ile Pro Gly Asn Tyr Thr Phe Thr Trp Thr Ala Arg
   225 230 235 240
   Leu Ala Phe Ser Tyr Ala Pro Ser Thr Thr Thr Ala Asp Val Asp Ile
   245 250 255
   Ile Leu Ser Ile Pro Met Phe Leu Arg Leu Tyr Leu Ile Ala Arg Val
   260 265 270
   Met Leu Leu His Ser Lys Leu Phe Thr Asp Ala Ser Ser Arg Ser Ile
   275 280 285
   Gly Ala Leu Asn Lys Ile Asn Phe Asn Thr Arg Phe Val Met Lys Thr
   290 295 300
   Leu Met Thr Ile Cys Pro Gly Thr Val Leu Leu Val Phe Ser Ile Ser
   305 310 315 320
   Leu Trp Ile Ile Ala Ala Trp Thr Val Arg Ala Cys Glu Arg Tyr His
   325 330 335
   Asp Gln Gln Asp Val Thr Ser Asn Phe Leu Gly Ala Met Trp Leu Ile
   340 345 350
   Ser Ile Thr Phe Leu Ser Ile Gly Tyr Gly Asp Met Val Pro Asn Thr
   355 360 365
   Tyr Cys Gly Lys Gly Val Cys Leu Leu Thr Gly Ile Met Gly Ala Gly
   370 375 380
   Cys Thr Ala Leu Val Val Ala Val Val Ala Arg Lys Leu Glu Leu Thr
   385 390 395 400
   Lys Ala Glu Lys His Val His Asn Phe Met Met Asp Thr Gln Leu Thr
   405 410 415
   Lys Arg Val Lys Asn Ala Ala Ala Asn Val Leu Arg Glu Thr Trp Leu
   420 425 430
   Ile Tyr Lys Asn Thr Lys Leu Val Lys Lys Ile Asp His Ala Lys Val
   435 440 445
   Arg Lys His Gln Arg Lys Phe Leu Gln Ala Ile His Gln Leu Arg Ser
   450 455 460
   Val Lys Met Glu Gln Arg Lys Leu Asn Asp Gln Ala Asn Thr Leu Val
   465 470 475 480
   Asp Leu Ala Lys Thr Gln Asn Ile Met Tyr Asp Met Ile Ser Asp Leu
   485 490 495
   Asn Glu Arg Ser Glu Asp Phe Glu Lys Arg Ile Val Thr Leu Glu Thr
   500 505 510
   Lys Leu Glu Thr Leu Ile Gly Ser Ile His Ala Leu Pro Gly Leu Ile
   515 520 525
   Ser Gln Thr Ile Arg Gln Gln Gln Arg Asp Phe Ile Glu Thr Gln Met
   530 535 540
   Glu Asn Tyr Asp Lys His Val Thr Tyr Asn Ala Glu Arg Ser Arg Ser
   545 550 555 560
   Ser Ser Arg Arg Arg Arg Ser Ser Ser Thr Ala Pro Pro Thr Ser Ser
   565 570 575
   Glu Ser Ser
(2) INFORMATION FOR SEQ ID NO:6:
   (i) SEQUENCE CHARACTERISTICS:
      (A) LENGTH: 551 amino acids
      (B) TYPE: amino acid
      (C) STRANDEDNESS: single
      (D) TOPOLOGY: linear
   (xi) SEQUENCE DESCRIPTION: SEQ ID NO:6:
   Met Ser Ser Cys Lys Tyr Ser Gly Gly Val Met Lys Pro Leu Ser Arg
   1 5 10 15
   Leu Ser Ala Ser Arg Arg Asn Leu Ile Glu Ala Glu Pro Glu Gly Gln
   20 25 30
   Pro Leu Gln Leu Phe Ser Pro Ser Asn Pro Pro Glu Ile Ile Ile Ser
   35 40 45
   Ser Arg Glu Asp Asn His Ala His Gln Thr Leu Leu His His Pro Asn
   50 55 60
   Ala Thr His Asn His Gln His Ala Gly Thr Thr Ala Gly Ser Thr Thr
   65 70 75 80
   Phe Pro Lys Ala Asn Lys Arg Lys Asn Gln Asn Ile Gly Tyr Lys Leu
   85 90 95
   Gly His Arg Arg Ala Leu Phe Glu Lys Arg Lys Arg Leu Ser Asp Tyr
   100 105 110
   Ala Leu Ile Phe Gly Met Phe Gly Ile Val Val Met Val Ile Glu Thr
   115 120 125
   Glu Leu Ser Trp Gly Leu Tyr Ser Lys Asp Ser Met Phe Ser Leu Ala
   130 135 140
   Leu Lys Cys Leu Ile Ser Leu Ser Thr Ile Ile Leu Leu Gly Leu Ile
   145 150 155 160
   Ile Ala Tyr His Thr Arg Glu Val Gln Leu Phe Val Ile Asp Asn Gly
   165 170 175
   Ala Asp Asp Trp Arg Ile Ala Met Thr Tyr Glu Arg Ile Leu Tyr Ile
   180 185 190
   Ser Leu Glu Met Leu Val Cys Ala Ile His Pro Ile Pro Gly Glu Tyr
   195 200 205
   Lys Phe Phe Trp Thr Ala Arg Leu Ala Phe Ser Tyr Thr Pro Ser Arg
   210 215 220
   Ala Glu Ala Asp Val Asp Ile Ile Leu Ser Ile Pro Met Phe Leu Arg
   225 230 235 240
   Leu Tyr Leu Ile Ala Arg Val Met Leu Leu His Ser Lys Leu Phe Thr
   245 250 255
   Asp Ala Ser Ser Arg Ser Ile Gly Ala Leu Asn Lys Ile Asn Thr Arg
   260 265 270
   Phe Val Met Lys Thr Leu Met Thr Ile Cys Pro Gly Thr Val Leu Leu
   275 280 285
   Met Phe Ser Ile Ser Leu Trp Ile Ile Ala Ala Trp Thr Val Arg Val
   290 295 300
   Cys Glu Arg Tyr His Asp Gln Gln Asp Val Thr Ser Asn Phe Leu Gly
   305 310 315 320
   Ala Met Trp Leu Ile Ser Ile Thr Phe Leu Ser Ile Gly Tyr Gly Asp
   325 330 335
   Met Val Pro His Thr Tyr Cys Gly Lys Gly Val Cys Leu Leu Thr Gly
   340 345 350
   Ile Met Gly Ala Gly Cys Thr Ala Leu Val Val Ala Val Val Ala Arg
   355 360 365
   Lys Leu Glu Leu Thr Lys Ala Glu Lys His Val His Asn Phe Met Met
   370 375 380
   Asp Thr Gln Leu Thr Lys Arg Ile Lys Asn Ala Ala Ala Asn Val Leu
   385 390 395 400
   Arg Glu Thr Trp Leu Ile Tyr Lys His Thr Lys Leu Leu Lys Lys Ile
   405 410 415
   Asp His Ala Lys Val Arg Lys His Gln Arg Lys Phe Leu Gln Ala Ile
   420 425 430
   His Gln Leu Arg Gly Val Lys Met Glu Gln Arg Lys Leu Ser Asp Gln
   435 440 445
   Ala Asn Thr Leu Val Asp Leu Ser Lys Met Gln Asn Val Met Tyr Asp
   450 455 460
   Leu Ile Thr Glu Leu Asn Asp Arg Ser Glu Asp Leu Glu Lys Gln Ile
   465 470 475 480
   Gly Ser Leu Glu Ser Lys Leu Glu His Leu Thr Ala Ser Phe Asn Ser
   485 490 495
   Leu Pro Leu Leu Ile Ala Asp Thr Leu Arg Gln Gln Gln Gln Gln Leu
   500 505 510
   Leu Thr Ala Phe Val Glu Ala Arg Gly Ile Ser Val Ala Val Gly Thr
   515 520 525
   Ser His Ala Pro Pro Ser Asp Ser Pro Ile Gly Ile Ser Ser Thr Ser
   530 535 540
   Phe Pro Glu Phe Leu Ile Phe
   545 550
(2) INFORMATION FOR SEQ ID NO:7:
   (i) SEQUENCE CHARACTERISTICS:
      (A) LENGTH: 2238 base pairs
      (B) TYPE: nucleic acid
      (C) STRANDEDNESS: single
      (D) TOPOLOGY: linear
   (xi) SEQUENCE DESCRIPTION: SEQ ID NO:7:
   GTCCTTCGGT GTCTGGGTGT GGTGAGTAGA GGTGTGTGTC ACAAAGTACA GACCATTGTG    60
   TGTGACAAAG CCCATCGTGT GTCTGTGTGT GTCTTTATCC ACGTGGATGG ACGTCTCTTT   120
   CTTGCTCTGC CCCAAGACAC ACCCTAGCCC CTCCTTATTC TCAAAAGGGG GAGCTGGGGA   180
   GCCTCCCCCT ACCCTGGGGC CTCCCCTGCC CCTCCCCGCC CTGCCTGGCC GTCACCACTC   240
   CCCAGAGGGC ACAGGGCTCT GCTGTGCCTC AGAGCAAAAG TCCCAGAGCC AGCAGAGCAG   300
   GCTGACGACC TGCAAGCCAC AGTGGCTGCC CTGTGCGTGC TGCGAGGTGG GGGACCCTGG   360
   GCAGGAAGCT GGCTGAGCCC CAAGACCCCG GGGGCCATGG GCGGGGATCT GGTGCTTGGC   420
   CTGGGGGCCT TGAGACGCCG AAAGCGCTTG CTGGACCAGG AGAAGTCTCT GGCCGGCTGG   480
   GCACTGGTGC TGGCAGGAAC TGGCATTGGA CTCATGGTGC TGCATGCAGA GATGCTGTGG   540
   TTCGGGGGGT GCTCGTGGGC GCTCTACCTG TTCCTGGTTA AATGCACGAT CAGCATTTCC   600
   ACCTTCTTAC TCCTCTGCCT CATCGTGGCC TTTCATGCCA AAGAGGTCCA GCTGTTCATG   660
   ACCGACAACG GGCTGCGGGA CTGGCGCGTG GCGCTGACCG GGCGGCAGGC GGCGCAGATC   720
   GTGCTGGAGC TGGTGGTGTG TGGGCTGCAC CCGGCGCCCG TGCGGGGCCC GCCGTGCGTG   780
   CAGGATTTAG GGGCGCCGCT GACCTCCCCG CAGCCCTGGC CGGGATTCCT GGGCCAAGGG   840
   GAAGCGCTGC TGTCCCTGGC CATGCTGCTG CGTCTCTACC TGGTGCCCCG CGCCGTGCTC   900
   CTGCGCAGCG GCGTCCTGCT CAACGCTTCC TACCGCAGCA TCGGCGCTCT CAATCAAGTC   960
   CGCTTCCGCC ACTGGTTCGT GGCCAAGCTT TACATGAACA CGCACCCTGG CCGCCTGCTG  1020
   CTCGGCCTCA CGCTTGGCCT CTGGCTGACC ACCGCCTGGG TGCTGTCCGT GGCCGAGAGG  1080
   CAGGCTGTTA ATGCCACTGG GCACCTTTCA GACACACTTT GGCTGATCCC CATCACATTC  1140
   CTGACCATCG GCTATGGTGA CGTGGTGCCG GGCACCATGT GGGGCAAGAT CGTCTGCCTG  1200
   TGCACTGGAG TCATGGGTGT CTGCTGCACA GCCCTGCTGG TGGCCGTGGT GGCCCGGAAG  1260
   CTGGAGTTTA ACAAGGCAGA GAAGCACGTG CACAACTTCA TGATGGATAT CCAGTATACC  1320
   AAAGAGATGA AGGAGTCCGC TGCCCGAGTG CTACAAGAAG CCTGGATGTT CTACAAACAT  1380
   ACTCGCAGGA AGGAGTCTCA TGCTGCCCGC AGGCATCAGC GCAAGCTGCT GGCCGCCATC  1440
   AACGCGTTCC GCCAGGTGCG GCTGAAACAC CGGAAGCTCC GGGAACAAGT GAACTCCATG  1500
   GTGGACATCT CCAAGATGCA CATGATCCTG TATGACCTGC AGCAGAATCT GAGCAGCTCA  1560
   CACCGGGCCC TGGAGAAACA GATTGACACG CTGGCGGGGA AGCTGGATGC CCTGACTGAG  1620
   CTGCTTAGCA CTGCCCTGGG GCCGAGGCAG CTTCCAGAAC CCAGCCAGCA GTCCAAGTAG  1680
   CTGGACCCAC GAGGAGGAAC CAGGCTACTT TCCCCAGTAC TGAGGTGGTG GACATCGTCT  1740
   CTGCCACTCC TGACCCAGCC CTGAACAAAG CACCTCAAGT GCAAGGACCA AAGGGGGCCC  1800
   TGGCTTGGAG TGGGTTGGCT TGCTGATGGC TGCTGGAGGG GACGCTGGCT AAAGTGGGTA  1860
   GGCCTTGGCC CACCTGAGGC CCCAGGTGGG AACATGGTCA CCCCCACTCT GCATACCCTC  1920
   ATCAAAAACA CTCTCACTAT GCTGCTATGG ACGACCTCCA GCTCTCAGTT ACAAGTGCAG  1980
   GCGACTGGAG GCAGGACTCC TGGGTCCCTG GGAAAGAGGG TACTAGGGGC CCGGATCCAG  2040
   GATTCTGGGA GGCTTCAGTT ACCGCTGGCC GAGCTGAAGA ACTGGGTATG AGGCTGGGGC  2100
   GGGGCTGGAG GTGGCGCCCC CTGGTGGGAC AACAAAGAGG ACACCATTTT TCCAGAGCTG  2160
   CAGAGAGCAC CTGGTGGGGA GGAAGAAGTG TAACTCACCA GCCTCTGCTC TTATCTTTGT  2220
   AATAAATGTT AAAGCCAG                                                2238
(2) INFORMATION FOR SEQ ID NO:8:
   (i) SEQUENCE CHARACTERISTICS:
      (A) LENGTH: 1250 base pairs
      (B) TYPE: nucleic acid
      (C) STRANDEDNESS: single
      (D) TOPOLOGY: linear
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 1624 base pairs
      (B) TYPE: nucleic acid
      (C) STRANDEDNESS: single
      (D) TOPOLOGY: linear
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 21 amino acids
      (8) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 10:
   Pro Ala Ser Phe Trp Trp Ala Thr Ile Thr Met Thr Thr Val Gly Tyr
   1               5                   10                  15
   Gly Asp Ile Tyr Pro
                   20
(2) INFORMATION FOR SEQ ID NO: 11:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:11:
      GTGGACATCT CCAAGGTACT AGGA. 24
(2) INFORMATION FOR SEQ ID NO:12:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:12:
      TTTACTGACA GGCTGTGTGT GCCA 24
(2) INFORMATION FOR SEQ ID NO:13:
   (i) SEQUENCE CHARACTERISTICS:
      (A) LENGTH: 1381 base pairs
      (B) TYPE: nucleic acid
      (C) STRANDEDNESS: single
      (D) TOPOLOGY: linear
   (xi) SEQUENCE DESCRIPTION: SEQ ID NO: 13:
   CAAGCTGGCT GCCATTTGCG TACTGAGAGG TGGGGGACCT GGGCAGGAAG CTGGCTGAGC    60
   CCCAAGACCT CAGGGGCCAT GGGCGGGGAG CTGGTGACTG GCCTGGGGGC CCTGAGACGG   120
   AGAAAGCGCC TGCTGGAGCA GGAGAAGAGG GTGGCCGGCT GGGCGTTGGT GCTGGCGGGA   180
   ACTGGCATCG GACTCATGGT TCTGCACGCT GAGATGTTGT GGTTCCTGGG CTGCAAGTGG   240
   GTGCTGTACC TGCTCCTGGT TAAGTGTTTG ATCACCCTGT CCACTGCCTT CCTCCTTTGT   300
   CTTATTGTGG TCTTCCATGC CAAGGAGGTC CAGCTGTTCA TGACTGACAA CGGGCTCCGG   360
   GACTGGCGCG TGGCGCTGAC CCGGCGGCAG GTGGCGCAGA TCCTGCTGGA GCTGTTGGTG   420
   TGCGGGGTGC ACCCGGTGCC CCTACGGAGC CCGCACTGCG CCCTGGCGGG GGAGGCCACC   480
   GACGCGCAGC CCTGGCCGGG TTTCCTGGGC GAAGGCGAGG CGTTGCTGTC CCTGGCCATG   540
   CTCCTGCGTC TCTACCTGGT GCCCCGCGCG GTGCTGCTGC GCAGCGGGGT CCTGCTCAAC   600
   GCGTCCTACC GCAGCATCGG GGCGCTCAAC CAAGTCCGCT TCCGCCACTG GTTCGTGGCC   660
   AAGCTGTACA TGAACACGCA CCCGGGTCGC CTGCTGCTGG GCCTCACGCT GGGTCTCTGG   720
   CTCACCACAG CTTGGGTGCT GTCTGTGGCT GAGAGGCAGG CTGTCAATGC CACGGGGCAC   780
   CTCACAGACA CACTGTGGCT GATTCCGATC ACATTCCTGA CCATTGGCTA TGGGGACGTG   840
   GTACCTGGCA CCATGTGGGG CAAGATTGTC TGCCTGTGCA CCGGAGTCAT GGGGGTCTGC   900
   TGCACAGCTC TCCTGGTGGC TGTGGTGGCT CGGAAGCTGG AGTTCAACAA GGCGGAGAAA   960
   CACGTGCACA ACTTCATGAT GGACATCCAT TATGCCAAAG AGATGAAGGA GTCAGCGGCG  1020
   CGGCTGCTGC AGGAAGCCTG GATGTACTAC AAGCACACTC GAAGGAAGGA CTCCCGGGCT  1080
   GCCCGCAGAC ATCAGCGCAA GATGCTGGCC GCCATCCACA CGTTCCGCCA GGTACGGCTG  1140
   AAACACCGGA AGCTCCGGGA ACAAGTGAAT TCCATGGTGG ACATCTCCAA GATGCACATG  1200
   ATCCTGTGCG ACCTGCAGCT GGGTCTCAGC TCCTCGCACC GTGCCCTGGA GAAGAGAATC  1260
   GACGGTCTGG CAGGAAAGCT GGATGCCCTG ACAGAGCTGC TCGGCACTGC TCTGCAGCAA  1320
   CAGCAGCTAC CAGAACCCAG TCAGGAGGCC ACATAGCTCC ACATGAACTC ACAGAAGAAC  1380
   A                                                                  1381
(2) INFORMATION FOR SEQ ID NO:14:
   (i) SEQUENCE CHARACTERISTICS:
      (A) LENGTH: 425 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:14:
   Met Gly Gly Glu Leu Val Thr Gly Leu Gly Ala Leu Arg Arg Arg Lys
   1 5 10 15
   Arg Leu Leu Glu Gln Glu Lys Arg Val Ala Gly Trp Ala Leu Val Leu
   20 25 30
   Ala Gly Thr Gly Ile Gly Leu Met Val Leu His Ala Glu Met Leu Trp
   35 40 45
   Phe Leu Gly Cys Lys Trp Val Leu Tyr Leu Leu Leu Val Lys Cys Leu
   50 55 60
   Ile Thr Leu Ser Thr Ala Phe Leu Leu Cys Leu Ile Val Val Phe His
   65 70 75 80
   Ala Lys Glu Val Gln Leu Phe Met Thr Asp Asn Gly Leu Arg Asp Trp
   85 90 95
   Arg Val Ala Leu Thr Arg Arg Gln Val Ala Gln Ile Leu Leu Glu Leu
   100 105 110
   Leu Val Cys Gly Val His Pro Val Pro Leu Arg Ser Pro His Cys Ala
   115 120 125
   Leu Ala Gly Glu Ala Thr Asp Ala Gln Pro Trp Pro Gly Phe Leu Gly
   130 135 140
   Glu Gly Glu Ala Leu Leu Ser Leu Ala Met Leu Leu Arg Leu Tyr Leu
   145 150 155 160
   Val Pro Arg Ala Val Leu Leu Arg Ser Gly Val Leu Leu Asn Ala Ser
   165 170 175
   Tyr Arg Ser Ile Gly Ala Leu Asn Gln Val Arg Phe Arg His Trp Phe
   180 185 190
   Val Ala Lys Leu Tyr Met Asn Thr His Pro Gly Arg Leu Leu Leu Gly
   195 200 205
   Leu Thr Leu Gly Leu Trp Leu Thr Thr Ala Trp Val Leu Ser Val Ala
   210 215 220
   Glu Arg Gln Ala Val Asn Ala Thr Gly His Leu Thr Asp Thr Leu Trp
   225 230 235 240
   Leu Ile Pro Ile Thr Phe Leu Thr Ile Gly Tyr Gly Asp Val Val Pro
   245 250 255
   Gly Thr Met Trp Gly Lys Ile Val Cys Leu Cys Thr Gly Val Met Gly
               260                 265                  270
   Val Cys Cys Thr Ala Leu Leu Val Ala Val Val Ala Arg Lys Leu Glu
           275                 280                 285
   Phe Asn Lys Ala Glu Lys His Val His Asn Phe Met Met Asp Ile His
       290                 295                 300
   Tyr Ala Lys Glu Met Lys Glu Ser Ala Ala Arg Leu Leu Gln Glu Ala
   305                 310                 315                 320
   Trp Met Tyr Tyr Lys His Thr Arg Arg Lys Asp Ser Arg Ala Ala Arg
                   325                 330                 335
   Arg His Gln Arg Lys Met Leu Ala Ala Ile His Thr Phe Arg Gln Val
               340                 345                 350
   Arg Leu Lys His Arg Lys Leu Arg Glu Gln Val Asn Ser Met Val Asp
           355                 360                 365
   Ile Ser Lys Met His Met Ile Leu Cys Asp Leu Gln Leu Gly Leu Ser
       370                 375                 380
   Ser Ser His Arg Ala Leu Glu Lys Arg Ile Asp Gly Leu Ala Gly Lys
   385                 390                 395                 400
   Leu Asp Ala Leu Thr Glu Leu Leu Gly Thr Ala Leu Gln Gln Gln Gln
                   405                 410                 415
   Leu Pro Glu Pro Ser Gln Glu Ala Thr
               420                 425

## Claims

1. A purified polynucleotide comprising a nucleic acid sequence encoding the polypeptide having the sequence of SEQ ID NO:3.

2. The polynucleotide of claim 1 wherein the polynucleotide sequence comprises SEQ ID NO:2.

3. An expression vector comprising the polynucleotide of claim 1.

4. An antisense molecule comprising the complement of the polynucleotide of claim 2.

5. An isolated host cell transformed with the expression vector of claim 3.

6. A purified polypeptide comprising the amino acid sequence of SEQ ID NO:3.

7. An antibody specific for the polypeptide of claim 6.

8. A method for producing cells which express a polypeptide having the sequence of SEQ ID NO:3, said method comprising culturing a host cell according to claim 5 under conditions suitable for the expression of said polypeptide.

9. A method for producing a polypeptide having the amino acid sequence of SEQ ID NO:3, said method comprising the steps of: culturing a host cell according to claim 5 under conditions suitable for the expression of said polypeptide, and recovering said polypeptide from the host cell culture.

10. A method for identifying compounds that modulate the activity of the polypeptide of claim 6, comprising:
(a) combining a test compound with the polypeptide of claim 6, and
(b) measuring an effect of the test compound on the polypeptide.

11. A method for identifying compounds that modulate the activity of a potassium channel comprising the amino acid sequence of SEQ ID NO:3, comprising:
(a) combining a test compound modulator of a potassium channel activity with a host-cell expressing a polypeptide of a potassium channel comprising the amino acid sequence of SEQ ID NO:3, and
(b) measuring an effect of the test compound on the channel.

12. A composition for the identification of a polypeptide sequence comprising the amino acid sequence of SEQ ID NO:3 comprising the antibody of claim 7.

13. A method of screening a plurality of compounds for binding affinity with the polypeptide of claim 6, said method comprising the steps of:
a) providing a plurality of compounds;
b) combining the compounds with the polypeptide of claim 6 for a time sufficient for a compound to bind the polypeptide; and
c) identifying the compounds which bind the polypeptide.

## Patentansprüche

1. Gereinigtes Polynukleotid, umfassend eine für das Polypeptid mit der Sequenz der SEQ ID NO:3 codierende Nukleinsäuresequenz.

2. Polynukleotid nach Anspruch 1, wobei die Polynukleotidsequenz die SEQ ID NO:2 umfaßt.

3. Expressionsvektor, umfassend das Polynukleotid nach Anspruch 1.

4. Antisense-Molekül, umfassend das Komplement des Polynukleotids nach Anspruch 2.

5. Isolierte Wirtszelle, transformiert mit dem Expressionsvektor nach Anspruch 3.

6. Gereinigtes Polypeptid, umfassend die Aminosäuresequenz der SEQ ID NO:3.

7. Antikörper, spezifisch für das Polypeptid nach Anspruch 6.

8. Verfahren zur Herstellung von Zellen, die ein Polypeptid mit der Sequenz der SEQ ID NO:3 exprimieren, wobei man in dem Verfahren eine Wirtszelle gemäß Anspruch 5 unter für die Expression des Polypeptids geeigneten Bedingungen kultiviert.

9. Verfahren zur Herstellung eines Polypeptids mit der Sequenz der SEQ ID NO:3, wobei man in dem Verfahren die Schritte des Kultivierens einer Wirtszelle gemäß Anspruch 5 unter für die Expression des Polypeptids geeigneten Bedingungen sowie des Gewinnens des Polypeptids aus der Wirtszellkultur durchführt.

10. Verfahren zur Identifizierung von Verbindungen, die die Aktivität des Polypeptids nach Anspruch 6 modulieren, bei dem man:
(a) eine Testverbindung mit dem Polypeptid nach Anspruch 6 kombiniert und
(b) eine Wirkung der Testverbindung auf das Polypeptid mißt.

11. Verfahren zur Identifizierung von Verbindungen, die die Aktivität eines die Aminosäuresequenz der SEQ ID NO:3 umfassenden Kaliumkanals modulieren, wobei man in dem Verfahren:
(a) einen Testverbindungsmodulator einer Kaliumkanalaktivität mit einer ein die Aminosäuresequenz der SEQ ID NO:3 umfassendes Polypeptid eines Kaliumkanals exprimierenden Wirtszelle kombiniert und
(b) eine Wirkung der Testverbindung auf den Kanal mißt.

12. Zusammensetzung zur Identifizierung einer die Aminosäuresequenz der SEQ ID NO:3 umfassenden Polypeptidsequenz, umfassend den Antikörper nach Anspruch 7.

13. Verfahren zum Screening mehrerer Verbindungen auf Bindungsaffinität mit dem Polypeptid nach Anspruch 6, wobei man in dem Verfahren die folgenden Schritte durchführt:
(a) Bereitstellen mehrerer Verbindungen;
(b) Kombinieren der Verbindungen mit dem Polypeptid nach Anspruch 6 über einen Zeitraum, der zur Bindung einer Verbindung an das Polypeptid ausreicht; und
(c) Identifizieren der das Polypeptid bindenden Verbindungen.

## Revendications

1. Polynucléotide purifié comprenant une séquence d'acide nucléique codant pour le polypeptide ayant la séquence de SEQ ID NO:3.

2. Polynucléotide selon la revendication 1, **caractérisé en ce que** la séquence polynucléotidique comprend SEQ ID NO:2.

3. Vecteur d'expression comprenant le polynucléotide selon la revendication 1.

4. Molécule antisens comprenant la séquence complémentaire du polynucléotide selon la revendication 2.

5. Cellule hôte isolée, transformée par le vecteur d'expression selon la revendication 3.

6. Polypeptide purifié comprenant la séquence d'acides aminés de SEQ ID NO:3.

7. Anticorps spécifique du polypeptide selon la revendication 6.

8. Procédé de production de cellules qui expriment un polypeptide ayant la séquence de SEQ ID NO:3, ledit procédé comprenant la culture d'une cellule hôte selon la revendication 5 dans des conditions propices à l'expression dudit polypeptide.

9. Procédé de production d'un polypeptide ayant la séquence d'acides aminés de SEQ ID NO:3, ledit procédé comprenant les étapes consistant à: cultiver une cellule hôte selon la revendication 5 dans des conditions propices à l'expression dudit polypeptide, et récupérer ledit polypeptide à partir de la culture de cellules hôtes.

10. Procédé d'identification de composés qui modulent l'activité du polypeptide selon la revendication 6, comprenant les étapes consistant à:
(a) combiner un composé à l'essai avec le polypeptide selon la revendication 6, et
(b) mesurer un effet du composé à l'essai sur le polypeptide.

11. Procédé d'identification de composés qui modulent l'activité d'un canal potassique comprenant la séquence d'acides aminés de SEQ ID NO:3, comprenant les étapes consistant à:
(a) combiner un composé à l'essai modulateur de l'activité d'un canal potassique avec une cellule hôte exprimant un polypeptide d'un canal potassique comprenant la séquence d'acides aminés de SEQ ID NO:3, et
(b) mesurer un effet du composé à l'essai sur le canal.

12. Composition pour l'identification d'une séquence polypeptidique comprenant la séquence d'acides aminés de SEQ ID NO:3, comprenant l'anticorps selon la revendication 7.

13. Procédé de criblage d'une pluralité de composés pour une affinité de liaison avec le polypeptide selon la revendication 6, ledit procédé comprenant les étapes consistant à:
(a) fournir une pluralité de composés;
(b) combiner les composés avec le polypeptide selon la revendication 6 pendant un temps suffisant pour qu'un composé se lie au polypeptide; et
(c) identifier les composés qui se lient au polypeptide.
